(19) Europäisches Patentamt
European Patent Office
Office européen des brevets

(11) EP 2 067 480 A1

(12) **EUROPEAN PATENT APPLICATION**
published in accordance with Art. 153(4) EPC

(43) Date of publication:
**10.06.2009 Bulletin 2009/24**

(21) Application number: **06810960.2**

(22) Date of filing: **30.09.2006**

(51) Int Cl.:
***A61K 38/00*** *(2006.01)* ***A61K 31/7088*** *(2006.01)*
***A61K 39/395*** *(2006.01)* ***A61K 48/00*** *(2006.01)*
***A61K 49/00*** *(2006.01)* ***A61P 3/04*** *(2006.01)*
***A61P 3/06*** *(2006.01)* ***A61P 3/10*** *(2006.01)*
***A61P 9/10*** *(2006.01)* ***A61P 9/12*** *(2006.01)*
***A61P 19/00*** *(2006.01)* ***G01N 33/15*** *(2006.01)*
***G01N 33/50*** *(2006.01)*

(86) International application number:
**PCT/JP2006/319605**

(87) International publication number:
**WO 2008/038394 (03.04.2008 Gazette 2008/14)**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicants:
- **Takeda Pharmaceutical Company Limited**
**Osaka- shi, Osaka 541-0045 (JP)**
- **Osaka University**
**Suita-shi**
**Osaka 565-0871 (JP)**

(72) Inventors:
- **KITA, Shunbun**
**Osaka-shi**
**Osaka 532-8686 (JP)**
- **SHIMOMURA, Iichirou**
**Suita-shi**
**Osaka 565-0871 (JP)**
- **YAMADA, Yukio**
**Osaka-shi**
**Osaka 532-8686 (JP)**

(74) Representative: **Brearley, Helen Rebecca et al**
**Elkington and Fife LLP**
**Prospect House**
**8 Pembroke Road**
**Sevenoaks**
**Kent TN13 1XR (GB)**

(54) **MUSCLIN RECEPTOR AND USE THEREOF**

(57) The present invention provides a receptor of musclin and a screening system for an agonist and/or antagonist of the receptor by means of the interaction of musclin and the receptor. Hence, provided is a screening method for a substance that alters the bindability (i) a protein containing the same or substantially the same amino acid sequence as the amino acid sequence shown by SEQ ID NO:2 or a partial peptide thereof or a salt thereof and (ii) musclin or a partial peptide thereof or a salt thereof, containing using both.

EP 2 067 480 A1

## Description

Technical Field

The present invention relates to a receptor of skeletal muscle specific secretory protein (musclin) and a screening method for a prophylactic/therapeutic drug for a disease involved by musclin, using the receptor, and the like.

Background Art

So-called lifestyle-related diseases such as diabetes, hyperlipemia, hypertension, and ischemic heart disease represent the biggest cause of medical financial difficulty in advanced countries, including Japan. Modern people are likely to suffer overeating and underexercise; as a result, excess energy is accumulated in fat tissue as neutral fat, which in turn causes obesity.

In recent years, it has been shown that various bioactive proteins secreted from fat tissue (generically referred to as adipocytokines) are involved in metabolic regulation and insulin resistance, and attempts have been made to apply them to the treatment of lifestyle-related diseases. Fat accumulation in obesity also occurs in the liver, muscles, pancreas, vascular walls and the like, which in turn causes metabolic disorders in these organs and lead to the development of insulin resistance, diabetes, hyperlipemia, hypertension and the like. These organs are thought to be mutually associated via endocrine factors, and to make complex and sophisticated metabolic regulation. Therefore, it is estimated that endocrine factors that exhibit the same actions as those of adipocytokines are produced and secreted from muscles, the liver, the small intestine, blood vessels and the like.

Shimomura et al. isolated the gene that encodes a secretory protein expressed specifically in mouse skeletal muscles [named "musclin"] using the signal sequence trap (SST) method, and reported that the expression of the gene (1) is remarkably increased by differentiation induction of muscle cells, (2) is decreased and re-increased by fasting and re-feeding, and (3) is elevated in obese diabetes models, and that musclin is expression-induced in response to hyperinsulinemia and regulates sugar uptake and glycogen synthesis in skeletal muscle cells (patent document 1 and non-patent document 1; in patent document 1, musclin is referred to as "SS169"). Other reports are available that a protein identical to musclin [called osteocrin or bone peptide-1] is expressed specifically in bone to regulate the phenotypes of osteoblasts (patent document 2 and non-patent document 2).

Musclin cannot exhibit the action thereof unless it interacts with a specific receptor in a target cell. Identification of musclin receptor is thought to significantly contribute to the elucidation of the interactions of musclin and the receptor, the development of receptor agonists/antagonists, and hence the development of prophylactic/therapeutic drugs for diabetes, insulin resistance, hypertension, hyperlipemia and the like. However, to date, it has never been reported on a receptor of musclin (or a protein identical thereto, osteocrin).

patent document 1: WO2004/1112 (full text)
patent document 2: WO03/054005 (full text)
non-patent document 1: Nishizawa, H. et al., Journal of Biological Chemistry, vol. 279 (No. 19), pp. 19391-19395 (2004)
non-patent document 2: Thomas, G. et al., Journal of Biological Chemistry, vol, 278 (No. 50), pp. 50563-50571 (2003)

Disclosure of the Invention

Problems to Be Solved by the Invention

Accordingly, it is an object of the present invention to identify a receptor of musclin and elucidate the interactions of musclin and the receptor, and to provide a screening system using musclin and the receptor for an agonist and/or antagonist of the receptor, that is, a pharmaceutical candidate compound that is effective as a prophylactic/therapeutic drug for diabetes, insulin resistance, hypertension, hyperlipemia and the like.

Means of Solving the Problems

The present inventors, taking note of the fact that musclin protein contains a region homologous to that of the natriuretic peptide (NP) family (atrial (ANP), brain (BNP), and type C (CNP) are known) and the like, examined the bindability of the receptors of the peptides (3 kinds of NPR1, NPR2, and NPR3 are known) and musclin, and found that musclin binds specifically to the receptor NPR3 (also called Npr3, Npr-3, NPR-3, Npr-c, NPR-C; herein, denoted as "NPR3"), which is reportedly involved in ANP clearance. The present inventors conducted further investigations based on these findings, and developed the present invention.

Accordingly, the present invention provides:

[1] a cell differentiation and/or metabolism regulator comprising a protein comprising the same or substantially the same amino acid sequence as the amino acid sequence shown by SEQ ID NO:2 or a partial peptide thereof or a salt thereof,
[2] the agent described in [1] above, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism,
[3] the agent described in [1] above, which is for prevention/treatment of 1 or more diseases selected from the group consisting of obesity, diabetes, impaired glucose tolerance, arteriosclerosis, hypertension, metabolic bone/cartilage diseases and hyperlipemia,
[4] a cell differentiation and/or metabolism regulator comprising a nucleic acid that encodes a protein comprising the same or substantially the same amino acid sequence as the amino acid sequence shown by SEQ ID NO:2 or a partial peptide thereof,
[5] the agent described in [4] above, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism,
[6] the agent described in [4] above, which is for prevention/treatment of 1 or more diseases selected from the group consisting of obesity, diabetes, impaired glucose tolerance, arteriosclerosis, hypertension, metabolic bone/cartilage diseases and hyperlipemia,
[7] a diagnostic agent for cell differentiation abnormalities and/or metabolic abnormalities comprising a nucleic acid that encodes a protein comprising the same or substantially the same amino acid sequence as the amino acid sequence shown by SEQ ID NO:2 or a partial polynucleotide thereof,
[8] the agent described in [7] above, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism,
[9] the agent described in [7] above, which is for diagnosis of 1 or more diseases selected from the group consisting of obesity, diabetes, impaired glucose tolerance, arteriosclerosis, hypertension, metabolic bone/cartilage diseases and hyperlipemia,
[10] a diagnostic agent for cell differentiation abnormalities and/or metabolic abnormalities comprising an antibody against a protein comprising the same or substantially the same amino acid sequence as the amino acid sequence shown by SEQ ID NO:2 or a partial peptide thereof or a salt thereof,
[11] the agent described in [10] above, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism,
[12] the agent described in [10] above, which is for diagnosis of 1 or more diseases selected from the group consisting of obesity, diabetes, impaired glucose tolerance, arteriosclerosis, hypertension, metabolic bone/cartilage diseases and hyperlipemia,
[13] a cell differentiation and/or metabolism regulator comprising an antibody against a protein comprising the same or substantially the same amino acid sequence as the amino acid sequence shown by SEQ ID NO:2 or a partial peptide thereof or a salt thereof,
[14] the agent described in [13] above, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism,
[15] the agent described in [13] above, which is for prevention/treatment of 1 or more diseases selected from the group consisting of obesity, diabetes, impaired glucose tolerance, arteriosclerosis, hypertension, metabolic bone/cartilage diseases and hyperlipemia,
[16] a cell differentiation and/or metabolism regulator comprising a nucleic acid comprising a base sequence complementary to a nucleic acid that encodes a protein comprising the same or substantially the same amino acid sequence as the amino acid sequence shown by SEQ ID NO:2 or a portion thereof,
[17] the agent described in [16] above, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism,
[18] the agent described in [16] above, which is for prevention/treatment of 1 or more diseases selected from the group consisting of obesity, diabetes, impaired glucose tolerance, arteriosclerosis, hypertension, metabolic bone/cartilage diseases and hyperlipemia,
[19] a screening method for a substance that exhibits cell differentiation and/or metabolism regulatory action, comprising using a protein comprising the same or substantially the same amino acid sequence as the amino acid sequence shown by SEQ ID NO:2 or a partial peptide thereof or a salt thereof,
[20] the method described in [19] above, wherein the protein comprising the same or substantially the same amino acid sequence as the amino acid sequence shown by SEQ ID NO:2 or the partial peptide thereof or the salt thereof is provided in the form of a cell that produces the same,
[21] the method described in [20] above, further comprising using a protein comprising a nucleic acid that encodes the same or substantially the same amino acid sequence as the amino acid sequence shown by SEQ ID NO:2 or a partial polynucleotide thereof, or an antibody against a protein comprising the same or substantially the same amino acid sequence as the amino acid sequence shown by SEQ ID NO:2 or a partial peptide thereof or a salt thereof,
[22] the method described in [19] above, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism,
[23] the method described in [19] above, wherein the substance possesses prophylactic/therapeutic activity on 1 or more diseases selected from the group consisting of obesity, diabetes, impaired glucose tolerance, arteriosclerosis, hypertension, metabolic bone/cartilage diseases and hyperlipemia,
[24] a screening method for a substance that alters the bindability of (i) a protein comprising the same or substantially the same amino acid sequence as the amino acid sequence shown by SEQ ID NO:2 or a partial peptide thereof or a salt thereof, and (ii) musclin or a partial peptide thereof or a salt thereof, comprising using both,
[25] the method described in [24] above, wherein the protein comprising the same or substantially the same amino acid sequence as the amino acid sequence shown by SEQ ID NO:2 or the partial peptide thereof or the salt thereof is provided in the form of a cell that produces the same,
[26] the method described in [25] above, wherein sugar uptake in cells or glycogen synthesis in cells is used as an index,
[27] the method described in [24] above, wherein the substance exhibits cell differentiation and/or metabolism regulatory action,
[28] the method described in [27] above, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism, and
[29] the method described in [27] above, wherein the substance possesses prophylactic/therapeutic activity on 1 or more diseases selected from the group consisting of obesity, diabetes, impaired glucose tolerance, arteriosclerosis, hypertension, metabolic bone/cartilage diseases and hyperlipemia, and the like.

Effect of the Invention

Because NPR3 functions as a receptor of musclin, by using NPR3 and musclin, it is possible to screen for a pharmaceutical candidate compound that is effective in the prophylaxis/treatment of diseases associated with abnormalities of cell differentiation or sugar/lipid /protein metabolism in skeletal muscles and the like. Best Mode for Carrying out the Invention

The musclin receptor protein used in the present invention is a protein comprising the same or substantially the same amino acid sequence as the amino acid sequence shown by SEQ ID NO:2. The protein is one of the receptors of the natriuretic peptide (NP) family (ANP, BNP, CNP), and is a protein identical to the receptor NPR3, which is reportedly involved in ANP clearance. Therefore, hereinunder, the musclin receptor protein of the present invention is sometimes referred to as "NPR3".

NPR3 may be a protein isolated and purified from cells [for example, liver cells, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, adipocytes, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, interstitial cells, or corresponding precursor cells, stem cells, cancer cells, and the like], or from any tissues where these cells are present [for example, brain, brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, lung, gastrointestinal organs (e.g., large intestine, small intestine), blood vessels, heart, thymus, spleen, submandibular gland, peripheral blood, prostate, testis, ovary, placenta, uterus, bone, joints, adipose tissues (e.g., brown adipose tissue, white adipose tissue), skeletal muscles and the like] of mammals (for example, humans, mice, rats, rabbits, sheep, swine, cattle, horses, cats, dogs, monkeys, chimpanzee and the like) and the like. The protein may also be a chemically synthesized protein or a protein biochemically synthesized using a cell-free translation system, or a recombinant protein produced from a transformant incorporating a nucleic acid having the base sequence encoding the above-described amino acid sequence.

As substantially the same amino acid sequence as the amino acid sequence shown by SEQ ID NO:2, an amino acid sequence having a homology of about 70% or more, preferably about 80% or more, more preferably about 90% or more, and particularly preferably about 95% or more, to the amino acid sequence shown by SEQ ID NO:2. As used herein, "homology" means the proportion (%) of the same and similar amino acid residues to all overlapping amino acid residues in the optimal alignment where two amino acid sequences are aligned using a mathematic algorithm known in the relevant technical field (preferably, the algorithm is such that a gap can be introduced into one or both of the sequences for the optimal alignment). "A similar amino acid" means an amino acid having similar physiochemical properties; as examples, amino acids classified under the same group, such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxy group (Ser, Thr), and amino acids having a small side chain (Gly, Ala, Ser, Thr, Met), can be mentioned. Substitution by such a similar amino acid is expected to produce no change in the phenotype of protein (i.e., conservative amino acid substitution). Specific examples of conservative amino acid substitution are known in the relevant technical field and described in various pieces of the literature (see, for example, Bowie et al., Science, 247: 1306-1310 (1990)).

Amino acid sequence homology herein can be calculated using the homology calculation algorithm NCBI BLAST (National Center for Biotechnology Information Basic Local Alignment Search Tool) under the following conditions (expectancy=10; gap allowed; matrix=BLOSUM62; filtering=OFF). Algorithms to determine the homology of an amino acid sequence include, for example, but are not limited to, the algorithm described in Karlin et al., Proc. Natl. Acad. Sci. USA, 90: 5873-5877 (1993) [the algorithm is incorporated in the NBLAST and XBLAST programs (version 2.0) (Altschul et al., Nucleic Acids Res., 25: 3389-3402 (1997))], the algorithm described in Needleman et al., J. Mol. Biol., 48: 444-453 (1970) [the algorithm is incorporated in the GAP program in the GCG software package], the algorithm described in Myers and Miller, CABIOS, 4: 11-17 (1988) [the algorithm is incorporated in the ALIGN program (version 2.0), which is part of the CGC sequence alignment software package], the algorithm described in Pearson et al., Proc. Natl. Acad. Sci. USA, 85: 2444-2448 (1988) [the algorithm is incorporated in the FASTA program in the GCG software package] and the like, and they can also be used preferably.

More preferably, substantially the same amino acid sequence as the amino acid sequence shown by SEQ ID NO:2 is an amino acid sequence having an identity of about 60% or more, preferably about 70% or more, more preferably about 80% or more, and particularly preferably about 90% or more, to the amino acid sequence shown by SEQ ID NO:2.

The protein comprising substantially the same amino acid sequence as the amino acid sequence shown by SEQ ID NO:2 is preferably, for example, a protein comprising the same or substantially the same amino acid sequence as the aforementioned amino acid sequence shown by SEQ ID NO:2, and having substantially the same quality of activity as a protein comprising the amino acid sequence shown by SEQ ID NO:2, or the like.

As examples of substantially the same quality of activity, ligand (i.e., musclin) binding activity, signal transmission activity and the like can be mentioned. "Substantially the same quality" means that the activities of the proteins are qualitatively (e.g., physiologically or pharmacologically) equivalent to each other. It is preferable, therefore, that activities such as ligand-binding activity and signal transmission activity be equivalent to each other (e.g., about 0.5 to about 2 times), but quantitative factors such as the extent of these activities and the molecular weights of the proteins may be different.

A measurement of an activity such as ligand-binding activity or signal transmission activity can be performed using a method known per se; for example, a measurement of an activity such as ligand-binding activity or signal transmission activity can be performed using a method known per se; for example, the measurement can be performed according to methods used in the screening methods for agonists and antagonists described below, methods with sugar uptake in cells/glycogen synthesis/degradation in cells, sugar release from cells, fat uptake in cells, fat release from cells, fat degradation in cells, fat synthesis in cells, proteinous/non-proteinous hormone secretion regulation, proteinous/non-proteinous hormone action regulation, appetite regulation, motor activity regulation, changes in body weight, changes in saccharides/lipids in blood, muscle cell differentiation/proliferation, osteoblast differentiation/proliferation or the like as an index, and the like.

Examples of the NPR3 of the present invention also include proteins comprising (1) an amino acid sequence having one or two or more amino acids (preferably about 1 to 100, more preferably about 1 to 50, and still more preferably several (1 to 5) amino acids) deleted from the amino acid sequence shown by SEQ ID NO:2, (2) an amino acid sequence having one or two or more amino acids (preferably about 1 to 100, more preferably about 1 to 50, and still more preferably about 1 to 10, particularly preferably several (1 to 5) amino acids) added to the amino acid sequence shown by SEQ ID NO:2, (3) an amino acid sequence having one or two or more amino acids (preferably about 1 to 50, more preferably about 1 to 10, and still more preferably several (1 to 5) amino acids) inserted in the amino acid sequence shown by SEQ ID NO:2, (4) an amino acid sequence having one or two or more amino acids (preferably about 1 to 50, more preferably about 1 to 10, and still more preferably several (1 to 5) amino acids) substituted with other amino acids in the amino acid sequence shown by SEQ ID NO:2, or (5) an amino acid sequence comprising a combination thereof, and the like. When an amino acid sequence is inserted, deleted or substituted as described above, the position of the insertion, deletion or substitution is not subject to limitation, as long as the protein activities are retained.

The NPR3 of the present invention is preferably the human NPR3 protein (GenBank accession number: NP_ 000899), which has the amino acid sequence shown by SEQ ID NO:2, or a homologue thereof in another mammal (for example, rat, mouse and bovine homologues registered at GenBank under accession numbers NP_037000, NP_032754 and NP_776552, respectively (having an identify of about 91%, about 90% or about 93%, to human NPR3, respectively) and the like).

In the present specification, proteins and peptides are denoted with the N-terminus (amino terminus) described as the left end and the C-terminus (carboxyl terminus) as the right end, in accordance with the common way of describing peptides. The NPR3 of the present invention, including a protein comprising the amino acid sequence shown by SEQ ID NO: 2, may have any of a carboxyl group (-COOH), a carboxylate ($-COO^-$), an amide ($-CONH_2$) or an ester (-COOR) at the C-terminus thereof.

As used herein, R in the ester is exemplified by $C_{1-6}$ alkyl groups, for example, as methyl, ethyl, n-propyl, isopropyl, n-butyl and the like; $C_{3-8}$ cycloalkyl groups, for example, cyclopentyl, cyclohexyl and the like; $C_{6-12}$ aryl groups, for example, phenyl, α-naphthyl and the like; phenyl-$C_{1-2}$ alkyl groups, for example, benzyl, phenethyl and the like; $C_{7-14}$ aralkyl groups such as α-naphthyl-$C_{1-2}$-alkyl groups such as α-naphthylmethyl; pivaloyloxymethyl groups; and the like.

When the NPR3 of the present invention has a carboxyl group (or carboxylate) at a position other than the C-terminus, the carboxyl group may be amidated or esterified, and such proteins are also included in the protein of the present invention. As examples of the ester used in this case, the above-described esters at the C-terminus can be mentioned.

Furthermore, the NPR3 of the present invention also includes those wherein the amino group of the amino acid residue at the N-terminus is protected with a protecting group (for example, $C_{1-6}$ acyl groups such as $C_{1-6}$ alkanoyl groups such as formyl group and acetyl group); those wherein the glutamyl group at the N-terminus, which can be produced upon cleavage in vivo, is pyroglutaminated; those wherein a substituent (for example, -OH, -SH, amino group, imidazole group, indole group, guanidino group and the like) on the side chain of an amino acid in the molecule is protected with an appropriate protecting group (for example, $C_{1-6}$ acyl groups such as $C_{1-6}$ alkanoyl groups such as formyl group and acetyl group, and the like), conjugated proteins such as what are called glycoproteins having sugar chains bound thereto, and the like.

A partial peptide of NPR3 (hereinafter sometimes simply abbreviated as "the partial peptide of the present invention") may be any peptide having the above-described partial amino acid sequence of NPR3, and having substantially the same quality of activity to that of NPR3. As used herein, "substantially the same quality of activity" has the same meaning as described above. A measurement of "substantially the same quality of activity" can be performed in the same manner as NPR3.

Specifically, examples of the partial peptide of the present invention include one having a partial amino acid sequence further comprising a region involved in the binding of musclin as a ligand and a region involved in the signal transduction mediated by the interaction, in the amino acid sequence shown by SEQ ID NO:2.

The partial peptide of the present invention is preferably a peptide having at least 50 or more, preferably 100 or more, and more preferably 200 or more amino acids.

On the other hand, peptides comprising a partial amino acid sequence of NPR3, but not having substantially the same quality of activity as NPR3, for example, one having a partial amino acid sequence comprising a region involved in the binding of a musclin, but not comprising a region involved in the signal transduction mediated by the interaction, in the amino acid sequence shown by SEQ ID NO:2, and the like, are not included in "the partial peptide of the present invention". However, because these peptides are capable of blocking signal transduction action via NPR3 by binding to musclin, they can be useful when the signal transduction action is to be suppressed.

Also, the partial peptide of the present invention may have any of a carboxyl group (-COOH), a carboxylate ($-COO^-$), an amide ($-CONH_2$), or an ester (-COOR) at the C-terminus thereof. As used herein, R in the ester is exemplified by the same examples as those mentioned with respect to NPR3. When the partial peptide of the present invention has a carboxyl group (or carboxylate) at a position other than the C-terminus, the carboxyl group may be amidated or esterified, and such partial peptides are also included in the partial peptide of the present invention. In this case, the ester is exemplified by the same examples as those mentioned with respect to the ester at the C-terminus.

Furthermore, the partial peptide of the present invention, like the above-described NPR3, also includes those wherein the amino group of the amino acid residue at the N-terminus is protected with a protecting group, those wherein the glutamine residue at the N-terminus is pyroglutaminated; those wherein a substituent on the side chain of an amino acid in the molecule is protected with an appropriate protecting group, conjugated peptides such as what are called glycopeptides having sugar chains bound thereto, and the like.

As the salt of NPR3 or a partial peptide thereof, physiologically acceptable salts with acids or bases can be mentioned, with preference given to physiologically acceptable acid adduct salts. Examples of such salts include salts with inorganic acids (for example, hydrochloric acid, phosphoric acid, hydrobromic acid, sulfuric acid), salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid), and the like.

NPR3 or a salt thereof can be produced from cells or tissues of mammals described above by a method of protein purification known per se. Specifically, NPR3 or a salt thereof can be prepared by homogenizing a tissue or cells of a mammal, removing the cell debris by low-speed centrifugation, centrifuging the supernatant at high speed to precipitate the cell membrane-containing fraction (if necessary, the cell membrane fraction is purified by density gradient centrifugation and the like), and subjecting the fraction to a chromatography such as reverse phase chromatography, ion exchange chromatography, or affinity chromatography, and the like.

NPR3 or a partial peptide thereof or a salt thereof (hereinafter sometimes generically referred to as "NPR3 species") can also be produced according to a known method of peptide synthesis.

The method of peptide synthesis may, for example, be any of solid phase synthesis and liquid phase synthesis. The desired protein can be produced by condensing a partial peptide or amino acids that can constitute an NPR3 species and the remaining portion, and, when the product has a protecting group, removing the protecting group.

Condensation and protecting group removal can be achieved by methods known per se, for example, the methods described in (1) to (5) below.

(1) M. Bodanszky and M.A. Ondetti: Peptide Synthesis, Interscience Publishers, New York (1966)
(2) Schroeder and Luebke: The Peptide, Academic Press, New York (1965)
(3) Nobuo Izumiya et al.: Peptide Gosei-no-Kiso to Jikken (Basics and experiments of peptide synthesis), Maruzen Co. (1975)
(4) Haruaki Yajima and Shunpei Sakakibara: Seikagaku Jikken Koza (Biochemical Experiment) 1, Tanpakushitsu no Kagaku (Chemistry of Proteins) IV, 205 (1977)
(5) Haruaki Yajima ed.: Zoku Iyakuhin no Kaihatsu (A sequel to Development of Pharmaceuticals), Vol. 14, Peptide Synthesis, Hirokawa Shoten

The protein (peptide) thus obtained can be purified and isolated using a known method of purification. Here, as examples of the method of purification, solvent extraction, distillation, column chromatography, liquid chromatography, recrystallization, combinations thereof and the like can be mentioned.

When the protein (peptide) obtained by the above-described method is the free form, the free form can be converted into an appropriate salt by a known method or a method based thereon; conversely, when the protein (peptide) is obtained in the form of a salt, the salt can be converted into the free form or another salt by a known method or a method based thereon.

To synthesize an NPR3 species, ordinary commercially available resins for protein synthesis may be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl)phenoxy resin and the like can be mentioned. Using such a resins, amino acids having $\alpha$-amino groups and side-chain functional groups protected as appropriate are condensed on the resin in accordance with the sequence of the desired protein (peptide) according to various methods of condensation methods known per se. At the end of the reaction, the protein and the like are excised from the resin and the various protecting groups are removed simultaneously; then, an intramolecular disulfide bond-forming reaction is performed in a highly diluted solution to give the desired protein (peptide) or an amide thereof.

For the above-described condensation of protected amino acids, various activation reagents for protein synthesis may be used, with preference given to carbodiimides. Useful carbodiimides include DCC, N,N'-diisopropylcarbodiimide, N-ethyl-N'-(3-dimethylaminopropyl)carbodiimide and the like. For activation with these reagents, protected amino acids, along with a racemization inhibitor (for example, HOBt, HOOBt), may be added directly to the resin, or may be added to the resin after being previously activated in the form of a symmetric acid anhydride, HOBt ester, or HOOBt ester.

Solvents used in the activation of protected amino acids or condensation with the resin can be selected as appropriate from among solvents known to be usable for protein condensation reactions. For example, acid amides such as N,N-dimethylformamide, N,N-dimethylacetamide, and N-methylpyrrolidone; halogenated hydrocarbons such as methylene chloride and chloroform; alcohols such as trifluoroethanol; sulfoxides such as dimethylsulfoxide; amines such as pyridine; ethers such as dioxane and tetrahydrofuran; nitriles such as acetonitrile and propionitrile; esters such as methyl acetate and ethyl acetate; appropriate mixtures of these solvents, and the like can be used. Reaction temperature is selected as appropriate from the range known to be useful for protein binding reactions, and is normally selected as appropriate from the range of about -20°C to 50°C. The activated amino acid derivative is normally used in an excess of 1.5 to 4 times. If a test using the ninhydrin reaction reveals insufficient condensation, the condensation can be completed by repeating the condensation reaction without splitting off the protecting groups. If the condensation is yet insufficient even after repeating the reaction, unreacted amino acids can be acetylated with acetic anhydride or acetylimidazole.

Protection and protecting groups for the functional groups that should not involve the reaction of the starting materials, splitting off the protecting groups, activation of the functional groups involved in the reaction, and the like can be selected as appropriate from among known groups or known means.
Examples of the protecting groups for the amino groups of the starting materials include Z, Boc, tertiary pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc and the like.
A carboxyl group can be protected by, for example, alkyl esterification (for example, esterification with a linear, branched or cyclic alkyl such as methyl, ethyl, propyl, butyl, tertiary butyl, cyclopentyl, cyclohexyl, cycloheptyl, cyclooctyl, or 2-adamantyl), aralkyl esterification (for example, benzyl esterification, 4-nitrobenzyl esterification, 4-methoxybenzyl esterification, 4-chlorobenzyl esterification, benzhydryl esterification), phenacyl esterification, benzyloxycarbonyl hydrazidation, tertiary butoxycarbonyl hydrazidation, trityl hydrazidation and the like.
The hydroxyl group of serine can be protected by, for example, esterification or etherification. Examples of groups suitable for this esterification include lower alkanoyl groups such as acetyl group, aroyl groups such as benzoyl group, groups derived from carbonic acid, such as benzyloxycarbonyl group and ethoxycarbonyl group, and the like. As examples of groups suitable for the etherification, benzyl group, tetrahydropyranyl group, t-butyl group and the like can be mentioned.
Examples of the protecting group for the phenolic hydroxyl group of tyrosine include Bzl, $Cl_2$-Bzl, 2-nitrobenzyl, Br-Z, tertiary butyl and the like.
Examples of the protecting group for the imidazole moiety of histidine include Tos, 4-methoxy-2,3,6-trimethylbenzenesulfonyl, DNP, benzyloxymethyl, Bum, Boc, Trt, Fmoc and the like.

As examples of the method used to remove (split off) the protecting group, catalytic reduction in a hydrogen gas stream in the presence of a catalyst such as Pd-black or Pd-carbon; acid treatment with anhydrous hydrogen fluoride, methanesulfonic acid, trifluoromethanesulfonic acid or trifluoroacetic acid, or a mixed solution thereof; treatment with a base such as diisopropylethylamine, triethylamine, piperidine or piperazine; reduction with sodium in liquid ammonia, and the like can be mentioned. The reaction of splitting of the protecting group by the above-described acid treatment is normally performed at a temperature of about -20°C to 40°C; in the acid treatment, addition of a cation scavenger such as anisole, phenol, thioanisole, meta-cresol, para-cresol, dimethylsulfide, 1,4-butanedithiol or 1,2-ethanedithiol is effective. The 2,4-dinitrophenyl group used as the protecting group for the imidazole moiety of histidine is removed by thiophenol treatment; the formyl group used as the protecting group for the indole moiety of tryptophan is removed by alkali treatment with dilute sodium hydroxide solution, dilute ammonia or the like, as well as by the above-described acid treatment in the presence of 1,2-ethanedithiol, 1,4-butanedithiol or the like.

Examples of activated carboxyl groups in the starting material include corresponding acid anhydrides, azides, activated esters (esters with alcohols (for example, pentachlorophenol, 2,4,5-trichlorophenol, 2,4-dinitrophenol, cyanomethyl alcohol, para-nitrophenol, HONB, N-hydroxysuccimide, N-hydroxyphthalimide, HOBt)]. Examples of activated amino groups in the starting material include corresponding phosphoric amides.

In another method of obtaining an amide of the protein (peptide), for example, the α-carboxyl group of the carboxy-terminal amino acid is first protected by amidation, and the peptide chain on the amino group side is then extended to a desired length; thereafter, a protein (peptide) having only the protecting group for the N-terminal α-amino group in the peptide chain removed and a protein (peptide) having only the protecting group for the C-terminal carboxyl group removed are prepared, and the two proteins (peptides) are condensed in a mixed solvent as described above. Details of the condensation reaction are the same as those described above. After the protected protein (protected peptide) obtained by the condensation is purified, all the protecting groups are removed by the above-described method to give the desired crude protein (crude peptide). This crude protein (crude peptide) may be purified by various known means of purification, and the major fraction may be lyophilized to give an amide of the desired protein (peptide).
An ester of the protein (peptide) can be obtained by, for example, condensing the α-carboxyl group of the carboxy-terminal amino acid with a desired alcohol to prepare an amino acid ester, and then following the same procedures as those for the above-described amide of the protein (peptide).

The partial peptide of the present invention or a salt thereof can also be produced by cleaving NPR3 or a salt thereof with an appropriate peptidase.

Furthermore, an NPR3 species can also be produced by culturing a transformant comprising a nucleic acid encoding NPR3 or a partial peptide thereof, and separating and purifying the NPR3 species from the culture obtained. The nucleic acid encoding NPR3 or a partial peptide thereof may be a DNA or RNA, or may be a DNA/RNA chimera. The nucleic acid is preferably a DNA. Also, the nucleic acid may be double stranded or single stranded. When the nucleic acid is double stranded, it may be a double-stranded DNA, a double-stranded RNA or a DNA:RNA hybrid. When the nucleic acid is single stranded, it may be a sense strand (i.e., code strand) or an antisense strand (i.e., non-code strand).
As the DNA encoding NPR3 or a partial peptide thereof, genomic DNAs, genomic DNA libraries, cDNAs derived from any cells [for example, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, adipocytes, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells, or corresponding precursor cells, stem cells, cancer cells, and the like], or any tissues where such cells are present [for example, brain, brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, lung, gastrointestinal organs (e.g., large intestine, small intestine), blood vessels, heart, thymus, spleen, submandibular gland, peripheral blood, prostate, testis, ovary, placenta, uterus, bone, joints, adipose tissues (e.g., brown adipose tissue, white adipose tissue), skeletal muscles and the like] of mammals (for example, humans, cattle, monkeys, horses, swine, sheep, goat, dogs, cats, guinea pigs, rats, mice, rabbits, hamsters and the like), synthetic DNAs and the like can be mentioned. Genomic DNA and cDNA encoding NPR3 or a partial peptide thereof can also be directly amplified by Polymerase Chain Reaction (hereinafter to be abbreviated as "PCR method") and Reverse Transcriptase-PCR (hereinafter to be abbreviated as "RT-PCR method") and using, as respective templates, genomic DNA fraction and total RNA or mRNA fraction prepared from the above-mentioned cell or tissue. Alternatively, genomic DNA and cDNA encoding NPR3 or a partial peptide thereof can also be cloned from genome DNA library and cDNA library prepared by inserting fragments of genomic DNA fraction and total RNA or mRNA fraction prepared from the above-mentioned cell or tissue into a suitable vector by colony or plaque hybridization method, PCR method and the like. The vector used for the library may be any of bacteriophage, plasmid, cosmid, phagemid and the like.

As examples of the DNA encoding NPR3, a DNA encoding a protein comprising the base sequence shown by SEQ ID NO:1, a DNA comprising a base sequence that hybridizes with the base sequence shown by SEQ ID NO:1 under high stringent conditions, and having substantially the aforementioned same quality of activity as NPR3 (e.g., binding activity to musclin, signal transmission activity and the like), and the like can be mentioned.

As examples of the DNA capable of hybridizing under high stringent conditions with the base sequence shown by SEQ ID NO:1, a DNA comprising a base sequence having a homology of about 50% or more, preferably about 60% or more, more preferably about 70% or more, particularly preferably about 80% or more, and most preferably about 90% or more, to the base sequence shown by SEQ ID NO:1 and the like can be used.

The homology of the base sequence in the present specification can be calculated using the homology calculation algorithm NCBI BLAST (National Center for Biotechnology Information Basic Local Alignment Search Tool) and under the following conditions (expectancy =10; allowing gap; filtering=ON; match score=1; mismatch score=-3). As other algorithm with which to determine the homology of the base sequence, the homology calculation algorithm of the above-mentioned amino acid sequence can be preferably used in the same manner.

The hybridization can be performed by a method known per se or a method based thereon, for example, the method described in Molecular Cloning, 2nd edition (J. Sambrook et al., Cold Spring Harbor Lab. Press, 1989) and the like. When a commercially available library is used, the hybridization can be performed according to the method described in the instruction manual attached. The hybridization can be performed preferably under highly stringent conditions.

As examples of the highly stringent conditions, conditions of a sodium salt concentration of about 19 mM to about 40 mM, preferably about 19 mM to about 20 mM, and a temperature of about 50°C to about 70°C, preferably about 60°C to about 65°C, and the like can be mentioned. In particular, the case of a sodium salt concentration of about 19 mM and a temperature of about 65°C is preferred.

The DNA that encodes NPR3 is preferably a DNA comprising a base sequence that encodes the human NPR3 protein (GenBank accession number: NM_000908), which is shown by the base sequence shown by SEQ ID NO:1, or a homologue thereof in another mammal (for example, rat, mouse and bovine homologues registered at GenBank under accession numbers NM_012868, NM_008728 and NM_174127, respectively (having an identify of about 87%, about 86% or about 89%, to human NPR3 cDNA, respectively) and the like).

The DNA encoding the partial peptide of the present invention may be any one, as long as it comprises the base sequence encoding a peptide comprising the same or substantially the same amino acid sequence as a portion of the amino acid sequence shown by SEQ ID NO:2. The DNA encoding the partial peptide of the present invention may also be any of genomic DNAs, genomic DNA libraries, cDNAs derived from the above-described cells/tissue, cDNA libraries derived from the above-described cells/tissue, and synthetic DNAs. The vector used for the library may be any of bacteriophage, plasmid, cosmid, phagemid and the like. The DNA encoding SS169 or a partial peptide thereof can also be amplified directly by the RT-PCR method using an mRNA fraction prepared from the above-described cells/tissues.

Specifically, as examples of the DNA encoding the partial peptide of the present invention, (1) a DNA comprising a partial base sequence of the base sequence shown by SEQ ID NO:1, or (2) a DNA having a base sequence that hybridizes under high stringent conditions with a DNA having the base sequence shown by SEQ ID NO:1, and encoding a peptide having substantially the aforementioned same quality of activity as NPR3 (e.g., binding activity to musclin, signal transmission activity and the like) and the like can be used.

As examples of the DNA capable of hybridizing under high stringent conditions with the base sequence shown by SEQ ID NO:1, a DNA comprising a base sequence having a homology of about 60% or more, preferably about 70% or more, more preferably about 80% or more, and most preferably about 85% or more, to the base sequence, and the like can be used.

The DNA encoding NPR3 or a partial peptide thereof can be cloned by amplification by the PCR method using a synthetic DNA primer having a portion of the base sequence encoding the NPR3 or a partial peptide thereof, or by hybridization of the DNA incorporated in an appropriate expression vector with a labeled DNA fragment or synthetic DNA encoding a portion or whole of the NPR3 protein. The hybridization can be performed according to, for example, the method described in Molecular Cloning, 2nd edition (ibidem) and the like. When a commercially available library is used, the hybridization can be performed according to the method described in the instruction manual attached to the library.

The base sequence of the DNA can be converted by a method known per se such as the ODA-LA PCR method, the Gapped duplex method, or the Kunkel method, or a method based thereon, using a known kit, for example, Mutan™-super Express Km (Takara Shuzo Co., Ltd.), Mutan™-K (Takara Shuzo Co., Ltd.) and the like.

The cloned DNA can be used as is or, if desired, after digestion with a restriction enzyme or addition of a linker, depending on the purpose of use. The DNA may have ATG as a translation initiation codon at the 5' end thereof, and may have TAA, TGA or TAG as a translation termination codon at the 3' end thereof. These translation initiation and termination codons may be added using an appropriate synthetic DNA adapter.

An expression vector comprising the DNA encoding NPR3 or a partial peptide thereof can be produced by, for example, excising the desired DNA fragment from the DNA encoding NPR3, and joining the DNA fragment downstream of a promoter in an appropriate expression vector.

As the expression vector, plasmids derived from *Escherichia coli* (e.g., pBR322, pBR325, pUC12 pUC13); plasmids derived from *Bacillus subtilis* (e.g., pUB110, pTP5, pC194); plasmids derived from yeast (e.g., pSH19, pSH15); insect cell expression plasmids (e.g., pFast-Bac); animal cell expression plasmids (e.g., pA1-11, pXT1, pRc/CMV, pRc/RSV, pcDNAI/Neo); bacteriophages such as λ phage; insect virus vectors such as baculovirus (e.g., BmNPV, AcNPV); animal virus vectors such as retrovirus, vaccinia virus, and adenovirus, and the like can be used.

The promoter may be any promoter that matches well with the host used for gene expression.

For example, when the host is an animal cell, the SRα promoter, the SV40 promoter, the LTR promoter, the CMV (cytomegalovirus) promoter, the RSV (Rous sarcoma virus) promoter, the MoMuLV (Moloney mouse leukemia virus) LTR, the HSV-TK (herpes simplex virus thymidine kinase) promoter and the like can be used. In particular, the CMV promoter, the SRα promoter and the like are preferable.

When the host is a bacterium of the genus *Escherichia,* the trp promoter, the lac promoter, the recA promoter, the $\lambda P_L$ promoter, the lpp promoter, the T7 promoter and the like are preferable.

When the host is a bacterium of the genus *Bacillus,* the SPO1 promoter, the SPO2 promoter, the penP promoter and the like are preferable.

When the host is a yeast, the PHO5 promoter, the PGK promoter, the GAP promoter, the ADH promoter and the like are preferable.

When the host is an insect cell, the polyhedrin promoter, the P10 promoter and the like are preferable.

As the expression vector, one optionally comprising an enhancer, a splicing signal, a poly A-addition signal, a selection marker, an SV40 replication origin (hereinafter sometimes abbreviated as SV40 ori) and the like, in addition to the above-described examples, can be used. As examples of the selection marker, the dihydrofolate reductase gene (hereinafter sometimes abbreviated as dhfr, methotrexate (MTX) resistance), the ampicillin resistance gene (hereinafter sometimes abbreviated as $amp^r$), the neomycin resistance gene (hereinafter sometimes abbreviated as $neo^r$, G418 resistance) and the like can be mentioned. In particular, when Chinese hamster cells lacking the dhfr gene are used in combination with the dhfr gene as the selection marker, it is also possible to select the desired gene on a thymidine-free medium.

Also, a base sequence encoding a signal sequence (signal codon) that matches with the host may be added as necessary to the 5' end side of the DNA encoding NPR3 or a partial peptide thereof (or substituted with a native signal codon). For example, when the host is a bacterium of the genus *Escherichia,* a PhoA signal sequence, a OmpA signal sequence and the like can be used; when the host is a bacterium of the genus *Bacillus,* an α-amylase signal sequence, a subtilisin signal sequence and the like can be used; when the host is a yeast, an MFα signal sequence, an SUC2 signal sequence and the like can be used; when the host is an animal cell, an insulin signal sequence, an α-interferon signal sequence, an antibody molecule signal sequence and the like can be used.

By transforming the host with the above-described expression vector comprising the DNA encoding NPR3 or a partial peptide thereof, and culturing the transformant obtained, an NPR3 species can be produced.

As examples of the host used, bacteria of the genus *Escherichia,* bacteria of the genus *Bacillus,* yeasts, insect cells, insects, animal cells and the like can be mentioned.

As examples of the bacteria of the genus *Escherichia, Escherichia coli* K12•DH1 [Proceedings of the National Academy of Sciences of the USA (Proc. Natl. Acad. Sci. USA), Vol. 60, 160 (1968)], *Escherichia coli* JM103 [Nucleic Acids Research, Vol. 9, 309 (1981)], *Escherichia coli* JA221 [Journal of Molecular Biology, Vol. 120, 517 (1978)], *Escherichia coli* HB101 [Journal of Molecular Biology, Vol. 41, 459 (1969)], *Escherichia coli* C600 [Genetics, Vol. 39, 440 (1954)] and the like can be used.

As examples of the bacteria of the genus *Bacillus, Bacillus subtilis* MI114 [Gene, Vol. 24, 255 (1983)], *Bacillus subtilis* 207-21 [Journal of Biochemistry, Vol. 95, 87 (1984)] and the like can be used.
As examples of the yeasts, *Saccharomyces cerevisiae* AH22, AH22R$^-$, NA87-11A, DKD-5D, and 20B-12, *Schizosaccharomyces pombe* NCYC1913 and NCYC2036, *Pichia pastoris* KM71 and the like can be used.

As the insect cells, an established cell line derived from the cabbage armyworm (*Spodoptera frugiperda* cells; Sf cells), MG1 cells derived from the *Trichoplusia ni* mid-intestine, High Five™ cells derived from *Trichoplusia ni* eggs, cells derived from *Mamestra brassicae,* cells derived from Estigmena acrea, and the like can be used when the virus is AcNPV, for example. When the virus is BmNPV, an established cell line derived from *Bombyx mori* (*Bombyx mori* N cells; BmN cells) and the like can be used as the insect cells. As examples of the Sf cells, Sf9 cells (ATCC CRL1711), Sf21 cells (both types of cells are described in Vaughn, J.L. et al., In Vivo, 13, 213-217 (1977)) and the like can be used.
As examples of the insects, *Bombyx mori* larvae and the like can be used [Maeda et al., Nature, Vol. 315, 592 (1985)].

As examples of the animal cells, monkey COS-7 cells, monkey Vero cells, Chinese hamster ovary cells (hereinafter abbreviated as CHO cells), CHO cells lacking the dhfr gene (hereinafter abbreviated as CHO(dhfr$^-$) cells), mouse L cells, mouse AtT-20 cells, mouse myeloma cells, rat GH3 cells, human FL cells and the like can be used.

Transformation can be performed according to a known method depending on the kind of the host.
Bacteria of the genus *Escherichia* can be transformed according to, for example, the methods described in Proceedings of the National Academy of Sciences of the USA (Proc. Natl. Acad. Sci. U.S.A.), Vol. 69, 2110 (1972), Gene, Vol. 17, 107 (1982) and the like.
Bacteria of the genus *Bacillus* can be transformed according to, for example, the methods described in Molecular & General Genetics, Vol. 168, 111 (1979) and the like.
Yeasts can be transformed according to, for example, the methods described in Methods in Enzymology, Vol. 194, 182-187 (1991), Proceedings of the National Academy of Sciences of the USA (Proc. Natl. Acad. Sci. U.S.A.), Vol. 75, 1929 (1978) and the like.
Insect cells and insects can be transformed according to, for example, the methods described in Bio/Technology, Vol. 6,47-55 (1988) and the like.
Animal cells can be transformed, for example, according to the methods described in Saibo Kogaku (Cell Engineering), extra issue 8, Shin Saibo Kogaku Jikken Protocol (New Cell Engineering Experimental Protocol), 263-267 (1995), published by Shujunsha, and Virology, Vol. 52, 456 (1973).

Cultivation of a transformant can be performed according to a known method depending on the kind of the host.
For example, when culturing a transformant whose host is a bacterium of the genus *Escherichia* or *Bacillus,* the medium used for cultivation is preferably a liquid medium. The medium preferably contains substances required for the growth of the transformant, such as carbon sources, nitrogen sources, and inorganic substances. As examples of the carbon sources, glucose, dextrin, soluble starch, sucrose and the like can be mentioned; as examples of the nitrogen sources, inorganic or organic substances such as ammonium salts, nitrate salts, corn steep liquor, peptone, casein, meat extract, soybean cake, and potato extract can be mentioned; as examples of the inorganic substances, calcium chloride, sodium dihydrogenphosphate, magnesium chloride and the like can be mentioned. In addition, yeast extract, vitamins, growth promoting factors and the like may be added to the medium. The pH of the medium is preferably about 5 to about 8.
The medium for cultivation of a transformant whose host is a bacterium of the genus *Escherichia* is preferably, for example, an M9 medium containing glucose and Casamino acids [Miller, Journal of Experiments in Molecular Genetics, 431-433, Cold Spring Harbor Laboratory, New York, 1972]. Chemicals such as 3β-indolylacrylic acid may be added to the medium as necessary to allow the promoter to function efficiently.
Cultivation of a transformant whose host is a bacterium of the genus *Escherichia* is normally performed at about 15°C to about 43°C for about 3 to about 24 hours. The culture may be aerated or agitated as necessary.
Cultivation of a transformant whose host is a bacterium of the genus *Bacillus* is normally performed at about 30°C to about 40°C for about 6 to about 24 hours. The culture may be aerated or agitated as necessary.
As examples of the medium for culturing a transformant whose host is a yeast, Burkholder's minimal medium [Bostian, K.L. et al., Proceedings of the National Academy of Sciences of the USA (Proc. Natl. Acad. Sci. USA), Vol. 77, 4505 (1980)], an SD medium containing 0.5% Casamino acid [Bitter, G.A. et al., Proceedings of the National Academy of Sciences of the USA (Proc. Natl. Acad. Sci. USA), Vol. 81, 5330 (1984)] and the like can be mentioned. The pH of the medium is preferably about 5 to about 8. Cultivation is normally performed at about 20°C to about 35°C for about 24 to about 72 hours. The culture may be aerated or agitated as necessary.
As examples of the medium for culturing a transformant whose host is an insect cell or an insect, Grace's Insect Medium [Grace, T.C.C., Nature, Vol. 195, 788 (1962)] having additives such as 10% inactivated bovine serum added thereto as appropriate, and the like can be used. The pH of the medium is preferably about 6.2 to about 6.4. Cultivation is normally performed at about 27°C for about 3 to about 5 days. The culture may be aerated or agitated as necessary.
As examples of the medium for culturing a transformant whose host is an animal cell, a minimal essential medium (MEM) containing about 5% to about 20% fetal bovine serum [Science, Vol. 122, 501 (1952)], Dulbecco's modified Eagle's medium (DMEM) [Virology, Vol. 8, 396 (1959)], RPMI 1640 medium [The Journal of the American Medical Association, Vol. 199, 519 (1967)], 199 medium [Proceeding of the Society for the Biological Medicine, Vol. 73, 1 (1950)] and the like can be used. The pH of the medium is preferably about 6 to about 8. Cultivation is normally performed at about 30°C to about 40°C for about 15 to about 60 hours. The culture may be aerated or agitated as necessary.

As described above, an NPR3 species can be produced in or outside the cells of a transformant.

The NPR3 species can be separated and purified from the culture obtained by culturing the aforementioned transformant according to a method known per se.

For example, when the NPR3 species is extracted from the cytoplasm of cultured cells, a method that comprises suspending the cells collected from the culture by a known method in an appropriate buffer, disrupting the cells by ultrasonication, lysozyme and/or freeze-thawing and the like, and performing centrifugation and filtration, to yield a crude extract of the soluble protein, and the like can be used as appropriate. The buffer may contain a protein denaturant such as urea or guanidine hydrochloride and a surfactant such as Triton X-100™. On the other hand, when the NPR3 species is extracted from a membrane fraction, a method that comprises disrupting cells in the same manner as described above, performing low-speed centrifugation to precipitate and remove cell debris, and centrifuging the supernatant at a high speed to precipitate a fraction containing the cell membrane (the cell membrane fraction is purified by density gradient centrifugation and the like as necessary) and the like can be used. When the NPR3 species is secreted outside the cells, a method that comprises separating the culture supernatant from the culture by centrifugation, filtration or the like, and the like can be used.

The NPR3 species contained in the soluble fraction, membrane fraction or culture supernatant thus obtained can be isolated and purified according to a method known per se. Such methods include methods based on differences in solubility, such as salting-out and solvent precipitation; methods based mainly on differences in molecular weight, such as dialysis, ultrafiltration, gel filtration, and SDS-polyacrylamide gel electrophoresis; methods based on differences in electric charge, such as ion exchange chromatography; methods based on specific affinity, such as affinity chromatography; methods based on differences in hydrophobicity, such as reverse phase high performance liquid chromatography; methods based on differences in isoelectric point, such as isoelectric focusing; and the like. These methods may be combined as appropriate.

When the protein or peptide thus obtained is in the free form, the free form can be converted into a salt by a method known per se or a method based thereon; when the protein or peptide is obtained in the form of a salt, the salt can be converted into the free form or another salt by a method known per se or a method based thereon.

The NPR3 species produced by the transformant can be treated with a suitable protein-modifying enzyme before or after the purification, so as to make an optionally chosen modification or to partially remove a polypeptide. As examples of the protein-modifying enzyme used, trypsin, chymotrypsin, arginyl endopeptidase, protein kinase, glycosidase and the like can be mentioned.

The presence of the NPR3 species thus obtained can be confirmed by an enzyme immunoassay, Western blotting and the like using a specific antibody.

Furthermore, an NPR3 species can be synthesized in vitro using a cell-free protein translation system comprising rabbit reticulocyte lysate, wheat germ lysate, *Escherichia coli* lysate or the like, with an RNA corresponding to the DNA encoding the above-described NPR3 or a partial peptide thereof as the template. Alternatively, an NPR3 species can also be synthesized using a cell-free transcription/translation system further comprising RNA polymerase, with the DNA encoding NPR3 or a partial peptide thereof as the template.

A nucleic acid comprising the base sequence encoding the NPR3 protein, i.e., a protein comprising the same or substantially the same amino acid sequence as the amino acid sequence shown by SEQ ID NO:2, or a portion thereof, or a base sequence complementary to the base sequence or a portion thereof, is meant to include not only the above-described nucleic acid encoding NPR3 or a partial peptide thereof, but also base sequences comprising a mismatch frame.

A nucleic acid comprising a base sequence complementary to the target region of the desired nucleic acid, i.e., a nucleic acid capable of hybridizing with the desired nucleic acid, can be said to be "antisense" against the desired nucleic acid. On the other hand, a nucleic acid comprising a base sequence having a homology to the target region of the desired nucleic acid can be said to be "sense" against the target nucleic acid. As used herein, "having a horology" or "(being) complementary" means having a homology or complementarity of about 70% or more, preferably about 80% or more, more preferably about 90% or more, and most preferably about 95% or more, between the base sequences.

A nucleic acid comprising a base sequence complementary to the base sequence encoding the NPR3 protein or a portion thereof (hereinafter also referred to as "the antisense nucleic acid of the present invention,") can be designed and synthesized on the basis of the base sequence information of a cloned or sequenced NPR3-encoding nucleic acid. Such nucleic acids are capable of inhibiting the replication or expression of the NPR3 gene. Hence, the antisense nucleic acid of the present invention is capable of hybridizing with the RNA transcribed from the NPR3 gene, and of inhibiting the synthesis (processing) or function (translation into protein) of mRNA.

The target region of the antisense nucleic acid of the present invention is not subject to limitation as to length, as long as hybridization of the antisense nucleic acid results in the inhibition of the translation of the NPR3 protein, and may be the whole sequence or a partial sequence of NPR3 RNA; for example, about 15 bases for the shortest and the whole sequence of mRNA or the initial transcription product for the longest can be mentioned. Considering the issues of the ease of synthesis and antigenicity, an oligonucleotide comprising about 15 to about 30 bases is preferred, but the length is not limited thereto. Specifically, for example, the 5'-end hairpin loop, 5'-end 6-base-pair repeat, 5'-end untranslated region, polypeptide translation initiation codon, protein coding region, ORF translation initiation codon, 3'-end untranslated region, 3'-end palindrome region, and 3'-end hairpin loop of the nucleic acid encoding the NPR3 precursor polypeptide, may be selected as the target region, but any other region within the NPR3 gene may also be selected as the target. For example, it is also preferable that the intron portion of the gene be the target region.
Furthermore, the antisense nucleic acid of the present invention may be capable of inhibiting RNA transcription by binding with the NPR3 gene, which is a double-stranded DNA, to form a triple strand (triplex, as well as inhibiting translation into protein by hybridizing with NPR3 mRNA or the initial transcription product.

Examples of the antisense nucleic acid include deoxypolynucleotides comprising 2-deoxy-D-ribose, ribonucleotides comprising D-ribose, other types of nucleotides which are N-glycosides of the purine or pyrimidine base, or other polymers having a non-nucleotide backbone (for example, commercially available nucleic acid polymers specific for protein nucleic acids and synthetic sequences) or other polymers comprising a special linkage (provided that the polymers comprise nucleotides having such an alignment that allows base pairing or base attachment, as found in DNA or RNA) and the like. These may be double-stranded DNAs; single-stranded DNAs, double-stranded RNAs, single-stranded RNAs, or DNA:RNA hybrids, and may also be unmodified polynucleotides (or unmodified oligonucleotides); those with known modifications, for example, those with labels known in the art, those with caps, those methylated, those with substitution of one or more naturally occurring nucleotides with their analogue, those with intramolecular modifications of nucleotides such as those with uncharged linkages (for example, methyl phosphonates, phosphotriesters, phosphoramidates, carbamates and the like) and those with charged linkages or sulfur-containing linkages (for example, phosphorothioates, phosphorodithioates and the like); those having side chain groups such as proteins (nucleases, nuclease inhibitors, toxins, antibodies, signal peptides, poly-L-lysine and the like) or saccharides (for example, monosaccharides and the like); those with intercalators (for example, acridine, psoralen and the like); those with chelators (for example, metals, radioactive metals, boron, oxidative metals and the like); those with alkylating agents; or those with modified linkages (for example, $\alpha$ anomeric nucleic acids and the like). As used herein, "nucleoside", "nucleotide" and "nucleic acid" may comprise not only the purine and pyrimidine bases, but also other modified heterocyclic bases. Such modified produced may comprise a methylated purine and pyrimidine, acylated purine and pyrimidine, and another heterocyclic ring. The modified nucleotide and modified nucleotide may have a modification in the sugar moiety thereof; for example, one or more hydroxyl groups may be substituted with halogens, aliphatic groups and the like, or may be converted into functional groups such as ethers and amines.

The antisense nucleic acid is an RNA, a DNA, or a modified nucleic acid (RNA, DNA). Specific examples of the modified nucleic acid include, but are not limited to, sulfur and thiophosphate derivatives of nucleic acids and those resistant to degradation of polynucleoside amides and oligonucleoside amides. The antisense nucleic acid of the present invention can be designed preferably on the following plan, that is, to increase the intracellular stability of the antisense nucleic acid, to increase the cell permeability of the antisense nucleic acid, to increase the affinity for the targeted sense strand, and to reduce the toxicity, if any, of the antisense nucleic acid. Many such modifications are known in the art, as disclosed in, for example, J. Kawakami, et al., Pharm. Tech. Japan, Vol. 8, p. 247, 1992; Vol. 8, p. 395, 1992; S. T. Crooke et al. ed., Antisense Research and Applications, CRC Press, 1993; and the like.

The antisense nucleic acid may comprise an altered or modified sugar, base, and bond, and can be supplied in a special form such as liposomes or microspheres, can be applied to gene therapy, and can be given in the form of an adduct. As substances for the adduct, polycations such as polylysine, which acts to neutralize the electric charge of the phosphate group backbone, and hydrophobic substances such as lipids that enhance the interaction with the cell membrane or increase nucleic acid uptake (for example, phospholipids, cholesterols and the like) can be mentioned. As lipids preferred for the adduct, cholesterol and derivatives thereof (for example, cholesteryl chloroformate, cholic acid and the like) can be mentioned. These substances may be attached to the 3' or 5' end of the nucleic acid, and can be attached via a base, sugar, or intramolecular nucleoside bond. As other groups, capping groups specifically placed at the 3' or 5' end of the nucleic acid to prevent its degradation by nucleases such as exonuclease and RNase, can be mentioned. Such capping groups include, but are not limited to, hydroxyl group protecting groups known in the art, including glycols such as polyethylene glycol and tetraethylene glycol.

Ribozymes capable of specifically cleaving NPR3 mRNA or the initial transcription product thereof inside the coding region (comprising the intron moiety in the case of the initial transcription product) can also be included in the antisense nucleic acid of the present invention. "A ribozyme" refers to an RNA having enzyme activity to cleave nucleic acids; since it has recently been shown that oligo-DNAs having the base sequence of the enzyme activity moiety likewise have nucleic acid cleavage activity, this term is used herein as a concept including DNAs, as long as they have sequence-specific nucleic acid cleavage activity. The most versatile ribozymes include self- splicing RNAs found in infectious RNAs such as viroid and virusoid, and are known to occur in the hammerhead type, the hairpin type and the like. The hammerhead type exhibits enzyme activity with about 40 bases, and is capable of specifically cleaving only the target mRNA by rendering several bases at each end adjacent to the hammerhead structure moiety (about 10 bases in total) a sequence complementary to the desired cleavage site of mRNA. Because this type of ribozyme utilizes only RNA as the substrate, it offers a further advantage of not attacking genomic DNA. When NPR3 MRNA. itself has a double strand structure, the target sequence can be made single-stranded by using a hybrid ribozyme joined with an RNA motif derived from a viral nucleic acid capable of specifically binding to RNA helicase [Proc. Natl. Acad. Sci. USA, 98(10): 5572-5577 (2001)]. Furthermore, when ribozyme is used in the form of an expression vector comprising the DNA encoding the ribozyme, it is also possible to make the ribozyme a hybrid ribozyme further joined with a sequence with tRNA modified to promote the transfer of the transcription product to cytoplasm [Nucleic Acids Res., 29(13): 2780-2788 (2001)].

Double-stranded oligo-RNAs (siRNAs) complementary to a partial sequence within the coding region of NPR3 mRNA or the initial transcription product thereof (comprising the intron moiety in the case of the initial transcription product) can also be included in the antisense nucleic acid of the present invention. What is called RNA interference (RNAi), the phenomenon in which introducing a short double-stranded RNA into cells results in the degradation of mRNAs complementary to the RNA, has been known to occur in nematodes, insects, plants and the like; since this phenomenon has recently been used to occur in mammalian cells as well [Nature, 411(6836): 494-498 (2001)], it is drawing attention as an alternative technique to ribozymes. siRNA can be designed as appropriate on the basis of the base sequence information on the mRNA to be a target, using a commercially available software program (e.g., RNAi Designer; Invitrogen).

The antisense oligonucleotide and ribozyme of the present invention can be prepared by determining the target region of mRNA or the initial transcription product thereof on the basis of NPR3 cDNA sequence or genomic DNA sequence information, and synthesizing a sequence complementary thereto using a commercially available automated DNA/RNA synthesizer (Applied Biosystems Company, Beckman Company and the like). siRNA having RNAi activity can be prepared by synthesizing a sense strand and an antisense strand, respectively, using an automated DNA/RNA synthesizer, denaturing them in an appropriate annealing buffer, for example, at about 90°C to about 95°C for about 1 minute, and annealing them at about 30°C to about 70°C for about 1 to about 8 hours. A longer double-stranded polynucleotide can be prepared by synthesizing complementary oligonucleotide strands to overlap alternately, annealing them, and ligating them using ligase.

The gene expression inhibitory activity of the antisense nucleic acid of the present invention can be examined using a transformant comprising the nucleic acid encoding NPR3, an in vivo or in vitro NPR3 gene expression system, or an in vivo or in vitro NPR3 protein translation system. The nucleic acid can be applied to cells by various methods known per se.

The present invention also provides an antibody against NPR3 or a partial peptide thereof or a salt thereof. The antibody may be a monoclonal antibody or a polyclonal antibody, as long as it has specific affinity for an NPR3 species. An antibody against an NPR3 species can be produced using the NPR3 species as the antigen according to a method of antibody or antiserum production known per se.

[Preparation of monoclonal antibody]

(a) Preparation of cells producing a monoclonal antibody

An NPR3 species is administered as is, or along with a carrier or a diluent, to a mammal at a site enabling antibody production by its administration. In order to increase antibody productivity upon the administration, Freund's complete adjuvant or Freund's incomplete adjuvant may be administered. Dosing is normally performed about two to 10 times in total every 2 to 6 weeks. As examples of the mammal used, monkeys, rabbits, dogs, guinea pigs, mice, rats, sheep, and goat can be mentioned, with preference given to mice and rats.

In preparing cells producing a monoclonal antibody, an individual showing an antibody titer is selected from among mammals, e.g., mice, immunized with an antigen, the spleen or lymph node is collected 2 to 5 days after final immunization, and antibody-producing cells contained therein are fused with myeloma cells, whereby a monoclonal antibody-producing hybridoma can be prepared. A measurement of antibody titer in an antiserum can be performed by, for example, reacting an NPR3 species labeled as described below with the antiserum, then determining the activity of labeling agent bound to the antibody. The fusion can be performed according to a known method, for example, the method of Koehler and Milstein [Nature, Vol. 256, p. 495 (1975)]. As examples of the fusogen, polyethylene glycol (PEG), Sendai virus and the like can be mentioned, with preference given to PEG.

As examples of the myeloma cells, NS-1, P3U1, SP2/0 and the like can be mentioned, with preference given to P3U1. The ratio by number of antibody-producing cells (splenocytes) and myeloma cells is preferably about 1:1 to 20:1; cell fusion can be efficiently performed by adding PEG (preferably PEG1000 to PEG6000) at a concentration of about 10% to 80%, and incubating the cells at about 20°C to 40°C, preferably about 30°C to 37°C, for about 1 to 10 minutes.

Various methods can be used for screening a monoclonal antibody-producing hybridoma; for example, a method that comprises adding a hybridoma culture supernatant to a solid phase (e.g., microplate) having an antigen such as a protein, directly or along with a carrier, adsorbed thereto, then adding an anti-immunoglobulin antibody (an anti-mouse immunoglobulin antibody is used when mouse cells are used for the cell fusion) labeled with a radioactive substance, an enzyme or the like, or Protein A, and detecting the monoclonal antibody bound to the solid phase; a method that comprises adding a hybridoma culture supernatant to a solid phase having an anti-immunoglobulin antibody or Protein A adsorbed thereto, adding a protein or the like labeled with a radioactive substance, an enzyme or the like, and detecting the monoclonal antibody bound to the solid phase; and the like can be mentioned.
Monoclonal antibodies can be selected according to a method known per se or a method based thereon; this selection can normally be achieved using a medium for animal cells supplemented with HAT (hypoxanthine, aminopterin, thymidine) and the like. Any selection and breeding medium can be used, as long as it allows the hybridoma to grow. For example, an RPMI 1640 medium containing 1% to 20%, preferably 10% to 20%, fetal calf serum, a GIT medium (Wako Pure Chemical Industries, Ltd.) containing 1% to 10% fetal calf serum, a serum free medium for hybridoma culture (SFM-101, Nissui Seiyaku Co., Ltd.) and the like can be used. Cultivation temperature is normally 20°C to 40°C, preferably about 37°C. Cultivation time is normally 5 days to 3 weeks, preferably 1 week to 2 weeks. The cultivation may be performed normally in the presence of 5% gaseous carbon dioxide. The antibody titer of hybridoma culture supernatant can be determined in the same manner as the above-described determination of antibody titer in antiserum.

(b) Purification of monoclonal antibody

Separation and purification of the monoclonal antibody can be performed according to a method of immunoglobulin separation and purification, as in ordinary separation and purification of polyclonal antibody [e.g., salting-out, alcohol precipitation, isoelectric point precipitation, electrophoresis, adsorption and desorption with ion exchangers (e.g., DEAE), ultracentrifugation, gel filtration, specific purification comprising collecting only the antibody using an activated adsorbent such as an antigen-bound solid phase, Protein A, or Protein G, and dissociating the linkage to separate the desired antibody].

[Preparation of polyclonal antibody]

The polyclonal antibody of the present invention can be produced according to a method known per se or a method based thereon. For example, the polyclonal antibody of the present invention can be produced by forming a complex of an immunoantigen (an antigen such as a protein) and a carrier protein, immunizing a mammal with the complex in the same manner as the above-described method of monoclonal antibody production, collecting a product containing an antibody against an NPR3 species from the immunized animal, and separating and purifying the antibody.
Regarding the complex of immunoantigen and carrier protein used to immunize a mammal, the kind of carrier protein and the mixing ratio of carrier and hapten may be any ones whatever they are, as long as an antibody against the immunizing hapten crosslinked to the carrier is efficiently produced; for example, a method that comprises coupling bovine serum albumin, bovine thyroglobulin, keyhole limpet hemocyanin or the like to the hapten in a ratio by weight of about 0.1 to 20, preferably about 1 to 5, to 1 of hapten, can be used.
Various condensing agents can be used for the coupling of hapten and carrier; glutaraldehyde, carbodiimide, maleimide activated ester, activated ester reagents comprising the thiol group or the dithiopyridyl group, and the like can be used.
The condensation product is administered as is, or along with a carrier or a diluent, to a mammal at a site enabling antibody production by its administration. In order to increase antibody productivity upon the administration, Freund's complete adjuvant or Freund's incomplete adjuvant may be administered. Dosing is normally performed about three to 10 times in total every 2 to 6 weeks.
The polyclonal antibody can be collected from blood, ascites fluid and the like, preferably blood, of a mammal immunized by the above-described method.
Polyclonal antibody titer in antiserum can be determined in the same manner as the above-described determination of antibody titer in serum. Separation and purification of the polyclonal antibody can be performed according to a method of immunoglobulin separation and purification, as in the above-described separation and purification of monoclonal antibody.
NPR3 functions as a receptor of musclin. Therefore, an NPR3 species, a nucleic acid that encodes NPR3 or a portion thereof, an antibody against an NPR3 species (hereinafter sometimes referred to as "an antibody of the present invention"), and an antisense nucleic acid against a nucleic acid that encodes NPR3 (hereinafter sometimes referred to as "an antisense nucleic acid of the present invention") have the applications shown below.

(1) Cell differentiation and/or metabolism regulator

Because musclin is secretorily expressed nearly specifically in skeletal muscles and highly expressed in diabetes model mice and post-fasting re-feeding, because the expression thereof is influenced by insulin and influences the action of insulin, and also because musclin is expressed in bone and can influence bone/cartilage metabolism, and for other reasons, it is evident that musclin plays an important role in the regulation of (i) cell differentiation and/or (ii) metabolism (for example, cell differentiation and/or metabolism (for example, sugar/lipid/protein metabolism) in the liver, fat tissue, skeletal muscles, pancreatic island, bone, cartilage and the like). Because NPR3 interacts with musclin to transduce signals concerning cell differentiation and/or metabolic regulation, a) an NPR3 species or b) a DNA that encodes an NPR3 species can be used as a regulator of cell differentiation and/or metabolism.
For example, for a patient in whom the physiological action of musclin, a ligand, is unexpectable because of a reduced NPR3 level in the body thereof (NPR3 deficiency), it is possible to increase the amount of NPR3 in the patient's body and allow musclin to act in full by a) administering an NPR3 species to the patient to replenish the amount of NPR3, or by b) (a) administering a DNA that encodes an NPR3 species to the patient and allowing the DNA to be expressed, or (b) introducing a DNA that encodes an NPR3 species into a subject cell and allowing the DNA to be expressed, and thereafter transplanting the cell to the patient, and the like. Accordingly, an NPR3 species and a DNA that encodes the same are useful as safe, less toxic prophylactic and/or therapeutic agents for a disease associated with NPR3 dysfunction. Diseases associated with NPR3 dysfunction include, but are not limited to, abnormalities of cell differentiation and/or metabolism, preferably abnormalities of cell differentiation and/or metabolism, preferably sugar/lipid/protein metabolism, in the liver, fat tissue, skeletal muscles, pancreatic island, bone, cartilage and the like; more specifically, for example, obesity, diabetes, impaired glucose tolerance, arteriosclerosis, hypertension, hyperlipemia, metabolic bone/cartilage diseases (e.g., osteoporosis, osteomalacia, rickets, fibrous osteisis, renal osteodystrophy, bone Behcet disease, ankylosing spondylitis, osteopetrosis, osteoarthritis, rheumatoid arthritis and the like) and the like.

A NPR3 species and a DNA that encode an NPR3 species (hereinafter sometimes referred to as "a DNA of the present invention") can be used as the above-described pharmaceutical after being mixed with a pharmacologically acceptable carrier to obtain a pharmaceutical composition as required.
As pharmacologically acceptable carriers, various organic or inorganic carrier substances conventionally used as preparation materials can be used, and blended as excipient, lubricant, binder, disintegrant for solid preparations; solvent, solubilizer, suspending agent, isotonizing agent, buffer, soothing agent for liquid preparations and the like. Where necessary, a preparation additive such as a preservative, an antioxidant, a colorant, a sweetener and the like can also be used.
Preferable examples of the excipient include lactose, sucrose, D-mannitol, D-sorbitol, starch, gelatinized starch, dextrin, crystalline cellulose, low-substituted hydroxypropylcellulose, sodium carboxymethylcellulose, gum arabic, pullulan, light anhydrous silicic acid, synthetic aluminum silicate, magnesium alumino metasilicate and the like.
Preferable examples of the lubricant include magnesium stearate, calcium stearate, talc, colloidal silica and the like.
Preferable examples of the binder include gelatinized starch, sucrose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose, polyvinylpyrrolidone and the like.
Preferable examples of the disintegrant include lactose, sucrose, starch, carboxymethylcellulose, calcium carboxymethylcellulose, croscarmellose sodium, sodium carboxymethyl starch, light anhydrous silicic acid, low-substituted hydroxypropylcellulose, and the like.
Preferable examples of the solvent include water for injection, saline, Ringer's solution, alcohol, propylene glycol, polyethylene glycol, sesame oil, corn oil, olive oil, cottonseed oil and the like.
Preferable examples of the solubilizers include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.
Preferable examples of the suspending agent include surfactant such as stearyltriethanolamine, sodium lauryl sulfate, lauryl aminopropionic acid, lecithin, benzalkonium chloride, benzethonium chloride, glycerol monostearate and the like; hydrophilic polymer such as polyvinyl alcohol, polyvinylpyrrolidone, sodium carboxymethylcellulose, methylcellulose, hydroxymethylcellulose, hydroxyethylcellulose, hydroxypropylcellulose and the like; polysorbates, polyoxyethylene hydrogenated castor oil and the like.
Preferable examples of the isotonizing agent include sodium chloride, glycerol, D-mannitol, D-sorbitol, glucose and the like.
Preferable examples of the buffering agent include buffer such as phosphate, acetate, carbonate, citrate etc. and the like.
Preferable examples of the soothing agent include benzyl alcohol and the like.
Preferable examples of the preservative include paraoxybenzoates, chlorobutanol, benzyl alcohol, phenethyl alcohol, dehydroacetic acid, sorbic acid and the like.
Preferable examples of the antioxidant include sulfite, ascorbate and the like.
Preferable examples of the colorant include aqueous edible tar pigment (e.g., food colors such as Food Color Red Nos. 2 and 3, Food Color Yellow Nos. 4 and 5, Food Color Blue Nos. 1 and 2 etc.), water-insoluble lake dye (e.g., aluminum salt etc. of the aforementioned aqueous food tar color), natural dye (e.g., β-carotene, chlorophyll, red iron oxide etc.) and the like.
Preferable examples of the sweetening agent include saccharin sodium, dipotassium glycyrrhizinate, aspartame, stevia and the like.

Examples of the dosage form of the aforementioned pharmaceutical composition include oral preparations such as tablet, capsule (including soft capsule, microcapsule), granule, powder, syrup, emulsion, suspension and the like; and parenteral preparations such as injection (e.g., subcutaneous injection, intravenous injection, intramuscular injection, intraperitoneal injection etc.), external preparation (e.g., transnasal preparations, transdermal preparation, ointment etc.), suppository (e.g., rectal suppository, vaginal suppository), pellet, drip infusion, a release controlled preparation (e.g., sustained-release microcapsule) and the like.

These pharmaceutical compositions can be produced by a method in common use in the field of drug formulation technology, for example, methods described in the Japanese Pharmacopoeia and the like. Specific methods of preparing preparations are described in detail below. The content of an active ingredient in the pharmaceutical composition varies depending on dosage form, the dose of the active ingredient and the like, and is, for example, about 0.1 to 100% by weight.

For example, an oral preparation is produced by adding an excipient (e.g., lactose, sucrose, starch, D-mannitol and the like), a disintegrant (e.g., carboxymethylcellulose calcium and the like), a binder (e.g., $\alpha$-starch, gum arabic, carboxymethylcellulose, hydroxypropylcellulose, polyvinylpyrrolidone and the like), a lubricant (e.g., talc, magnesium stearate, polyethylene glycol 6000 and the like) and the like, to active ingredients, and compressing them, with coating performed, if necessary, using a coating base for the purpose of taste masking, enteric dissolution or sustained release, by a method known per se.

As examples of the coating base, sugar-coating bases, water-soluble film-coating bases, enteric film-coating bases, sustained-release film-coating bases and the like can be mentioned.

As the sugar-coating base, sucrose is used, which may be used in combination with one or two or more kinds selected from among talc, precipitated calcium carbonate, gelatin, gum arabic, pullulan, carnauba wax and the like.

As examples of the water-soluble film-coating base, cellulose polymers such as hydroxypropylcellulose, hydroxypropylmethylcellulose, hydroxyethylcellulose and methylhydroxyethylcellulose; synthetic polymers such as polyvinylacetal diethylaminoacetate, aminoalkylmethacrylate copolymer E [Eudragit E (trade name), Roehm Pharma GmbH], and polyvinylpyrrolidone; polysaccharides such as pullulan; and the like can be mentioned.

As examples of the enteric film-coating base, cellulose polymers such as hydroxypropylmethylcellulose phthalate, hydroxypropylmethylcellulose acetate succinate, carboxymethylethylcellulose, and cellulose acetate phthalate; acrylate polymers such as methacrylate copolymer L [Eudragit L (trade name), Roehm Pharma GmbH], methacrylate copolymer LD [Eudragit L-30D55 (trade name), Roehm Pharma GmbH], and methacrylate copolymer S [Eudragit S (trade name), Roehm Pharma GmbH]; naturally occurring substances such as shellac; and the like can be mentioned.

As examples of the sustained-release film-coating base, cellulose polymers such as ethylcellulose; acrylate polymers such as aminoalkylmethacrylate copolymer RS [Eudragit RS (trade name), Roehm Pharma GmbH] and ethyl acrylate/methyl methacrylate copolymer suspension [Eudragit NE (trade name), Roehm Pharma GmbH]; and the like can be mentioned.

The above-described coating bases may be used in a suitable mixture of two or more kinds thereof. Also, a light-blocking agent such as titanium oxide or iron sesquioxide may be used during coating.

An injection is produced by dissolving, suspending or emulsifying active ingredients, along with a dispersing agent (e.g., polysorbate 80, polyoxyethylene hydrogenated castor oil 60, polyethylene glycol, carboxymethylcellulose, sodium alginate and the like), a preservative (e.g., methylparaben, propylparaben, benzyl alcohol, chlorobutanol, phenol and the like), an isotonizing agent (e.g., sodium chloride, glycerin, D-mannitol, D-sorbitol, glucose and the like) and the like, in an aqueous solvent (e.g., distilled water, physiological saline, Ringer's solution and the like) or an oily solvent (e.g., vegetable oils such as olive oil, sesame oil, cottonseed oil and corn oil, propylene glycol and the like). If desired, additives such as a solubilizer (e.g., sodium salicylate, sodium acetate and the like), a stabilizer (e.g., human serum albumin and the like), and a soothing agent (e.g., benzyl alcohol and the like) may be used. The injection is usually filled in suitable ampoules.

Because the preparation thus obtained is safe and less toxic, it can be administered orally or non-orally to, for example, mammals (for example, humans, rats, rabbits, sheep, swine, cattle, cats, dogs, monkeys, and the like).

When a DNA of the present invention is used as the above-described prophylactic/therapeutic agent, the DNA of the present invention, alone or after being inserted into an appropriate expression vector such as a retrovirus vector, adenovirus vector, or adeno-associated viral vector, can be administered according to a conventional method. The DNA of the present invention can also be administered as it stands, or together with an auxiliary substance to promote its uptake, with a gene gun or a catheter such as a hydrogel catheter.

The dose of the NPR3 species varies depending on the subject of administration, target organ, symptoms, method of administration and the like; in oral administration, the dose is normally about 0.1 to 100 mg, preferably about 1.0 to 50 mg, and more preferably about 1.0 to 20 mg, per day, in, for example, a patient with a sugar/lipid metabolic abnormality (assuming a 60 kg body weight). In parenteral administration, the single dose varies depending on the subject of administration, target organ, symptoms, method of administration and the like; for example, in the form of an injection, the dose is normally about 0.01 to 30 mg, preferably about 0.1 to 20 mg, and more preferably about 0.1 to 10 mg, per day, in, for example, a patient with a sugar/lipid metabolic abnormality (assuming a 60 kg body weight). In cases where the subject of administration is other than a human, a dose corresponding to a human dose per 60 kg body weight can be administered.
The dose of the DNA of the present invention varies depending on the subject of administration, target organ, symptoms, method of administration and the like; in oral administration, the dose is normally about 0.1 to 100 mg, preferably about 1.0 to 50 mg, and more preferably about 1.0 to 20 mg, per day, in, for example, a patient with a sugar/lipid metabolic abnormality (assuming a 60 kg body weight). In parenteral administration, the single dose varies depending on the subject of administration, target organ, symptoms, method of administration and the like; for example, in the form of an injection, the dose is normally about 0.01 to 30 mg, preferably about 0.1 to 20 mg, and more preferably about 0.1 to 10 mg, per day, in, for example, a patient with a sugar/lipid metabolic abnormality (assuming a 60 kg body weight). In cases where the subject of administration is other than a human, a dose corresponding to a human dose per 60 kg body weight can be administered.

Meanwhile, an antibody against an NPR3 species (an antibody of the present invention) is capable of inhibiting the interaction of musclin and NPR3 to inactivate (that is, neutralize) the signal transduction function involved by NPR3, for example, cell differentiation and/or metabolism regulatory activity. Meanwhile, a nucleic acid comprising a base sequence complementary to the base sequence that encodes NPR3 or a portion thereof (including ribozyme and siRNA; an antisense nucleic acid of the present invention) is capable of inhibiting the expression of NPR3 by blocking the transcription of the NPR3 gene, the processing of the transcription product and/or the translation from mRNA. Therefore, (1) an antibody of the present invention or (2) an antisense nucleic acid of the present invention, as a cell differentiation and/or metabolism regulator in the orientation reverse to that of the above-described NPR3 species or a DNA of the present invention, can be used as pharmaceutical, for example, a prophylactic/therapeutic agent for diseases associated with the overexpression of NPR3 and the like. As diseases associated with the overexpression of NPR3, abnormalities of cell differentiation and/or metabolism, preferably abnormalities of cell differentiation and/or metabolism, preferably sugar/lipid/protein metabolism, in the liver, fat tissue, skeletal muscles, pancreatic island, bone, cartilage and the like, can be mentioned; more specifically, examples include, but are not limited to, obesity, diabetes, impaired glucose tolerance, arteriosclerosis, hypertension, hyperlipemia, metabolic bone/cartilage diseases and the like.

An antibody of the present invention and an antisense nucleic acid of the present invention can each be prepared as a pharmaceutical preparation as with the aforementioned NPR3 species and a DNA of the present invention, respectively. The antisense nucleic acid can also be administered as it stands with a gene gun or a catheter such as a hydrogel catheter.
The dose of an antibody of the present invention varies depending on the subject of administration, target organ, symptoms, method of administration and the like; in the case of oral administration, the dose is normally about 0.1 to 100 mg, preferably about 1.0 to 50 mg, and more preferably about 1.0 to 20 mg, per day, in, for example, a patient with an abnormality of sugar/lipid metabolism (assuming a 60 kg body weight). In the case of parenteral administration, the single dose varies depending on the subject of administration, target organ, symptoms, method of administration and the like; for example, in the form of an injection formulation, it is advantageous that the dose is normally about 0.01 to 30 mg, preferably about 0.1 to 20 mg, and more preferably about 0.1 to 10 mg, per day, in, for example, a patient with an abnormality of sugar/lipid metabolism (assuming a 60 kg body weight). In cases where the subject of administration is other than a human, a dose corresponding to a human dose per 60 kg body weight can be administered.
The dose of an antisense nucleic acid of the present invention varies depending on the subject of administration, target organ, symptoms, method of administration and the like; in the case of oral administration, the dose is normally about 0.1 to 100 mg, preferably about 1.0 to 50 mg, and more preferably about 1.0 to 20 mg, per day, in, for example, a patient with an abnormality of sugar/lipid metabolism (assuming a 60 kg body weight). In the case of parenteral administration, the single dose varies depending on the subject of administration, target organ, symptoms, method of administration and the like; for example, in the form of an injection formulation, it is advantageous that the dose is normally about 0.01 to 30 mg, preferably about 0.1 to 20 mg, and more preferably about 0.1 to 10 mg, per day, in, for example, a patient with an abnormality of sugar/lipid metabolism (assuming a 60 kg body weight). In cases where the subject of administration is other than a human, a dose corresponding to a human dose per 60 kg body weight can be administered.

(2) Genetic diagnostic reagents

Because a nucleic acid encoding NPR3 or a portion thereof (hereinafter referred to as "the sense nucleic acid of the present invention") or the antisense nucleic acid of the present invention is capable of detecting an abnormality in the DNA or mRNA encoding NPR3 in a mammal (for example, rats, rabbits, sheep, swine, cattle, cats, dogs, monkeys and the like) (gene abnormality) when used as a probe, it is useful as, for example, a genetic diagnostic reagent for damage, mutation or decreased expression of the DNA or mRNA, increased expression or overexpression of the DNA or mRNA, and the like.
The above-described genetic diagnosis using the sense or antisense nucleic acid of the present invention can be performed by, for example, methods known per se, such as Northern hybridization and the PCR-SSCP method (Genomics, Vol. 5, pp. 874-879 (1989), Proceedings of the National Academy of Sciences of the United States of America, Vol. 86, pp. 2766-2770 (1989)).
For example, if decreased expression of NPR3 is detected by Northern hybridization and the like, for example, the subject can be diagnosed as being likely to have a disease associated with dysfunction of NPR3, or being likely to suffer from the disease in the future.
If overexpression of NPR3 is detected by Northern hybridization and the like, for example, the subject can be diagnosed as being likely to have a disease associated with overexpression of NPR3, or being likely to suffer from the disease in the future.
As examples of diseases associated with dysfunction or overexpression of NPR3, abnormalities of cell differentiation and/or metabolism, preferably abnormalities of cell differentiation and/or metabolism, preferably sugar/lipid/protein metabolism, in the liver, fat tissue, skeletal muscles, pancreatic island, bone, cartilage and the like, more specifically, for example, obesity, diabetes, impaired glucose tolerance, arteriosclerosis, hypertension, hyperlipemia, metabolic bone/cartilage diseases and the like can be mentioned.

(3) Screening a compound that changes the expression amount of NPR3

The sense nucleic acid of the present invention can be used as a probe for screening a compound that changes the expression amount of NPR3.
Accordingly, the present invention provides a method of screening a compound that changes the expression amount of NPR3, which comprises measuring the amount of NPR3 mRNA contained in, for example, (i) (1) blood, (2) a particular organ, (3) a tissue or cells isolated from an organ, of a non-human mammal, or (ii) a transformant or the like.
Specifically, the amount of NPR3 mRNA is measured as described below.

(i) A drug (for example, anti-obesity drugs, anti-diabetic drugs, antihypertensive drugs, vasoactive drugs, anticancer agents and the like) or a physical stress (for example, soaking stress, electric shock, brightness/darkness, low temperatures and the like) or the like is given to a normal or disease model non-human mammal (for example, mice, rats, rabbits, sheep, swine, cattle, cats, dogs, monkeys and the like, more specifically, obese mice, diabetic mice, hypertensive rats, arteriosclerotic rabbits, cancer-bearing mice and the like); after a given time has elapsed, blood or a particular organ (for example, liver, pancreas, kidney, muscles and the like), a tissue (for example, brown or white adipose tissue and the like) or cells (skeletal muscle cells, adipocytes and the like) are obtained.
The NPR3 mRNA contained in the cells and the like obtained can be quantified by, for example, extracting the mRNA from the cells and the like by an ordinary method, and using a technique such as TaqMan PCR, and can also be analyzed by performing Northern blot by a means known per se.
(ii) A transformant expressing NPR3 or a partial peptide thereof can be prepared according to the method described above, and the mRNA of the NPR3 or a partial peptide thereof contained in the transformant can be quantified and analyzed in the same manner as described above.

Screening of a compound that changes the expression amount of NPR3 can be performed by:

(i) administering a test compound to a normal or disease model non-human mammal at a given time before (before 30 minutes to before 24 hours, preferably before 30 minutes to before 12 hours, more preferably before 1 hour to before 6 hours) or after (after 30 minutes to after 3 days, preferably after 1 hour to after 2 days, more preferably after 1 hour to after 24 hours) giving a drug, a physical stress or the like, or at the same time as the drug or physical stress, and quantifying and analyzing the amount of NPR3 mRNA contained in the cells after a given time has elapsed after administration (after 30 minutes to after 3 days, preferably after 1 hour to after 2 days, more preferably after 1 hour to after 24 hours), and can also be preformed by:
(ii) mixing a test compound in the medium at the start of cultivation of the transformant according to a conventional method, and quantifying and analyzing the amount of mRNA of NPR3 or a partial peptide thereof contained in the transformant after cultivation for a given time (after 1 day to after 7 days, preferably after 1 day to after 3 days, more preferably after 2 days to after 3 days).

A compound obtained using the above-described screening method or a salt thereof is a compound having an action to alter the expression level of NPR3, specifically, (a) a compound that enhances a cell stimulatory activity via an interaction of NPR3 and musclin (for example, intracellular cAMP production, inositol phosphate production, Ca uptake, cGMP production, sugar uptake in cells/glycogen synthesis/degradation in cells, sugar release from cells, fat uptake in cells, fat release from cells, fat degradation in cells, fat synthesis in cells, proteinous/non-proteinous hormone secretion regulation, proteinous/non-proteinous hormone action regulation, appetite regulation, motor activity regulation, changes in body weight, changes in saccharides/lipids in blood, regulation of muscle cell differentiation and proliferation, osteoblast differentiation and proliferation and the like, and the like) by increasing the expression level of NPR3, or (b) a compound that weakens the cell stimulatory activity by reducing the expression level of NPR3.
As the compound used, peptides, proteins, non-peptide compounds, synthetic compounds, fermentation products and the like can be mentioned; these compounds may be novel compounds or known compounds.
A compound that enhances the cell stimulation activity is useful as a safe and less toxic pharmaceutical for enhancing a physiological activity of NPR3.
A compound that weakens the cell stimulation activity is useful as a safe and less toxic pharmaceutical for reducing a physiological activity of NPR3.

When a compound obtained using the above-described screening method or a salt thereof is used as a pharmaceutical, it can be formulated in the same manner as with the aforementioned NPR3 species.
Because the preparation thus obtained is safe and less toxic, it can be administered to, for example, a mammal (for example, humans, rats, rabbits, sheep, swine, cattle, cats, dogs, monkeys and the like).
The dose of the compound or a salt thereof varies depending on the subject of administration, target organ, symptoms, method of administration and the like; in oral administration, the dose is normally about 0.1 to 100 mg, preferably about 1.0 to 50 mg, and more preferably about 1.0 to 20 mg, per day, in, for example, a patient with a sugar/lipid metabolic abnormality (assuming a 60 kg body weight). In parenteral administration, the single dose varies depending on the subject of administration, target organ, symptoms, method of administration and the like; for example, in the form of an injection, the dose is normally about 0.01 to 30 mg, preferably about 0.1 to 20 mg, and more preferably about 0.1 to 10 mg, per day, in, for example, a patient with a sugar/lipid metabolic abnormality (assuming a 60 kg body weight). In cases where the subject of administration is other than a human, a dose corresponding to a human dose per 60 kg body weight can be administered.

Because NPR3 is expressed specifically in skeletal muscles and functions as a receptor of musclin, which is highly expressed in diabetes model mice and post-fasting refeeding, and also because NPR3 is expressed in bone and can influence bone/cartilage metabolism, and for other reasons, as stated above, NPR3 is thought to play an important role in the regulation of (i) cell differentiation and/or (ii) metabolism (for example, cell differentiation and/or metabolism (for example, sugar/lipid/protein metabolism) in the liver, fat tissue, skeletal muscles, pancreatic island, bone, cartilage and the like). Therefore, a compound that alters the expression level of NPR3 can be used as a regulator of (i) cell differentiation and/or (ii) metabolism (for example, cell differentiation and/or metabolism (for example, sugar/lipid/protein metabolism) in the liver, fat tissue, skeletal muscles, pancreatic island, bone, cartilage and the like), preferably as a prophylactic/therapeutic agent for abnormalities of cell differentiation and/or metabolism (specifically, for example, obesity, diabetes, impaired glucose tolerance, arteriosclerosis, hypertension, hyperlipemia, metabolic bone/cartilage diseases and the like).
When the compound is used as the above-described pharmaceutical, it can be prepared as a pharmaceutical preparation in the same manner as with an NPR3 species as described above.
Because the preparation thus obtained is safe and less toxic, it can be administered to, for example, a mammal (for example, humans, rats, rabbits, sheep, swine, cattle, cats, dogs, monkeys and the like).
The dose of the compound or a salt thereof varies depending on the subject of administration, target organ, symptoms, method of administration and the like; in oral administration, the dose is normally about 0.1 to 100 mg, preferably about 1.0 to 50 mg, and more preferably about 1.0 to 20 mg, per day, in, for example, a patient with a sugar/lipid metabolic abnormality (assuming a 60 kg body weight). In parenteral administration, the single dose varies depending on the subject of administration, target organ, symptoms, method of administration and the like; for example, in the form of an injection, the dose is normally about 0.01 to 30 mg, preferably about 0.1 to 20 mg, and more preferably about 0.1 to 10 mg, per day, in, for example, a patient with a sugar/lipid metabolic abnormality (assuming a 60 kg body weight). In cases where the subject of administration is other than a human, a dose corresponding to a human dose per 60 kg body weight can be administered.

(4) Quantitation of musclin

Because an NPR3 species has bindability for musclin, it enables highly sensitive quantitation of the concentration of the musclin in vivo.
The method of quantitation of the present invention can be used in combination with, for example, the competitive method. That is, by bringing a test sample in contact with an NPR3 species, the musclin concentration in the test sample can be measured. Specifically, for example, the method of quantitation of the present invention can be used according to a method described in (1) or (2) below and the like or a method based thereon.

(1) Hiroshi Irie, ed., "Radioimmunoassay" (Kodansha Ltd., published in 1974)
(2) Hiroshi Irie, ed., "Sequel to the Radioimmunoassay" (Kodansha Ltd., 1979)

(5) Screening for compounds that alter the bindability of NPR3 and musclin (agonists, antagonists and the like)

A compound that changes the bindability between NPR3 and musclin (e.g., peptides, proteins, non-peptide compounds, synthetic compounds, fermentation products and the like) or a salt thereof can be efficiently screened by using an NPR3 species, or by constructing an expression system for recombinant NPR3, and using an affinity assay system based on the expression system.
Such compounds include (a) a compound having a cell stimulatory activity via NPR3 (for example, intracellular cAMP production, inositol phosphate production, Ca uptake, cGMP production, sugar uptake in cells and glycogen synthesis/degradation in cells, sugar release from cells, fat uptake in cells, fat release from cells, fat degradation in cells, fat synthesis in cells, proteinous/non-proteinous hormone secretion regulation, proteinous/non-proteinous hormone action regulation, appetite regulation, motor activity regulation, changes in body weight, changes in saccharides/lipids in blood, regulation of muscle cell differentiation and proliferation, osteoblast differentiation and proliferation and the like, and the like) (agonist), (b) a compound not having the cell stimulatory activity (antagonist), (d) a compound that enhances the binding force between NPR3 and musclin, or (d) a compound that reduces the binding force between NPR3 and musclin and the like.
Accordingly, the present invention provides a screening method for a compound that alters the bindability of NPR3 and musclin or a salt thereof, comprising making a comparison between (i) a case where an NPR3 species and musclin or a partial peptide thereof or a salt thereof (hereinafter comprehensively referred to as "a musclin species") are brought into contact with each other and (ii) a case where an NPR3 species and a musclin species and a test compound are brought into contact with each other.
The above-described screening method comprises measuring and comparing the amount of musclin species bound to NPR3, a cell stimulatory activity and the like in cases (i) and (ii).
More specifically, the present invention provides:

(1) a screening method for a compound that alters the bindability of NPR3 and musclin or a salt thereof, comprising measuring and comparing the amount of a labeled musclin species bound to an NPR3 species in a case where the labeled musclin species is brought into contact with the NPR3 species, and a case where the labeled musclin species and a test compound are brought into contact with the NPR3 species,
(2) a screening method for a compound that alters the bindability of NPR3 and musclin or a salt thereof, comprising measuring and comparing the amount of a labeled musclin species bound to a cell that produces NPR3 or a membrane fraction thereof in a case where the labeled musclin species is brought into contact with the cell that produces NPR3 or the membrane fraction thereof, and a case where the labeled musclin species and a test compound are brought into contact with the cell or membrane fraction,
(3) a screening method for a compound that alters the bindability of NPR3 and musclin or a salt thereof, comprising measuring and comparing the amount of a labeled musclin species bound to an NPR3 species in a case where the labeled musclin species is brought into contact with the NPR3 species expressed on the cell membrane by culturing a transformant comprising a DNA of the present invention, and a case where the labeled musclin species and a test compound are brought into contact with the NPR3 species expressed on the cell membrane by culturing a transformant comprising a DNA of the present invention,
(4) a screening method for a compound that alters the bindability of NPR3 and musclin or a salt thereof, comprising measuring and comparing a cell stimulatory activity via NPR3 (for example, intracellular cAMP production, inositol phosphate production, Ca uptake, cGMP production, sugar uptake in cells/glycogen synthesis/degradation in cells, sugar release from cells, fat uptake in cells, fat release from cells, fat degradation in cells, fat synthesis in cells, proteinous/non-proteinous hormone secretion regulation, proteinous/non-proteinous hormone action regulation, appetite regulation, motor activity regulation, changes in body weight, changes in saccharides/lipids in blood, regulation of muscle cell differentiation and proliferation, osteoblast differentiation and proliferation and the like, and the like) in a case where a compound that activates NPR3 (for example, a musclin species and the like) is brought into contact with a cell that expresses NPR3 on the cell membrane thereof and a case where the compound that activates NPR3 and a test compound are brought into contact with a cell that expresses NPR3 on the cell membrane thereof, and
(5) a screening method for a compound that alters the bindability of NPR3 and musclin or a salt thereof, comprising measuring and comparing a cell stimulatory activity via NPR3 (for example, intracellular cAMP production, inositol phosphate production, Ca uptake, cGMP production, sugar uptake in cells/glycogen synthesis/degradation in cells, sugar release from cells, fat uptake in cells, fat release from cells, fat degradation in cells, fat synthesis in cells, proteinous/non-proteinous hormone secretion regulation, proteinous/non-proteinous hormone action regulation, appetite regulation, motor activity regulation, changes in body weight, changes in saccharides/lipids in blood, muscle cell differentiation and proliferation, regulation of osteoblast differentiation and proliferation and the like, and the like) in a case where a compound that activates NPR3 (for example, a musclin species and the like) is brought into contact with an NPR3 species expressed on the cell membrane by culturing a transformant comprising the DNA of the present invention, and a case where the compound that activates NPR3 and a test compound are brought into contact with an NPR3 species expressed on the cell membrane by culturing a transformant comprising the DNA of the present invention.

The screening method of the present invention is hereinafter described specifically.
First, the NPR3 species used in the screening method of the present invention may be any one comprising the above-described NPR3 or a partial peptide thereof or a salt thereof, and the cell membrane fraction of an organ of an NPR3-producing mammal is preferred. However, because human-derived organs, in particular, are extremely difficult to obtain, the NPR3 species used in the screening method of the present invention is preferably a human-derived NPR3 species expressed in a large amount using a transformant.
An NPR3 species is produced using the method described above, which is preferably performed by expressing the DNA of the present invention in mammalian cells or insect cells. A cDNA is normally used as the DNA fragment encoding the desired portion of the protein, but is not construed as limiting. For example, a gene fragment or a synthetic DNA may also be used. For introducing a DNA fragment encoding NPR3 or a partial peptide thereof into host animal (or insect) cells and efficiently expressing the same, it is preferable to insert the DNA fragment downstream of an SV40-derived promoter, a retrovirus promoter, a metallothionein promoter, a human heat shock promoter, a cytomegalovirus promoter, an SR$\alpha$ promoter, the polyhedrin promoter of nuclear polyhedrosis virus (NPV), which is a baculovirus having insect hosts, or the like.
Therefore, the NPR3 species used in the screening method of the present invention may be an NPR3 species purified according to a method known per se, or may be used as cells producing the NPR3 species or a cell membrane fraction thereof.
In the above-described screening method, when using cells producing an NPR3 species, the cells may be fixed with glutaraldehyde, formalin and the like. This fixation can be performed according to a method known per se.
Cells producing an NPR3 species refer to host cells expressing the NPR3 species; as the host cells, *Escherichia coli, Bacillus subtilis,* yeasts, insect cells, animal cells and the like can be used.
The aforementioned cell membrane fraction refers to a fraction rich in cell membrane obtained by a method known per se after cell disruption. As the method of cell disruption, cell squashing using a Potter-Elvehjem homogenizer, disruption using a Waring blender or Polytron (manufactured by Kinematica Inc.), disruption by ultrasonication, disruption by cell spraying through a thin nozzle under an increased pressure using a French press or the like, and the like can be mentioned. For cell membrane fractionation, fractionation by centrifugal forces, such as fractional centrifugation or density gradient centrifugation, is mainly used. For example, a cell disruption fluid is centrifuged at a low speed (500 rpm to 3000 rpm) for a short time (normally about 1 to 10 minutes), the supernatant is centrifuged at a higher speed (15000 rpm to 30000 rpm) normally for 30 minutes to 2 hours, and the precipitate obtained is used as the membrane fraction. The membrane fraction is rich in the NPR3 species expressed and cell-derived membrane components such as phospholipids and membrane proteins.
The amount of NPR3 species in the cells producing the NPR3 species and the membrane fraction thereof is preferably $10^3$ to $10^8$ molecules per cell, more preferably $10^5$ to $10^7$ molecules. Note that as the expression amount increases, musclin binding activity per unit of membrane fraction (specific activity) increases so that it is possible to construct a highly sensitive screening system, and to assay a large number of samples in the same lot.
To perform methods (1) to (3) above for screening a compound that changes the bindability between NPR3 and musclin, for example, an appropriate NPR3 species-containing membrane fraction and labeled musclin species are necessary.
As the NPR3-containing membrane fraction, a naturally occurring NPR3-containing membrane fraction or a recombinant NPR3 species-containing membrane fraction having an activity equivalent to that thereof, or the like is desirable. As used herein, equivalent activity means equivalent musclin-binding activity, signal transmission activity or the like.
As a labeled musclin species, a protein or a partial peptide thereof or a salt thereof, described as an "an NPR3 species" in the above-described patent document 1, labeled according to a conventional method, and the like are used. For example, the protein or partial peptide thereof or salt thereof and the like, labeled with [$^3$H], [$^{125}$I] [$^{14}$C], [$^{35}$S] or the like, is used.
Specifically, to perform screening of a compound that changes the bindability between NPR3 and musclin, cells producing an NPR3 species or a membrane fraction thereof are(is) first suspended in a buffer suitable for the screening to prepare a standard preparation of NPR3 species. The buffer may be any buffer that does not interfere with the binding of NPR3 and musclin, such as a phosphate buffer or Tris-hydrochloride buffer having a pH value of 4 to 10 (desirably a pH value of 6 to 8). To reduce non-specific binding, a surfactant such as CHAPS, Tween-80™ (Kao-Atlas Co.), digitonin or deoxycholate may be added to the buffer. Furthermore, to suppress the degradation of receptors and ligands by proteases, a protease inhibitor such as PMSF, leupeptin, E-64 (manufactured by Peptide Research Laboratory, Co.), or pepstatin may be added. A given amount (5,000 cpm to 500,000 cpm) of labeled musclin species is added to 0.01 ml to 10 ml of a suspension of NPR3 species, in the presence of a $10^{-4}$M to $10^{-10}$M test compound. To determine non-specific binding (NSB), a reaction tube containing the unlabeled ligand in large excess is also provided. The reaction is performed at about 0°C to 50°C, desirably about 4°C to 37°C, for about 20 minutes to 24 hours, desirably about 30 minutes to 3 hours. After the reaction, the reaction mixture is filtered through glass fiber filter paper and the like, and washed with an appropriate amount of the same buffer; thereafter, the residual radioactivity on the glass fiber filter paper is counted using a liquid scintillation counter or a γ-counter. A test compound showing a specific binding (B-NSB) of, for example, not more than 50%, as the percent ratio to the count ($B_0$-NSB) calculated by subtracting non-specific binding (NSB) from the count in the absence of antagonizing substance ($B_0$), can be selected as a candidate substance capable of inhibiting the antagonism.

To embody the above-described methods (4) to (5) for screening a compound that alters the bindability of NPR3 and musclin, for example, a cell stimulatory activity via NPR3 (for example, intracellular cAMP production, inositol phosphate production, Ca uptake, cGMP production, sugar uptake in cells/glycogen synthesis/degradation in cells, sugar release from cells, fat uptake in cells, fat release from cells, fat degradation in cells, fat synthesis in cells, proteinous/non-proteinous hormone secretion regulation, proteinous/non-proteinous hormone action regulation, appetite regulation, motor activity regulation, changes in body weight, changes in saccharides/lipids in blood, regulation of muscle cell differentiation and proliferation, osteoblast differentiation and proliferation and the like, and the like) can be measured using a publicly known method or a commercially available assay kit.

Specifically, first, cells producing an NPR3 species are cultured on a multi-well plate or the like. In performing the screening, the medium is replaced with a fresh medium or an appropriate non-cytotoxic buffer in advance, and a test compound and the like are added, followed by incubation for a given time; thereafter, the cells are extracted or the supernatant is recovered, and the resulting product is quantified by each method. If it is difficult to detect the production of an indicator substance for cell stimulation activity due to a degrading enzyme contained in the cells, the assay may be performed with the addition of an inhibitor of the degrading enzyme. Activities such as cAMP production suppression can be detected as suppressive effects on the production of cells having baseline production previously increased with forskolin or the like.

To perform the screening with a measurement of a cell stimulation activity, appropriate cells expressing an NPR3 species on the membrane thereof are necessary. As the cells expressing an NPR3 species, cell lines producing naturally occurring NPR3, cell lines expressing the aforementioned recombinant NPR3 species and the like are desirable.

As examples of the test compound used, peptides, proteins, non-peptide compounds, synthetic compounds, fermentation products, cell extracts, plant extracts, animal tissue extracts and the like can be mentioned; these compounds may be novel compounds or known compounds.

A screening kit for a compound that alters the bindability of NPR3 and musclin or a salt thereof comprises an NPR3 species, a cell that produces an NPR3 species or a membrane fraction thereof and the like.

As examples of the screening kit of the present invention, the following can be mentioned.

1. Screening reagents

(1) Assay buffer solution and wash buffer solution

Hanks' balanced salt solution (manufactured by Gibco Co.) supplemented with 0.05% bovine serum albumin (manufactured by Sigma Co.).

The solution is sterilized by filtration through a filter of 0.45 μm pore size and stored at 4°C, or may be prepared freshly just before use.

(2) Reference standards of NPR3 species

CHO cells allowed to express an NPR3 species, passaged with a 12-well plate at $5\times10^5$ cells/well, cultured at 37°C, 5% $CO_2$, 95% air for 2 days.

(3) Labeled musclin species

A musclin species labeled with a commercially available [3H], [$^{125}$I], [$^{14}$C], [$^{35}$S] or the like

An aqueous solution of the ligand (receptor) is stored at 4°C or -20°C and diluted to 1 μM with the assay buffer solution just before use.

(4) Standard solution of musclin

The musclin is dissolved in PBS containing 0.1% bovine serum albumin (manufactured by Sigma Co.) to obtain a concentration of 1 mM, and stored at -20°C.

2. Assay method

(1) After NPR3 species-expressing CHO cells cultured on a 12-well tissue culture plate are washed twice with 1 ml of the assay buffer solution, 490 μl of the assay buffer solution is added to each well.

(2) After 5 μl of $10^{-3}$ to $10^{-10}$M test compound solution is added, 5 μl of labeled musclin species is added and the reaction is performed at room temperature for 1 hour. To determine non-specific binding, 5 μl of $10^{-3}$M standard musclin solution, instead of the test compound, is added in advance.

(3) The reaction liquid is removed and the wells are washed 3 times with 1 ml of the wash buffer solution. The labeled musclin bound to the cells (or plate) is dissolved in 0.2N NaOH-1% SDS, and mixed with 4 ml of liquid scintillator A (manufactured by Wako Pure Chemical Industries, Ltd.).

(4) Radioactivity is measured using a liquid scintillation counter (manufactured by Beckman Co.), and percent maximum binding (PMB) is calculated using the following equation [Equation 1].

[Equation 1]

$$PMB = [(B - NSB)/(B_0 - NSB)] \times 100$$

PMB: percent maximum binding
B: value obtained in the presence of test compound
NSB: non-specific binding
$B_0$: maximum binding

A compound obtained using the above-described screening method or kit for screening or a salt thereof is a compound having an action to alter the bindability of NPR3 and musclin, specifically, (a) a compound having a cell stimulatory activity via a musclin-NPR3 interaction (for example, intracellular cAMP production, inositol phosphate production, Ca uptake, cGMP production, sugar uptake in cells/glycogen synthesis/degradation in cells, sugar release from cells, fat uptake in cells, fat release from cells, fat degradation in cells, fat synthesis in cells, proteinous/non-proteinous hormone secretion regulation, proteinous/non-proteinous hormone action regulation, appetite regulation, motor activity regulation, changes in body weight, changes in saccharides/lipids in blood, regulation of muscle cell differentiation and proliferation, osteoblast differentiation and proliferation and the like, and the like) (agonist), (b) a compound not having the cell stimulatory activity (antagonist), (c) a compound that enhances the binding force between NPR3 and musclin, or (d) a compound that reduces the binding force between NPR3 and musclin.

As the compound used, peptides, proteins, non-peptide compounds, synthetic compounds, fermentation products and the like can be mentioned; these compounds may be novel compounds or known compounds.

Because an agonist for NPR3 has a similar physiological activity to that of a musclin for NPR3, it is useful as a safe and less toxic pharmaceutical depending on the musclin activity.

Because an antagonist for NPR3 is capable of suppressing a physiological activity of a musclin for NPR3, it is useful as a safe and less toxic pharmaceutical for suppressing the musclin activity.

A compound that enhances the bindability between NPR3 and musclin is useful as a safe and less toxic pharmaceutical for enhancing a physiological activity of a musclin for NPR3.

A compound that reduces the bindability between NPR3 and musclin is useful as a safe and less toxic pharmaceutical for reducing a physiological activity of a musclin for NPR3.

(6) Prophylactic/therapeutic agents for various diseases comprising a compound that alters the bindability of NPR3 and musclin (agonist, antagonist)

Because NPR3 is expressed specifically in skeletal muscles and functions as a receptor of musclin, which is highly expressed in diabetes model mice and post-fasting refeeding, and also because NPR3 is expressed in bone and can influence bone/cartilage metabolism, and for other reasons, as stated above, NPR3 is thought to play an important role in the regulation of (i) cell differentiation and/or (ii) metabolism (for example, cell differentiation and/or metabolism (for example, sugar/lipid/protein metabolism) in the liver, fat tissue, skeletal muscles, pancreatic island, bone, cartilage and the like). Therefore, a compound that alters the bindability of NPR3 and musclin (agonist, antagonist) can be used as a regulator of (i) cell differentiation and/or (ii) metabolism (for example, cell differentiation and/or metabolism (for example, sugar/lipid/protein metabolism) in the liver, fat tissue, skeletal muscles, pancreatic island, bone, cartilage and the like), preferably, as a prophylactic/therapeutic agent for abnormalities of cell differentiation and/or metabolism (specifically, for example, obesity, diabetes, impaired glucose tolerance, arteriosclerosis, hypertension, hyperlipemia, metabolic bone/cartilage diseases and the like).
Because the preparation thus obtained is safe and of low toxicity, it can be administered to, for example, mammals (for example, humans, rats, rabbits, sheep, pigs, bovines, cats, dogs, monkeys and the like).
The dose of the compound or a salt thereof varies depending on the subject of administration, target organ, symptoms, method of administration and the like; in oral administration, the dose is normally about 0.1 to 100 mg, preferably about 1.0 to 50 mg, and more preferably about 1.0 to 20 mg, per day, in, for example, a patient with a sugar/lipid metabolic abnormality (assuming a 60 kg body weight). In parenteral administration, the single dose varies depending on the subject of administration, target organ, symptoms, method of administration and the like; for example, in the form of an injection, the dose is normally about 0.01 to 30 mg, preferably about 0.1 to 20 mg, and more preferably about 0.1 to 10 mg, per day, in, for example, a patient with a sugar/lipid metabolic abnormality (assuming a 60 kg body weight). In cases where the subject of administration is other than a human, a dose corresponding to a human dose per 60 kg body weight can be administered.

(7) Quantitation of NPR3 species

Because the antibody of the present invention is capable of specifically recognizing an NPR3 species, it can be used for quantitation of NPR3 species in a test fluid, particularly quantitation by sandwich immunoassay, and the like. Accordingly, the present invention provides, for example, (i) a method of quantifying an NPR3 species in a test fluid, which comprises competitively reacting the antibody of the present invention and the test fluid, a labeled protein and the like, and measuring the ratio of labeled protein and the like bound to the antibody, and (ii) a method of quantifying an NPR3 species in a test fluid, which comprises simultaneously or sequentially reacting the test fluid, the antibody of the present invention as immobilized on a carrier, and the antibody of the present invention as labeled, and measuring the activity of labeling agent on the insolubilizing carrier.
In (ii) above, the insolubilized antibody and the labeled antibody preferably have antigen recognition sites such that they do not mutually interfere with their binding to NPR3 species (for example, one antibody recognizes the N-terminus of the NPR3 species, and the other antibody reacts with the C-terminus of the NPR3 species, and the like).
Using a monoclonal antibody against an NPR3 species (hereinafter sometimes referred to as the monoclonal antibody of the present invention), the NPR3 species can be measured, and can also be detected by tissue staining and the like. For these purposes, the antibody molecule itself may be used, and the F(ab')2, Fab' or Fab fraction of the antibody molecule may also be used. The assay method using an antibody against an NPR3 species is not to be subject to limitation; any assay method can be used, as long as the amount of antibody, antigen, or antibody-antigen complex corresponding to the amount of antigen (for example, the amount of NPR3) in a test fluid is detected by a chemical or physical means and calculated from a standard curve generated using standard solutions containing known amounts of antigen. For example, nephelometry, the competitive method, immunometric method, and sandwich method are advantageously used, with preference given to the sandwich method described below, in terms of sensitivity and specificity.
As examples of the labeling agent used for the assay method using a labeled substance, radioisotopes, enzymes, fluorescent substances, luminescent substances and the like can be mentioned. As examples of the radioisotopes used, [$^{125}$I], [$^{131}$I], [$^{3}$H], [$^{14}$C] and the like can be mentioned. As examples of the enzymes, stable enzymes of high specific activity are preferred; for example, β-galactosidase, β-glucosidase, alkaline phosphatase, peroxidase, malate dehydrogenase and the like can be mentioned. As examples of the fluorescent substances, fluorescamine, fluorescein isothiocyanate and the like can be mentioned. As examples of the luminescent substances, luminol, luminol derivatives, luciferin, lucigenin and the like can be mentioned. Furthermore, the biotin-avidin system may also be used for the binding of an antibody or antigen and the labeling agent.
For insolubilization of the antigen or antibody, physical adsorption may be used, and methods based on chemical binding, which are normally used to insolubilize or immobilize proteins or enzymes and the like, may also be used. As examples of the carrier, insoluble polysaccharides such as agarose, dextran, and cellulose; synthetic resins such as polystyrene, polyacrylamide and silicone; glass and the like can be used.
In the sandwich method, the monoclonal antibody of the present invention as insolubilized is reacted with a test fluid (primary reaction), and further reacted with the monoclonal antibody of the present invention as labeled (secondary reaction), and the activity of the labeling agent on the insolubilizing carrier is then measured, whereby the amount of NPR3 in the test fluid can be quantified. The primary and secondary reactions may be performed in the reverse order, and may be performed simultaneously or at a time interval. The labeling agent and the method of insolubilization may be the same as those described above.
In immunoassays by the sandwich method, the antibody used for an immobilized antibody or a labeled antibody need not always be one kind; a mixture of two or more kinds of antibodies may be used to increase assay sensitivity.
In assaying an NPR3 species by the sandwich method, the monoclonal antibodies of the present invention used for the primary and secondary reactions are preferably antibodies having mutually different sites for binding with the NPR3 species. That is, regarding the antibodies used for the primary and secondary reactions, for example, when the antibody used for the secondary reaction recognizes the C-terminus region of the NPR3 species, the antibody used for the primary reaction is preferably an antibody that recognizes a region other than the C-terminus region, for example, the N-terminus region.

The monoclonal antibody of the present invention can be used for assay systems other than the sandwich method, for example, the competitive method, immunometric method, nephelometry and the like. In the competitive method, an antigen in a test fluid and a labeled antigen are competitively reacted with an antibody, then the unreacted labeled antigen (F) and the labeled antigen bound to the antibody (B) are separated (B/F separation); the amount of labeled antigen in B or F is measured to quantify the amount of antigen in the test fluid. For this reaction, the liquid phase method, which uses a soluble antibody and polyethylene glycol and a secondary antibody to the above-described antibody and the like for B/F separation, and the immobilization method, which uses a soluble primary antibody and a solid-immobilized secondary antibody, are available.

In the immunometric method, an antigen in a test fluid and an immobilized antigen are competitively reacted with a given amount of labeled antibody, and thereafter the solid phase is separated from the liquid phase, or an antigen in a test fluid is reacted with an excess amount of labeled antibody, an immobilized antigen is then added to bind the unreacted labeled antibody to the solid phase, and the solid phase is separated from the liquid phase. Subsequently, the amount of labeled antibody in either phase is measured to quantify the amount of antigen in the test fluid.

In nephelometry, the amount of insoluble precipitate from the antigen-antibody reaction within the gel or in the solution is measured. Even when the amount of antigen in the test fluid is small and only a small amount of precipitate is obtained, laser nephelometry, which is based on laser scattering, and the like can be used advantageously.

In applying these individual immunoassays to the quantification of an NPR3 species, no special conditions, procedures or the like need to be set forth. The assay system for the NPR3 species may be constructed with ordinary technical considerations of those skilled in the art on ordinary conditions and procedures in the respective methods. For details of these general techniques, reference can be made to reviews, texts and the like [see, for example, Hiroshi Irie, ed., "Radioimmunoassay" (Kodansha Ltd., published in 1974), Hiroshi Irie, ed., "Sequel to the Radioimmunoassay" (Kodansha Ltd., published in 1979), Eiji Ishikawa et al., ed., "Enzyme Immonoassay" (Igakushoin, published in 1978), Eiji Ishikawa et al., ed., "Enzyme Immonoassay" (2nd ed.) (Igakushoin, published in 1982), Eiji Ishikawa et al., ed., "Enzyme Immonoassay" (3rd ed.) (Igakushoin, published in 1987), Methods in ENZYMOLOGY, Vol. 70 (Immunochemical Techniques (Part A)), ibidem, Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibidem, Vol. 92 (Immunochemical Techniques (Part E: Monoclonal Antibodies and General Immunoassay Methods)), ibidem, Vol. 121 (Immunochemical Techniques (Part I: Hybridoma Technology and Monoclonal Antibodies)) (all published by Academic Press Publishing) and the like].

As described above, an NPR3 species can be quantified highly sensitively by using the antibody of the present invention. Furthermore, various diseases associated with NPR3 hypofunction or hyperfunction can be diagnosed by quantifying NPR3 or a salt thereof in vivo using the antibody of the present invention.

The antibody of the present invention can also be used to specifically detect NPR3 or a salt thereof present in a test sample such as body fluid or tissue. The antibody of the present invention can also be used to prepare an antibody column for purification of an NPR3 species, to detect an NPR3 species in each fraction during purification, to analyze the behavior of NPR3 in subject cells, and the like.

(8) Screening a compound that changes the amount of NPR3 in the cell membrane

Because the antibody of the present invention is capable of specifically recognizing an NPR3 species, it can be used for screening a compound that changes the amount of NPR3 in the cell membrane.

Accordingly, the present invention provides, for example:

(i) a method of screening a compound that changes the amount of NPR3 in the cell membrane, which comprises disrupting (1) blood, (2) a particular organ, or (3) a tissue, cells or the like isolated from an organ, of a non-human mammal, then isolating a cell membrane fraction, and quantifying the NPR3 contained the cell membrane fraction,

(ii) a method of screening a compound that changes the amount of NPR3 in the cell membrane, which comprises disrupting a transformant expressing NPR3 or a partial peptide thereof, or the like, then isolating a cell membrane fraction, and quantifying the NPR3 species contained in the cell membrane fraction,

(iii) a method of screening a compound that changes the amount of NPR3 in the cell membrane, which comprises identifying NPR3 on the cell membrane by cutting into sections (1) blood, (2) a particular organ, or (3) a tissue, cells or the like isolated from an organ, of a non-human mammal, then quantifying the extent of staining of NPR3 on the cell surface layer using an immunostaining technique, and

(iv) a method of screening a compound that changes the amount of NPR3 species in the cell membrane, which comprises identifying the NPR3 species on the cell membrane by cutting into sections a transformant expressing NPR3 or a partial peptide thereof, or the like, then quantifying the extent of staining of the NPR3 species on the cell surface layer using an immunostaining technique.

Quantitation of the NPR3 species contained in the cell membrane fraction is specifically performed as described below. (i) A drug (for example, anti-obesity drugs, anti-diabetic drugs, antihypertensive drugs, vasoactive drugs, anticancer agents, anti-osteoporotic drugs, antirheumatic drugs and the like) or a physical stress (for example, soaking stress, electric shock, brightness/darkness, low temperatures and the like) or the like is given to a normal or disease model non-human mammal (for example, mice, rats, rabbits, sheep, swine, cattle, cats, dogs, monkeys and the like, more specifically, obese mice, diabetic mice, hypertensive rats, arteriosclerotic rabbits, cancer-bearing mice, osteoporotic mice, chronic rheumatoid arthritic mice and the like); after a given time has elapsed, blood or a particular organ (for example, liver, kidney, pancreas, muscles, bone, joint and the like), a tissue (for example, brown or white adipose tissue, pancreatic islet, bone tissue, cartilage and the like) or cells (for example, adipocytes, muscle cells, pancreatic endocrine cells, hepatocytes, renal cells, osteocytes, osteoblasts, osteoclasts, chondrocytes, chondroblasts, chondroclasts or the like) are obtained. The cells and the like obtained are suspended in an appropriate buffer solution (for example, Tris-HCl buffer solution, phosphate buffer solution, HEPES buffer solution and the like) and the like, and the cells or the like are disrupted using a surfactant (for example, Triton X100™, Tween 20™ and the like) and the like, and further treated using techniques such as centrifugation, filtration and column fractionation to yield a cell membrane fraction.

The cell membrane fraction refers to a fraction rich in cell membrane obtained by a method known per se after cell disruption. As the method of cell disruption, cell squashing using a Potter-Elvehjem homogenizer, disruption using a Waring blender or Polytron (manufactured by Kinematica Inc.), disruption by ultrasonication, disruption by cell spraying through a thin nozzle under an increased pressure using a French press or the like, and the like can be mentioned. For cell membrane fractionation, fractionation by centrifugal forces, such as fractional centrifugation or density gradient centrifugation, is mainly used. For example, a cell disruption fluid is centrifuged at a low speed (500 rpm to 3000 rpm) for a short time (normally about 1 to 10 minutes), the supernatant is centrifuged at a higher speed (15000 rpm to 30000 rpm) normally for 30 minutes to 2 hours, and the precipitate obtained is used as the membrane fraction. The membrane fraction is rich in NPR3 species and cell-derived membrane components such as phospholipids and membrane proteins.

The NPR3 species contained in the cell membrane fraction can be quantified by, for example, a sandwich immunoassay using the antibody of the present invention, Western blot analysis and the like.
The sandwich immunoassay can be performed in the same manner as the method described above, and Western blot can be performed by a means known per se.

(ii) A transformant expressing NPR3 or a partial peptide thereof can be prepared according to the method described above, and the NPR3 species contained in the cell membrane fraction can be quantified.

Screening of a compound that changes the amount of NPR3 in the cell membrane can be performed by:

(i) administering a test compound to a normal or disease model non-human mammal at a given time before (before 30 minutes to before 24 hours, preferably before 30 minutes to before 12 hours, more preferably before 1 hour to before 6 hours) or after (after 30 minutes to after 3 days, preferably after 1 hour to after 2 days, more preferably after 1 hour to after 24 hours) a drug, a physical stress or the like is given, or at the same time as the drug or physical stress, and quantifying the amount of NPR3 in the cell membrane after a given time has elapsed after administration (after 30 minutes to after 3 days, preferably after 1 hour to after 2 days, more preferably after 1 hour to after 24 hours), and can also be preformed by:
(ii) mixing a test compound in a medium at the start of cultivation of a transformant according to a conventional method, and quantifying the amount of NPR3 species in the cell membrane after cultivation for a given time (after 1 day to after 7 days, preferably after 1 day to after 3 days, more preferably after 2 days to after 3 days).

Identification of the NPR3 species contained in the cell membrane fraction is specifically performed as described below. (iii) A drug (for example, anti-obesity drugs, anti-diabetic drugs, antihypertensive drugs, vasoactive drugs, anticancer agents, anti-osteoporotic drugs, antirheumatic drugs and the like) or a physical stress (for example, soaking stress, electric shock, brightness/darkness, low temperatures and the like) or the like is given to a normal or disease model non-human mammal (for example, mice, rats, rabbits, sheep, swine, cattle, cats, dogs, monkeys and the like, more specifically, obese mice, diabetic mice, hypertensive rats, arteriosclerotic rabbits, cancer-bearing mice, osteoporotic mice, chronic rheumatoid arthritic mice and the like); after a given time has elapsed, blood or a particular organ (for example, liver, kidney, pancreas, muscles, bone, joint and the like), a tissue (for example, brown or white adipose tissue, pancreatic islet, bone tissue, cartilage and the like) or cells (for example, adipocytes, muscle cells, pancreatic endocrine cells, hepatocytes, renal cells, osteocytes, osteoblasts, osteoclasts, chondrocytes, chondroblasts, chondroclasts and the like) are obtained. The cells or the like obtained are cut into tissue sections according to a conventional method, and immunostaining is performed using the antibody of the present invention. The amount of NPR3 species in the cell membrane can be determined, quantitatively or qualitatively, by quantifying the extent of staining of NPR3 on the cell surface layer to identify the NPR3 on the cell membrane.

(iv) The NPR3 species contained in a cell membrane fraction can also be identified in a similar way using a transformant expressing NPR3 or a partial peptide thereof and the like.

A compound obtained using the above-described screening method or a salt thereof is a compound having an action to alter the amount of NPR3 in the cell membrane, specifically, (a) a compound that enhances a cell stimulatory activity via a musclin-NPR3 interaction (for example, intracellular cAMP production, inositol phosphate production, Ca uptake, cGMP production, sugar uptake in cells/glycogen synthesis/degradation in cells, sugar release from cells, fat uptake in cells, fat release from cells, fat degradation in cells, fat synthesis in cells, proteinous/non-proteinous hormone secretion regulation, proteinous/non-proteinous hormone action regulation, appetite regulation, motor activity regulation, changes in body weight, changes in saccharides/lipids in blood, regulation of muscle cell differentiation and proliferation, osteoblast differentiation and proliferation and the like, and the like) by increasing the amount of NPR3 in the cell membrane, or (b) a compound that weakens the cell stimulatory activity by reducing the amount of NPR3 in the cell membrane.
As the compound used, peptides, proteins, non-peptide compounds, synthetic compounds, fermentation products and the like can be mentioned; these compounds may be novel compounds or known compounds.
A compound that enhances the cell stimulation activity is useful as a safe and less toxic pharmaceutical for enhancing a physiological activity of NPR3.
A compound that weakens the cell stimulation activity is useful as a safe and less toxic pharmaceutical for reducing a physiological activity of NPR3.

(9) Prophylactic/therapeutic agents for various diseases comprising a compound that alters the amount of NPR3 in the cell membrane

Because NPR3 is expressed specifically in skeletal muscles and functions as a receptor of musclin, which is highly expressed in diabetes model mice and post-fasting refeeding, and also because NPR3 is expressed in bone and can influence bone/cartilage metabolism, and for other reasons, as stated above, NPR3 is thought to play an important role in the regulation of (i) cell differentiation and/or (ii) metabolism (for example, cell differentiation and/or metabolism (for example, sugar/lipid/protein metabolism) in the liver, fat tissue, skeletal muscles, pancreatic island, bone, cartilage and the like). Therefore, a compound that alters the amount of NPR3 in the cell membrane can be used as a regulator of (i) cell differentiation and/or (ii) metabolism (for example, cell differentiation and/or metabolism (for example, sugar/lipid/protein metabolism) in the liver, fat tissue, skeletal muscles, pancreatic island, bone, cartilage and the like), preferably, as a prophylactic/therapeutic agent for abnormalities of cell differentiation and/or metabolism (specifically, for example, obesity, diabetes, impaired glucose tolerance, arteriosclerosis, hypertension, hyperlipemia, metabolic bone/cartilage diseases and the like).
Because the preparation thus obtained is safe and less toxic, it can be administered to, for example, mammals (for example, humans, rats, rabbits, sheep, swine, cattle, cats, dogs, monkeys and the like).
The dose of the compound or a salt thereof varies depending on the subject of administration, target organ, symptoms, method of administration and the like; in oral administration, the dose is normally about 0.1 to 100 mg, preferably about 1.0 to 50 mg, and more preferably about 1.0 to 20 mg, per day, in, for example, a patient with a sugar/lipid metabolic abnormality (assuming a 60 kg body weight). In parenteral administration, the single dose varies depending on the subject of administration, target organ, symptoms, method of administration and the like; for example, in the form of an injection, the dose is normally about 0.01 to 30 mg, preferably about 0.1 to 20 mg, and more preferably about 0.1 to 10 mg, per day, in, for example, a patient with a sugar/lipid metabolic abnormality (assuming a 60 kg body weight). In cases where the subject of administration is other than a human, a dose corresponding to a human dose per 60 kg body weight can be administered.

(10) Preparation of non-human transgenic animal bearing a DNA encoding NPR3

The present invention provides a non-human mammal bearing a DNA encoding exogenous NPR3 (hereinafter abbreviated as the exogenous DNA of the present invention) or a variant DNA thereof (sometimes abbreviated as the exogenous variant DNA of the present invention).
Accordingly, the present invention provides:

[1] a non-human mammal bearing the exogenous DNA of the present invention or a variant DNA thereof,

[2] the animal described in term [1], wherein the non-human mammal is a rodent,
[3] the animal described in term [2], wherein the rodent is a mouse or a rat, and
[4] a recombination vector comprising the exogenous DNA of the present invention or a variant DNA thereof, and expressible in a mammal.

A non-human mammal bearing the exogenous DNA of the present invention or a variant DNA thereof (hereinafter abbreviated as the DNA-transfected animal of the present invention) can be prepared by transferring a desired DNA into an unfertilized egg, a fertilized egg, a spermatozoon, a germinal cell (including its precursor cell) or the like, preferably in the embryogenic stage in the development of the non-human mammal (more preferably in the single-cell or fertilized-egg stage and generally before the 8-cell phase), by the calcium phosphate method, the electric pulse method, the lipofection method, the aggregation method, the microinjection method, the particle gun method, the DEAE-dextran method or the like. Also, it is possible to transfer the exogenous DNA of the present invention into a somatic cell, a living organ, a tissue cell or the like by the DNA transfer method, and utilize them for cell culture, tissue culture and the like, and these cells may be fused with the above-described germinal cells by a method of cell fusion known per se to create the DNA transgenic animal of the present invention.
As examples of the non-human mammal used, cattle, swine, sheep, goat, rabbits, dogs, cats, guinea pigs, hamsters, mice, rats and the like can be mentioned. In particular, preferred from the viewpoint of creating a pathologic animal model system are rodents, especially mice (for example, C57BL/6 line, DBA2 line and the like, which are pure lines, $B6C3F_1$ line, $BDF_1$ line, $B6D2F_1$ line, BALB/c line, ICR line and the like, which are cross lines) or rats (for example, Wistar, SD and the like), since they have relatively short ontogeny and lifecycles and are easy to breed.
As "the mammal" in a recombination vector expressible in a mammal, humans and the like can be mentioned, in addition to the above-described non-human mammals.
The exogenous DNA of the present invention refers to the DNA of the present invention once isolated/extracted from a mammal, rather than the DNA of the present invention inherently possessed by a non-human mammal.
As the variant DNA of the present invention, those resulting from a variation (for example, mutations and the like) in the base sequence of the original DNA of the present invention, specifically DNAs having a base addition, deletion, substitution with another base or the like, and the like can be mentioned, and abnormal DNAs are included.
The abnormal DNA means a DNA that allows the expression of abnormal NPR3, and is exemplified by a DNA that allows the expression of a protein that suppresses the function of normal NPR3, and the like.
The exogenous DNA of the present invention may be derived from a mammal of any of the same species as, or a different species from, the subject animal. In transferring the DNA of the present invention into the subject animal, it is generally advantageous to use the DNA of the present invention as a DNA construct having the DNA bound downstream of a promoter capable of allowing the expression of the DNA in animal cells. For example, when the human DNA of the present invention is transferred, a DNA-transfected mammal that highly expresses the DNA of the present invention can be prepared by microinjecting a DNA construct (e.g., vector and the like) having the human DNA of the present invention ligated downstream of various promoters allowing the expression of a DNA derived from various mammals (for example, rabbits, dogs, cats, guinea pigs, hamsters, rats, mice and the like) bearing the DNA of the present invention, which is highly homologous to the human DNA, into a fertilized egg of the subject mammal, for example, a mouse fertilized egg.
As the expression vector for carrying the DNA of the present invention, *Escherichia coli*-derived plasmids, *Bacillus subtilis*-derived plasmids, yeast-derived plasmids, bacteriophages such as λ phage, retroviruses such as Moloney leukemia virus, animal viruses such as vaccinia virus and baculovirus, and the like can be used. In particular, *Escherichia coli*-derived plasmids, *Bacillus subtilis*-derived plasmids, yeast-derived plasmids and the like are preferably used.
As examples of the promoter that regulates the DNA expression, 1) promoters of DNA derived from viruses (e.g., simian virus, cytomegalovirus, Moloney mouse leukemia virus, JC virus, breast cancer virus, poliovirus and the like) and 2) promoters derived from various mammals (humans, rabbits, dogs, cats, guinea pigs, hamsters, rats, mice and the like), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase β I subunit, dystrophin, tartrate-resistant alkaline phosphatase, atrial natriuretic factor, endothelial receptor tyrosine kinase (generally abbreviated as Tie2), sodium-potassium adenosine triphosphorylase (Na, K-ATPase), neurofilament light chain, metallothioneins I and IIA, metalloproteinase 1 tissue inhibitor, MHC class I antigen (H-2L), H-ras, renin, dopamine β-hydroxylase, thyroid peroxidase (TPO), peptide chain elongation factor 1α (EF-1α), β actin, α and β myosin heavy chains, myosin light chains 1 and 2, myelin basic protein, thyroglobulin, Thy-1, immunoglobulins, H-chain variable region (VNP), serum amyloid P component, myoglobin, troponin C, smooth muscle α actin, preproencephalin A, vasopressin and the like can be used. Among them, the cytomegalovirus promoter, the human protein elongation factor 1α (EF-1α) promoter, the human and chicken β actin promoters and the like, which enable high expression in the whole body, are preferred.
The above-described vector preferably has a sequence that terminates the transcription of desired messenger RNA in the DNA-transfected animal (generally referred to as a terminator); for example, sequences of DNAs derived from viruses or various mammals can be used, with preference given to the simian virus SV40 terminator and the like.

In addition, for the purpose of increasing the expression of the desired exogenous DNA, the splicing signal and enhancer region of each DNA, a portion of the intron of an eukaryotic DNA and the like may also be joined upstream of the 5' end of the promoter region, between the promoter region and the translational region, or downstream of the 3' end of the translational region, depending on the purpose of use.

The translational region of normal NPR3 can be acquired as the whole genomic DNA or a portion thereof from DNAs derived from the liver, kidney, adipose tissue, or muscle of various mammals (for example, humans, rabbits, dogs, cats, guinea pigs, hamsters, rats, mice and the like) and from various commercially available genomic DNA libraries, or using as the source material a cDNA prepared by a known method from RNA derived from the aforementioned organs or tissues. Also, an exogenous abnormal DNA can be obtained by mutating the translational region of normal NPR3 obtained from the above-described cells or tissues by point mutagenesis.

The translational region can be prepared as a DNA construct expressible in the transgenic animal by an ordinary DNA engineering technique wherein the DNA is joined downstream of the aforementioned promoter (and, if desired, upstream of the transcription termination site).

The exogenous DNA of the present invention at the fertilized egg cell stage is transferred in a manner such that the DNA is certainly present in all the germinal cells and somatic cells of the subject mammal. The presence of the exogenous DNA of the present invention in the germinal cells of the animal prepared by DNA transfer means that all offspring of the animal prepared will carry the exogenous DNA of the present invention in all of the germinal cells and somatic cells thereof. The offspring of the animal of this species that have inherited the exogenous DNA of the present invention have the exogenous DNA of the present invention in all of the germinal cells and somatic cells thereof.

A non-human mammal transfected with the normal exogenous DNA of the present invention can be grown over generations as animals bearing that DNA in an ordinary breeding environment after being confirmed to stably retain the exogenous DNA by mating.

The exogenous DNA of the present invention at the fertilized egg cell stage is transferred in a manner such that the DNA is present in excess in all of the germinal cells and somatic cells of the subject mammal. The presence of the exogenous DNA of the present invention in excess in the germinal cells of the animal prepared by DNA transfer means that all offspring of the animal prepared will carry the exogenous DNA of the present invention in excess in all of the germinal cells and somatic cells thereof. The offspring of the animal of this species that have inherited the exogenous DNA of the present invention have the exogenous DNA of the present invention in excess in all of the germinal cells and somatic cells thereof.

By obtaining a homozygous animal having the transferred DNA in both the homologous chromosomes, and mating a male and female of this animal, the animal can be grown over generations so that all offspring thereof will retain the DNA in excess.

A non-human mammal having the normal DNA of the present invention highly expresses the normal DNA of the invention; the normal DNA may eventually cause NPR3 hyperfunction by enhancing the function of the endogenous normal DNA, and the animal can be used as a pathologic model animal for the disease. For example, using an animal transfected with the normal DNA of the present invention, it is possible to elucidate the pathological mechanisms of NPR3 hyperfunction and diseases associated with NPR3, and to investigate therapeutic methods for these diseases.

Also, because a mammal transfected with the exogenous normal DNA of the present invention has a symptom of increased free NPR3, it can be used for screening tests for therapeutic drugs for diseases associated with NPR3.

On the other hand, a non-human mammal bearing the exogenous abnormal DNA of the present invention can be grown over generations as an animal bearing the DNA in an ordinary breeding environment after being confirmed to stably retain the exogenous DNA by mating. Furthermore, the desired exogenous DNA can be used as the source material as incorporated in the aforementioned plasmid. A DNA construct with a promoter can be prepared by an ordinary DNA engineering technique. The abnormal DNA of the present invention at the fertilized egg cell stage is transferred in a manner such that the DNA is present in all of the germinal cells and somatic cells of the subject mammal. The presence of the abnormal DNA of the present invention in the germinal cells of the animal prepared by DNA transfer means that all offspring of the animal prepared will carry the abnormal DNA of the present invention in all of the germinal cells and somatic cells thereof. The offspring of the animal of this species that have inherited the exogenous DNA of the present invention have the abnormal DNA of the present invention in all of the germinal cells and somatic cells thereof. By obtaining a homozygous animal having the introduced DNA in both the homologous chromosomes, and mating a male and female of this animal, the animal can be grown over generations so that all the offspring thereof will carry the DNA.

A non-human mammal bearing the abnormal DNA of the present invention highly expresses the abnormal DNA of the present invention; the DNA may finally cause functionally inactive adiaphoria to NPR3 by inhibiting the function of endogenous normal DNA, and the animal can be utilized as a pathological model animal for the disease. For example, using an animal transfected with the abnormal DNA of the present invention, it is possible to elucidate the pathological mechanism of functionally inactive adiaphoria to NPR3, and to investigate a therapeutic method for this disease.

Regarding specific applicability, an animal highly expressing the abnormal DNA of the present invention serves as a model for elucidating the functional impairment (dominant negative action) of normal NPR3 due to abnormal NPR3 in functionally inactive adiaphoria to NPR3.

Because a mammal transfected with the exogenous abnormal DNA of the present invention has a symptom of increased free abnormal NPR3, it can also be utilized for screening tests for a therapeutic drug for functionally inactive adiaphoria to NPR3.

As examples of other potential applications of the above-described two kinds of DNA-transfected animal of the present invention, the following can be mentioned:

(1) use as cell sources for tissue culture,
(2) analysis of association with proteins specifically expressed or activated by NPR3 and the like, by a direct analysis of DNA or RNA in a tissue of the DNA-transfected animal of the present invention, or by an analysis of the NPR3 produced,
(3) research into the functions of cells generally difficult-to-culture tissues using cells of DNA-bearing tissues cultured by standard tissue culture technology,
(4) screening of a drug that enhances a function of cells using the cells described in (3) above,
(5) isolation and purification of variant NPR3 and preparation of its antibody, and the like.

Furthermore, using the DNA-transfected animal of the present invention, it is possible to examine the clinical symptoms in diseases associated with NPR3, including functionally inactive adiaphoria to NPR3 and the like, and it is also possible to obtain more extensive pathological findings in various organs of a model of a disease associated with NPR3, thus contributing to the development of new therapeutic methods, and to research and treatment for secondary diseases due to the disease.

It is also possible to excise each organ from the DNA-transfected animal of the present invention, shred the organ, then acquire liberated DNA-transfected cells using a protein-degrading enzyme such as trypsin, and culture the cells or establish a line of the cultured cells. Furthermore, it is possible to examine the associations with identification, apoptosis, differentiation or growth of NPR3-producing cells, or the signal transmission mechanisms involved therein, to examine abnormalities thereof, and the like, thus providing effective research materials for the elucidation of the action of NPR3.

Furthermore, for the development of therapeutic drugs for diseases associated with NPR3, including functionally inactive adiaphoria to NPR3, using the DNA-transfected animal of the present invention, it is possible to provide an effective and rapid screening method for the therapeutic drugs for the diseases, using the above-described test method, quantification method and the like. Also, using the DNA-transfected animal of the present invention or the exogenous DNA expression vector of the present invention, it is possible to investigate and develop a DNA therapy for a disease associated with NPR3.

(11) Preparation of non-human knockout animal having the NPR3-encoding gene inactivated therein

The present invention provides non-human mammalian embryonic stem cells having the DNA of the present invention inactivated therein and a non-human mammal deficient in the expression of the DNA of the present invention. Accordingly, the present invention provides:

[1] non-human mammalian embryonic stem cells having the DNA of the present invention inactivated therein,
[2] the embryonic stem cells described in term [1], wherein the DNA has been inactivated by introducing a reporter gene (e.g., the β-galactosidase gene derived from *Escherichia coli*),
[3] the embryonic stem cells described in term [1], which is resistant to neomycin,
[4] the embryonic stem cells described in term [1], wherein the non-human mammal is a rodent,
[5] the embryonic stem cells described in term [4], wherein the rodent is a mouse,
[6] a non-human mammal deficient in the expression of the DNA of the present invention, wherein the DNA has been inactivated,
[7] the non-human mammal described in term [6], wherein the DNA has been inactivated by introducing a reporter gene (e.g., the β-galactosidase gene derived from *Escherichia coli*), the reporter gene being expressible under the control of a promoter for the DNA of the present invention,
[8] the non-human mammal described in term [6], wherein the non-human mammal is a rodent,
[9] the non-human mammal described in term [8], wherein the rodent is a mouse, and
[10] a method of screening a compound that promotes or inhibits the promoter activity for the DNA of the present invention or a salt thereof, which comprises administering a test compound to the animal described in term [7], and detecting the expression of the reporter gene.

Non-human mammalian embryonic stem cells having the DNA of the present invention inactivated therein refer to embryonic stem cells (hereinafter abbreviated as ES cells) of a non-human mammal, wherein the DNA has substantially no capability of NPR3 expression (hereinafter also referred to as the knockout DNA of the present invention) as a result of artificially mutating the DNA of the present invention possessed by the non-human mammal to suppress the capability of DNA expression, or to substantially deprive of the activity of the NPR3 encoded by the DNA.
The non-human mammal is exemplified by the same examples as those mentioned above.
The method of artificially mutating the DNA of the present invention can be performed by, for example, deleting a portion or whole of the DNA sequence, or inserting, or replacing with, another DNA, by a gene engineering technique. For example, by shifting the reading frame of codon or destroying the function of promoter or exon with these mutations, the knockout DNA of the present invention may be prepared.

Specifically, non-human mammalian embryonic stem cells having the DNA of the present invention inactivated therein (hereinafter abbreviated as the DNA-inactivated ES cells of the present invention or the knockout ES cells of the present invention) can be obtained by isolating the DNA of the present invention existing in the desired non-human mammal; constructing a DNA strand having a DNA sequence to destroy the gene by inserting a drug resistance gene represented by the neomycin resistance gene and the hygromycin resistance gene, or a reporter gene represented by lacZ (β-galactosidase gene) and cat (chloramphenicol acetyltransferase gene) or the like into the exon moiety of the DNA of the present invention to destroy the exon function, or inserting a DNA sequence that terminates gene transcription (for example, poly A addition signal and the like) into the intron moiety between exons to prevent the synthesis of complete mRNA; transferring the DNA strand into a chromosome of the animal by, for example, homologous recombination; analyzing the thus-obtained ES cells by a Southern hybridization analysis using a DNA sequence on the DNA of the present invention or in the vicinity thereof as a probe, or by a PCR method using a DNA sequence on the targeting vector and a DNA sequence in the vicinity other than the DNA of the present invention used to prepare the targeting vector, as primers; and selecting the knockout ES cells of the present invention.
The starting ES cell to have the DNA of the present invention inactivated therein by homologous recombination and the like may be of an already established cell line as described above, or of a cell line newly established by the known method of Evans and Kaufman. For example, in the case of mouse ES cells, an ES cell of the 129 line is currently generally used as the starting cell; however, since the immunological background of the 129 line is unclear, an ES cell line established using, for example, C57BL/6 mice, or $BDF_1$ mice ($F_1$ between C57BL/6 and DBA/2), which have been improved by crossing with DBA/2 to increase the otherwise small number of collectable eggs of C57BL/6, and the like can, also be used advantageously, for the purpose of obtaining a pure line of ES cells of clear immunological genetic background, and the like. Because $BDF_1$ mice are backgrounded by C57BL/6 mice, in addition to being advantageous in that the number of collectable eggs is large and the eggs are tough, the ES cells obtained using a $BDF_1$ mouse, when used to create a pathological model mouse, can be used advantageously in that the genetic background thereof can be replaced with C57BL/6 mouse by back-crossing with C57BL/6 mouse.
For establishing a line of ES cells, blastocysts at day 3.5 after fertilization are normally used; in addition, by collecting 8-cell embryos and culturing them to the blastocyst stage, a large number of early embryos can be obtained efficiently. Although the ES cells used may be of either sex, male ES cells are normally more convenient in creating a germ line chimera. Also, to reduce operating time for painstaking cultivation, it is desirable to make sex identification as early as possible.

As an example of the method of sex identification of ES cells, a method that comprises amplifying and detecting a gene in the sex-determining region on Y chromosome by a PCR method can be mentioned. Using this method, a number of ES cells in one colony (about 50 cells) is enough for karyotype analysis, compared to the conventional method, which requires about $10^6$ cells for the same purpose; therefore, it is possible to perform primary selection of ES cells in the early stage of cultivation by sex identification, thus enabling a significant reduction in operating time in the early stage of cultivation because early selection of male cells has become possible.
Secondary selection can be performed by, for example, confirming the chromosome number by the G-banding method, and the like. Although the chromosome number of the ES cells obtained is desirably 100% of the normal number, it is desirable that a gene of the ES cells is knocked out and then re-cloned into normal cells (for example, cells having a chromosome number of 2n = 40 for mice) if a 100% number is difficult to achieve due to physical procedures during cell line establishment, and the like.
Although the embryonic stem cell line thus obtained generally shows very good growing capacity, it is likely to lose its capability of ontogeny, so that it must be subcultured carefully. For example, cells of this line are cultured using a method of cultivation on appropriate feeder cells such as STO fibroblasts in the presence of LIF (1 to 10000 U/ml) in a carbon dioxide incubator (preferably under 5% carbon dioxide and 95% air or under 5% oxygen, 5% carbon dioxide and 90% air) at about 37°C, or the like; they are subcultured using, for example, a method comprising a treatment with a trypsin/EDTA solution (normally 0.001% to 0.5% trypsin/0.1 to 5 mM EDTA, preferably about 0.1% trypsin/1 mM EDTA) to give single cells, and sowing them on newly prepared feeder cells, or the like. This subculture is normally performed every 1 to 3 days, during which it is desirable that the cells be examined, and any morphologically abnormal cells, if found, are desirably discarded.

ES cells can be differentiated into various types of cells, such as the parietal muscle, visceral muscles, and myocardium when subjected to monolayer culture to the extent of a high density, or to suspension culture to the extent of the formation of a cell mass, under appropriate conditions [M.J. Evans and M.H. Kaufman, Nature, Vol. 292, p. 154, 1981; G. R. Martin, Proceedings of the National Academy of Sciences of the USA (Proc. Natl. Acad. Sci. U.S.A.), Vol. 78, p. 7634, 1981; T.C. Doetschman et al., Journal of Embryology and Experimental Morphology (J. Embryol. Exp. Morphol.), Vol. 87, p. 27, 1985], and cells deficient in the expression of the DNA of the present invention obtained by differentiating the ES cells of the present invention are useful in cytobiological investigations of NPR3 or NPR3 in vitro.

A non-human mammal deficient in the expression of the DNA of the present invention can be distinguished from a normal animal by measuring the amounts of mRNA in the animals by a known method, and indirectly comparing the expression amounts thereof.

The non-human mammal is exemplified by the same examples as those mentioned above.

A non-human mammal deficient in the expression of the DNA of the present invention can be produced by, for example, introducing a targeting vector prepared as described above into a mouse embryonic stem cell or mouse ovum to cause homologous recombination to replace the DNA sequence having the DNA of the present invention inactivated therein in the introduced targeting vector with the DNA of the present invention on a chromosome of the mouse embryonic stem cell or mouse ovum, whereby the DNA of the present invention can be knocked out. Since many recombinations in mammals are non-homologous, as examples of means of screening cells having undergone homologous recombination, a method that comprises inserting a drug resistance gene such as the neomycin resistance gene into the DNA of the present invention, constructing a targeting vector comprising the thymidine kinase (tk) gene in the vicinity of the DNA of the present invention, introducing the vector into an embryonic stem cell or ovum, and selecting cells that survive in the presence of a drug corresponding to the inserted drug resistance gene (for example, G418 and the like for the neomycin resistance gene) and ganciclovir, can be mentioned. That is, if the insertion variant DNA of the present invention is incorporated onto a chromosome by homologous recombination, the cells are resistant to ganciclovir because the tk gene is eliminated, whereas in the case of incorporation by non-homologous recombination, the cells are susceptible to ganciclovir because the tk gene is incorporated at the same time. If the diphtheria toxin gene or the like is used instead of the tk gene, cells undergoing random insertion will die due to the production of the toxin, so that selection with a single drug is possible.

The final confirmation of cells having the DNA of the present invention knocked out therein can be achieved by a Southern hybridization analysis using a DNA sequence on the DNA of the present invention or in the vicinity thereof as a probe, or by a PCR method using a DNA sequence on the targeting vector and a DNA sequence in the vicinity other than the mouse-derived DNA of the present invention used in the targeting vector, as primers.

When a non-human mammalian embryonic stem cell is used, a cell line having the DNA of the present invention inactivated therein by homologous gene recombination is cloned, its cell is injected into a non-human mammalian embryo or blastocyst at an appropriate stage, for example, the 8-cell stage, and the chimeric embryo prepared is transplanted into the uterus of a pseudopregnant female of the non-human mammal. The animal created is a chimeric animal consisting of both cells having the normal locus of the DNA of present invention and cells having an artificially mutated locus of the DNA of the present invention.

When some of the germ cells of the chimeric animal have a mutated locus of the DNA of the present invention, such a chimeric individual and a normal individual may be mated; from the animal population thus obtained, an individual all of whose tissues consist of cells having an artificially mutated locus of the DNA of the present invention can be selected by, for example, coat color judgment and the like. Because the individual thus obtained is normally a hetero-expression-deficient individual, such hetero-expression-deficient individuals may be mated to obtain an individual homo-deficient in the expression of the protein of the present invention out of the pups thereof.

When an ovum is used, for example, a transgenic non-human mammal incorporating a targeting vector in a chromosome can be obtained by injecting a DNA solution into the nucleus of the ovum by the microinjection method, and such an individual is obtained by selecting one having a mutation in the locus of the DNA of the present invention by homologous gene recombination from among these transgenic non-human mammals.

An individual having the DNA of the present invention thus knocked out therein can also be grown over generations in an ordinary breeding environment after animal individuals obtained by mating as well are confirmed to have the DNA knocked out therein.

Furthermore, establishment and maintenance of a germ line can be performed according to conventional methods. That is, homozygous animals having the inactivated DNA in both the homologous chromosomes can be obtained by mating a male and female animal bearing the inactivated DNA. These homozygous animals can be efficiently propagated by breeding in a ratio of one normal individual and a plurality of homozygous animals relative to the dam. By mating a male and female heterozygous animal, homozygous and heterozygous animals having the inactivated DNA are propagated over generations.

Non-human mammalian embryonic stem cells having the DNA of the present invention inactivated therein are highly useful in creating a non-human mammal deficient in the expression of the DNA of the present invention.

Also, because a non-human mammal deficient in the expression of the DNA of the present invention lacks various biological activities induced by NPR3, it can serve as models of diseases caused by inactivation of biological activities of NPR3, and is hence useful in elucidating the causes of these diseases and investigating therapies therefor.

(11a) Method of screening a compound having a therapeutic/prophylactic effect for a disease caused by deficiency, damage and the like of the DNA of the present invention.

A non-human mammal deficient in the expression of the DNA of the present invention can be used to screen a compound having a therapeutic/prophylactic effect for a disease caused by deficiency, damage and the like of the DNA of the present invention.

Accordingly, the present invention provides a method of screening a compound having a therapeutic/prophylactic effect for a disease caused by deficiency, damage and the like of the DNA of the present invention or a salt thereof, which comprises administering a test compound to a non-human mammal deficient in the expression of the DNA of the present invention, and observing and measuring changes in the animal.

As the non-human mammal deficient in the expression of the DNA of the present invention used for the screening method, the same examples as those mentioned above can be mentioned.

As examples of the test compound used, peptides, proteins, non-peptide compounds, synthetic compounds, fermentation products, cell extracts, plant extracts, animal tissue extracts, plasma and the like can be mentioned; these compounds may be novel compounds or known compounds.

Specifically, the therapeutic/prophylactic effect of a test compound can be tested by treating a non-human mammal deficient in the expression of the DNA of the present invention with the test compound, comparing the animal with a non-treated control animal, with the changes in the organs, tissues, disease symptoms and the like in the animals as indexes.

Examples of the method of treating a test animal with a test compound include oral administration, intravenous injection and the like, which can be selected as appropriate for the symptoms of the test animal, the properties of the test compound, and the like. A dose of the test compound can be selected as appropriate according to the method of administration, properties of the test compound and the like.

When a test compound is administered to a test animal in the screening method, the test compound can be selected as a compound having a therapeutic/prophylactic effect for a disease such as impaired glucose tolerance, if the blood glucose level in the test animal decreases by about 10% or more, preferably about 30% or more, and more preferably about 50% or more.

A compound obtained using the screening method is a compound selected from among the above-described test compounds, and can be used as a safe, less toxic therapeutic/prophylactic agent for a disease caused by NPR3 deficiency or damage and the like, for example, an abnormality of (i) cell differentiation and/or (ii) metabolism (for example, cell differentiation and/or metabolism (for example, sugar/lipid/protein metabolism) in the liver, fat tissue, skeletal muscles, pancreatic island, bone, cartilage and the like), specifically, for example, obesity, diabetes, impaired glucose tolerance, arteriosclerosis, hypertension, hyperlipemia, metabolic bone/cartilage diseases and the like.

A compound obtained by the screening method may have formed a salt; as examples of the salt of the compound, physiologically acceptable salts with acids (e.g., inorganic acids, organic acids and the like), bases (e.g., alkali metals and the like) and the like can be mentioned, with preference given to physiologically acceptable acid adduct salts. As examples of such salts, salts with inorganic acids (for example, hydrochloric acid, phosphoric acid, hydrobromic acid, sulfuric acid and the like), salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid and the like) and the like can be mentioned.

When a compound obtained by the screening method or a salt thereof is used as a pharmaceutical, it can be formulated in the same manner as with the aforementioned NPR3 species.

Because the preparation thus obtained is safe and less toxic, it can be administered to, for example, mammals (for example, humans, rats, mice, guinea pigs, rabbits, sheep, swine, cattle, horses, cats, dogs, monkeys and the like).

The dose of the compound or a salt thereof varies depending on the subject of administration, target organ, symptoms, method of administration and the like; in the case of oral administration, the dose is normally about 0.1 to 100 mg, preferably about 1.0 to 50 mg, and more preferably about 1.0 to 20 mg, per day, in, for example, a patient with an abnormality of sugar/lipid metabolism (assuming a body weight of 60 kg). In parenteral administration, the single dose varies depending on the subject of administration, target organ, symptoms, method of administration and the like; for example, in the form of an injection, the dose is normally about 0.01 to 30 mg, preferably about 0.1 to 20 mg, and more preferably about 0.1 to 10 mg, per day, in, for example, a patient with a sugar/lipid metabolic abnormality (assuming a 60 kg body weight). In cases where the subject of administration is other than a human, a dose corresponding to a human dose per 60 kg body weight can be administered.

(11b) Method of screening a compound that promotes or inhibits the activity of a promoter for the DNA of the present invention

The present invention provides a method of screening a compound that promotes or inhibits the activity of a promoter for the DNA of the present invention or a salt thereof, which comprises administering a test compound to a non-human mammal deficient in the expression of the DNA of the present invention, and detecting the expression of a reporter gene.
In the above-described screening method, as the non-human mammal deficient in the expression of the DNA of the present invention, one having the DNA of the present invention inactivated therein by introducing a reporter gene, wherein the reporter gene is expressible under the control of a promoter for the DNA of the present invention, out of the aforementioned non-human mammals deficient in the expression of the DNA of the present invention, is used.
The test compound is exemplified by the same examples as those mentioned above.
As the reporter gene used, the same examples as those mentioned above can be mentioned, with preference given to the β-galactosidase gene (lacZ), the soluble alkaline phosphatase gene or the luciferase gene and the like.
In a non-human mammal deficient in the expression of the DNA of the present invention having the DNA of the present invention replaced with a reporter gene therein, the reporter gene occurs under the control of a promoter for the DNA of the present invention; therefore, promoter activity can be detected by tracing the expression of a substance encoded by the reporter gene.
For example, when a portion of the NPR3-encoding DNA region is replaced with the β-galactosidase gene (lacZ) derived from *Escherichia coli*, β-galactosidase is expressed, in place of NPR3, in tissues where NPR3 is otherwise expressed. Therefore, for example, by staining using a reagent that serves as a substrate for β-galactosidase, such as 5-bromo-4-chloro-3-indolyl-β-galactopyranoside (X-gal), the expression state of NPR3 in the animal body can be examined conveniently. Specifically, an NPR3-deficient mouse or a tissue section thereof is fixed with glutaraldehyde and the like, washed with phosphate-buffered saline (PBS), then reacted with a staining solution containing X-gal at room temperature or around 37°C for about 30 minutes to 1 hour, thereafter the tissue specimen is washed with 1 mM EDTA/PBS solution to stop the β-galactosidase reaction, and the color developed is examined. The lacZ-encoding mRNA may be detected according to a conventional method.
A compound obtained using the above-described screening method or a salt thereof is a compound selected from among the above-described test compounds, and promoting or inhibiting the activity of a promoter for the DNA of the present invention.
A compound obtained by the screening method may have formed a salt; as examples of the salt of the compound, physiologically acceptable salts with acids (e.g., inorganic acids, organic acids and the like), bases (e.g., alkali metals and the like) and the like can be mentioned, with preference given to physiologically acceptable acid adduct salts. As examples of such salts, salts with inorganic acids (for example, hydrochloric acid, phosphoric acid, hydrobromic acid, sulfuric acid and the like), salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid and the like) and the like can be mentioned.
Because a compound that promotes the activity of a promoter for the DNA of the present invention or a salt thereof is capable of promoting the expression of NPR3 and promoting the function of NPR3, it is useful as, for example, a pharmaceutical such as a prophylactic/therapeutic drug for a disease associated with NPR3 dysfunction and the like. Because a compound that inhibits the activity of a promoter for the DNA of the present invention or a salt thereof is capable of inhibiting the expression of NPR3 and inhibiting the function of NPR3, it is useful as, for example, a pharmaceutical such as a prophylactic/therapeutic drug for a disease associated with NPR3 overexpression and the like.
As examples of diseases associated with dysfunction or overexpression of NPR3, an abnormality of (i) cell differentiation and/or (ii) metabolism (for example, cell differentiation and/or metabolism (for example, sugar/lipid/protein metabolism) in the liver, fat tissue, skeletal muscles, pancreatic island, bone, cartilage and the like), specifically, for example, obesity, diabetes, impaired glucose tolerance, arteriosclerosis, hypertension, hyperlipemia, metabolic bone/cartilage diseases and the like can be mentioned.
Furthermore, compounds derived from a compound obtained by the above-described screening can also be used in the same manner.
When a compound obtained by the screening method or a salt thereof is used as a pharmaceutical, it can be formulated in the same manner as with the aforementioned NPR3 species.
Because the preparation thus obtained is safe and less toxic, it can be administered to, for example, mammals (for example, humans, rats, mice, guinea pigs, rabbits, sheep, swine, cattle, horses, cats, dogs, monkeys and the like).
While the dose of the compound or a salt thereof varies depending on the administration subject, target organ, condition, administration method and the like, in oral administration of a compound that promotes or inhibits promoter activity, the dose is normally about 0.1 to 100 mg, preferably about 1.0 to 50 mg, and more preferably about 1.0 to 20 mg, per day, in, for example, a patient with a sugar/lipid metabolic abnormality (assuming a 60 kg body weight). In parenteral administration, the single dose varies depending on the subject of administration, target organ, symptoms, method of administration, and the like; for example, in the form of an injection, the dose is normally about 0.01 to 30 mg, preferably about 0.1 to 20 mg, and more preferably about 0.1 to 10 mg, per day, in, for example, a patient with a sugar/lipid metabolic abnormality (assuming a 60 kg body weight). In cases where the subject of administration is other than a human, a dose corresponding to a human dose per 60 kg body weight can be administered.

As stated above, a non-human mammal deficient in the expression of the DNA of the present invention is highly useful in screening a compound that promotes or inhibits the activity of a promoter for the DNA of the present invention or a salt thereof, and can contribute significantly to the elucidation of the causes of various diseases due to deficiency of the expression of the DNA of the present invention or the development of a prophylactic/therapeutic drug.
Also, provided that a gene encoding one of various proteins is joined downstream of a DNA comprising an NPR3 promoter region, and this DNA construct is injected into an animal ovum to prepare what is called a transgenic animal, it is also possible to allow the animal to specifically synthesize the protein, and investigate its action in vivo. Furthermore, provided that an appropriate reporter gene is bound to the above-described promoter portion, and a cell line that expresses the gene is established, the cell line can be used as a screening system for a low-molecular compound that acts to specifically promote or suppress the in vivo productivity of NPR3 itself.

In the specification and drawings, where bases, amino acids, etc. are denoted by their codes, they are based on conventional codes in accordance with the IUPAC-IUB Commission on Biochemical Nomenclature or by the common codes in the art, examples of which are shown below. For amino acids that may have the optical isomer, L form is presented unless otherwise indicated.

DNA : deoxyribonucleic acid
cDNA : complementary deoxyribonucleic acid
A : adenine
T : thymine
G : guanine
C : cytosine
RNA : ribonucleic acid
mRNA : messenger ribonucleic acid
dATP : deoxyadenosine triphosphate
dTTP : deoxythymidine triphosphate
dGTP : deoxyguanosine triphosphate
dCTP : deoxycytidine triphosphate
ATP : adenosine triphosphate
EDTA : ethylenediaminetetraacetic acid
SDS : sodium dodecyl sulfate

Gly : glycine
Ala : alanine
Val : valine
Leu : leucine
Ile : isoleucine
Ser : serine
Thr : threonine
Cys : cysteine
Met : methionine
Glu : glutamic acid
Asp : aspartic acid
Lys : lysine
Arg : arginine
His : histidine
Phe : phenylalanine
Tyr : tyrosine
Trp : tryptophan
Pro : proline
Asn : asparagine
Gln : glutamine

pGlu : pyroglutamic acid
Me : methyl group
Et : ethyl group
Bu : butyl group
Ph : phenyl group
TC : thiazolidine-4(R)-carboxamide group

Substituents, protecting groups and reagents frequently mentioned herein are represented by the symbols shown below.

Tos : p-toluenesulfonyl
CHO : formyl
Bzl : benzyl
$Cl_2Bzl$ : 2,6-dichlorobenzyl
Bom : benzyloxymethyl
Z : benzyloxycarbonyl
Cl-Z : 2-chlorobenzyloxycarbonyl
Br-Z : 2-bromobenzyloxycarbonyl
Boc : t-butoxycarbonyl
DNP : dinitrophenol
Trt : trityl
Bum : t-butoxymethyl
Fmoc : N-9-fluorenylmethoxycarbonyl
HOBt : 1-hydroxybenztriazole
HOOBt : 3,4-dihydro-3-hydroxy-4-oxo-1,2,3-benzotriazine
HONB : 1-hydroxy-5-norbornane-2,3-dicarboxyimide
DCC : N,N'-dicyclohexylcarbodiimide

The sequence identification numbers in the sequence listing herein show the following sequences.
[SEQ ID NO:1]
Shows the base sequence of the coding sequence of human NPR3 cDNA.

atgccgtctc tgctggtgct cactttctcc ccgtgcgtac tactcggctg ggcgttgctg 60
gccggcggca ccggtggcgg tggcgttggc ggcggcggcg gtggcgcggg cataggcggc 120
ggacgccagg agagagaggc gctgccgcca cagaagatcg aggtgctggt gttactgccc 180
caggatgact cgtacttgtt ttcactcacc cgggtgcggc cggccatcga gtatgctctg 240
cgcagcgtgg agggcaacgg gactgggagg cggcttctgc cgccgggcac tcgcttccag 300
gtggcttacg aggattcaga ctgtgggaac cgtgcgctct tcagcttggt ggaccgcgtg 360
gcggcggcgc ggggcgccaa gccagacctt atcctggggc cagtgtgcga gtatgcagca 420
gcgccagtgg cccggcttgc atcgcactgg gacctgccca tgctgtcggc tggggcgctg 480
gccgctggct tccagcacaa ggactctgag tactcgcacc tcacgcgcgt ggcgcccgcc 540
tacgccaaga tgggcgagat gatgctcgcc ctgttccgcc accaccactg gagccgcgct 600
gcactggtct acagcgacga caagctggag cggaactgct acttcaccct cgaggggggtc 660
cacgaggtct tccaggagga gggtttgcac acgtccatct acagtttcga cgagaccaaa 720
gacttggatc tggaagacat cgtgcgcaat atccaggcca gtgagagagt ggtgatcatg 780
tgtgcgagca gtgacaccat ccggagcatc atgctggtgg cgcacaggca tggcatgacc 840
agtggagact acgccttctt caacattgag ctcttcaaca gctcttccta tggagatggc 900
tcatggaaga gaggagacaa acacgacttt gaagctaagc aagcatactc gtccctccag 960
acagtcactc tactgaggac agtgaaacct gagtttgaga agttttccat ggaggtgaaa 1020
agttcagttg agaaacaagg gctcaatatg gaggattacg ttaacatgtt tgttgaagga 1080
ttccacgatg ccatcctcct ctacgtcttg gctctacatg aagtactcag agctggttac 1140
agcaaaaagg atggagggaa aattatacag cagacttgga acagaacatt tgaaggtatc 1200
gccgggcagg tgtccataga tgccaacgga gaccgatatg gggatttctc tgtgattgcc 1260
atgactgatg tggaggcggg cacccaggag gttattggtg attattttgg aaaagaaggt 1320
cgttttgaaa tgcggccgaa tgtcaaatat ccttggggcc ctttaaaact gagaatagat 1380
gaaaaccgaa ttgtagagca tacaaacagc tctccctgca aatcatgtgg cctagaagaa 1440
tcggcagtga caggaattgt cgtggggggct ttactaggag ctggcttgct aatggccttc 1500
tactttttca ggaagaaata cagaataacc attgagaggc gaacccagca agaagaaagt 1560
aaccttggaa aacatcggga attacgggaa gattccatca gatcccattt ttcagtagct 1620
taa 1623

[SEQ ID NO:2]
Shows the amino acid sequence of human NPR3.

Met Pro Ser Leu Leu Val Leu Thr Phe Ser Pro Cys Val Leu Leu Gly
1 5 10 15
Trp Ala Leu Leu Ala Gly Gly Thr Gly Gly Gly Gly Val Gly Gly Gly
20 25 30
Gly Gly Gly Ala Gly Ile Gly Gly Gly Arg Gln Glu Arg Glu Ala Leu
35 40 45
Pro Pro Gln Lys Ile Glu Val Leu Val Leu Leu Pro Gln Asp Asp Ser
50 55 60
Tyr Leu Phe Ser Leu Thr Arg Val Arg Pro Ala Ile Glu Tyr Ala Leu
65 70 75 80
Arg Ser Val Glu Gly Asn Gly Thr Gly Arg Arg Leu Leu Pro Pro Gly
85 90 95
Thr Arg Phe Gln Val Ala Tyr Glu Asp Ser Asp Cys Gly Asn Arg Ala
100 105 110
Leu Phe Ser Leu Val Asp Arg Val Ala Ala Ala Arg Gly Ala Lys Pro
115 120 125
Asp Leu Ile Leu Gly Pro Val Cys Glu Tyr Ala Ala Ala Pro Val Ala
130 135 140
Arg Leu Ala Ser His Trp Asp Leu Pro Met Leu Ser Ala Gly Ala Leu
145 150 155 160
Ala Ala Gly Phe Gln His Lys Asp Ser Glu Tyr Ser His Leu Thr Arg
165 170 175
Val Ala Pro Ala Tyr Ala Lys Met Gly Glu Met Met Leu Ala Leu Phe
180 185 190
Arg His His His Trp Ser Arg Ala Ala Leu Val Tyr Ser Asp Asp Lys
195 200 205
Leu Glu Arg Asn Cys Tyr Phe Thr Leu Glu Gly Val His Glu Val Phe
210 215 220
Gln Glu Glu Gly Leu His Thr Ser Ile Tyr Ser Phe Asp Glu Thr Lys
225 230 235 240
Asp Leu Asp Leu Glu Asp Ile Val Arg Asn Ile Gln Ala Ser Glu Arg
245 250 255
Val Val Ile Met Cys Ala Ser Ser Asp Thr Ile Arg Ser Ile Met Leu

260 265 270

Val Ala His Arg His Gly Met Thr Ser Gly Asp Tyr Ala Phe Phe Asn

275 280 285

Ile Glu Leu Phe Asn Ser Ser Ser Tyr Gly Asp Gly Ser Trp Lys Arg

290 295 300

Gly Asp Lys His Asp Phe Glu Ala Lys Gln Ala Tyr Ser Ser Leu Gln

305 310 315 320

Thr Val Thr Leu Leu Arg Thr Val Lys Pro Glu Phe Glu Lys Phe Ser

325 330 335

Met Glu Val Lys Ser Ser Val Glu Lys Gln Gly Leu Asn Met Glu Asp

340 345 350

Tyr Val Asn Met Phe Val Glu Gly Phe His Asp Ala Ile Leu Leu Tyr

355 360 365

Val Leu Ala Leu His Glu Val Leu Arg Ala Gly Tyr Ser Lys Lys Asp

370 375 380

Gly Gly Lys Ile Ile Gln Gln Thr Trp Asn Arg Thr Phe Glu Gly Ile

385 390 395 400

Ala Gly Gln Val Ser Ile Asp Ala Asn Gly Asp Arg Tyr Gly Asp Phe

405 410 415

Ser Val Ile Ala Met Thr Asp Val Glu Ala Gly Thr Gln Glu Val Ile

420 425 430

Gly Asp Tyr Phe Gly Lys Glu Gly Arg Phe Glu Met Arg Pro Asn Val

435 440 445

Lys Tyr Pro Trp Gly Pro Leu Lys Leu Arg Ile Asp Glu Asn Arg Ile

450 455 460

Val Glu His Thr Asn Ser Ser Pro Cys Lys Ser Cys Gly Leu Glu Glu

465 470 475 480

Ser Ala Val Thr Gly Ile Val Val Gly Ala Leu Leu Gly Ala Gly Leu

485 490 495

Leu Met Ala Phe Tyr Phe Phe Arg Lys Lys Tyr Arg Ile Thr Ile Glu

500 505 510

Arg Arg Thr Gln Gln Glu Glu Ser Asn Leu Gly Lys His Arg Glu Leu

515 520 525

Arg Glu Asp Ser Ile Arg Ser His Phe Ser Val Ala

530 535 540

The present invention is hereinafter described in further detail by means of the following Examples, which, however, are for illustrative purposes only and do not limit the scope of the invention in any way.

Examples

Example 1 Cloning of a cDNA that encodes mouse musclin (mMusclin) protein

A PCR was performed from the mouse skeletal muscle cDNA library Quick clone cDNA (BD) with the oligo-DNA shown by SEQ ID NO:3 as the sense strand primer, and with the oligo-DNA shown by SEQ ID NO:4 as the antisense strand primer, to yield a sequence upstream of the 5' end with each primer as the starting point (SEQ ID NO:5).
SEQ ID NO:3
TTAGCATCAC AGGAGTTTGG AAC 23
SEQ ID NO:4
TCAGCCTCTG GAACTGGAGA GCC 23

SEQ ID NO:5

TTAGCATCAC AGGAGTTTGG AACAGCAAGC TTGCAGTCTC CACCCACAGC CAGAGAAGAG 60
AAGTCAGCCA CTGAGCTTTC GGCTAAGCTC CTGCGTCTTG ATGATCTGGT GTCCTTAGAG 120
AATGACGTAT TTGAGACCAA GAAAAAGAGA AGCTTCTCTG GCTTTGGGTC TCCCCTTGAC 180
AGACTCTCAG CTGGGTCTGT AGAGCATAGA GGGAAACAAA GGAAAGCAGT AGATCATTCA 240
AAAAAGCGGT TTGGTATTCC CATGGATCGG ATTGGTAGAA ACCGGCTCTC CAGTTCCAGA 300
GGCTGA 306

Example 2 Preparation of His-tag Tyr Cys mouse musclin (HYC-mMusclin) protein

A gene (SEQ ID NO:7) that encodes mature mMusclin deprived of the signal sequence of mMusclin (SEQ ID NO:6) was subcloned into PET-15b (Novagen); on the basis of the pET-15b-mMusclin plasmid vector obtained, by PCR-based site-directed mutagenesis, the vector-derived sequence between the His-tag sequence and the mMusclin-encoding sequence was modified, whereby the plasmid vector pET15M-mMusclin having the ORF shown by SEQ ID NO: 8 was obtained.
The plasmid vector obtained was introduced into competent cells of BL21(DE3) (Novagene) and the like, and protein expression was induced by shaking culture and IPTG, to accumulate the His-Tyr-Cys-mMusclin protein in the cell bodies, and the protein was purified by purification using a chelate column utilizing His-tag.

SEQ ID NO:6

ATGCTGGACT GGAGATTGGC AAGTACACAC TTCATCCTGG CTATGATTGT GATGCTGTGG 60
GGCTCAGGAA AGGCATTCTC TGTGGAC 87

SEQ ID NO:7

TTAGCATCAC AGGAGTTTGG AACAGCAAGC TTGCAGTCTC CACCCACAGC CAGAGAAGAG 60
AAGTCAGCCA CTGAGCTTTC GGCTAAGCTC CTGCGTCTTG ATGATCTGGT GTCCTTAGAG 120
AATGACGTAT TTGAGACCAA GAAAAAGAGA AGCTTCTCTG GCTTTGGGTC TCCCCTTGAC 180
AGACTCTCAG CTGGGTCTGT AGAGCATAGA GGGAAACAAA GGAAAGCAGT AGATCATTCA 240
AAAAAGCGGT TTGGTATTCC CATGGATCGG ATTGGTAGAA ACCGGCTCTC CAGTTCCAGA 300
GGC 303

SEQ ID NO:8

ATGGGCAGCA GCCATCATCA TCATCATCAC AGCAGCGGCC TGGTGCCGTA CGGCTGCCAT 60
ATGTTAGCAT CACAGGAGTT TGGAACAGCA AGCTTGCAGT CTCCACCCAC AGCCAGAGAA 120
GAGAAGTCAG CCACTGAGCT TTCGGCTAAG CTCCTGCGTC TTGATGATCT GGTGTCCTTA 180
GAGAATGACG TATTTGAGAC CAAGAAAAAG AGAAGCTTCT CTGGCTTTGG GTCTCCCCTT 240
GACAGACTCT CAGCTGGGTC TGTAGAGCAT AGAGGGAAAC AAAGGAAAGC AGTAGATCAT 300
TCAAAAAAGC GGTTTGGTAT TCCCATGGAT CGGATTGGTA GAAACCGGCT CTCCAGTTCC 360
AGAGGCTGA 369

Example 3 Preparation of full-length mMusclin and C-terminal mMusclin (mCA) protein

By introducing a fragment amplified with a gene comprising the mMusclin ORF as the template using the primers shown by SEQ ID NO:9 and SEQ ID NO:10 into the LIC multicloning site of the pET41-XaLIC vector (Novagene) by the technique of LIC-cloning, an Escherichia coli expression type plasmid vector that encodes thioredoxin (Trx)-fused mMusclin full-length protein was prepared. Likewise, by amplification using SEQ ID NO:11 and SEQ ID NO:10, an Escherichia coli expression type plasmid vector that encodes thioredoxin-fused mCA protein was prepared.
SEQ ID NO:9
GGTATTGAGG GTCGCTTAGC ATCACAGGAG TTTGGAACAG 40
SEQ ID NO:10
AGAGGAGAGT TAGAGCCTCA GCCTCTGGAA CTGGAGAGCC GG 42
SEQ ID NO:11
GGTATTGAGG GTCGCAGCTT CTCTGGCTTT GGGTCTCCCC 40

The plasmid vector obtained was introduced into competent cells of BL21(DE3) (Novagene) and the like, and protein expression was induced by shaking culture and IPTG to accumulate Trx-His-mMusclin protein or Trx-His-mCA protein in the cell bodies, and the protein was purified by purification using a chelate column utilizing His-tag.
The fusion protein obtained was subjected to limited degradation with Xa, the degradation product was applied to a HiTrap SP-HP column, previously equilibrated with a buffer solution comprising 25 mM HEPES and 100 mM NaCl (pH 7.8), and the binding Trx-His-mMusclin protein or Trx-His-mCA protein was eluted with 25 mM HEPES, 1000 mM NaCl (pH 7.8), whereby purified full-length mMusclin or mCA separate from the fusion protein was obtained.

Example 4 Preparation of biotin-labeled mMusclin

The full-length mMusclin obtained in Example 3, 2.5 mg/ml x 250 μl, was mixed with 45 μl of 10 nmol/μl Sulfo-NHS-LC-Biotin under ice cooling overnight, whereby labeled mMusclin incorporating about 5 molecules of biotin/1 molecule of mMusclin (BK-mMusclin) was prepared.

His-Tyr-Cys-mMusclin prepared by Example 2, 1 mg/ml x 500 μl, was reduced in the presence of EDTA using TCEP (Pierce), and mixed with a final concentration of 0.4 mM of Maleimide PE02 Biotin at room temperature overnight, whereby labeled mMusclin incorporating about 1 molecule of biotin/1 molecule introduced Cys residue specifically (BC-mMusclin) was prepared.

Example 5 Preparation of hNpr-3 expressing FreeStyle293 cells

A hNpr3 expression vector (hNpr3/pCMV6-XL4, NM_000908 Clone# PL1154_A07) was purchased from OriGene. 0.3 μg (Low) or 1.0 μg (high) of the hNpr3 expression vector was transfected transiently per 1.0 x$10^6$ FreeStyle293 cells by a conventional method; 48 hours later, the cells were collected and suspended in PBS-1.0% BSA at 5x$10^6$ cells/ml. A 1-ml portion of each cell lyzate was incubated with a final concentration of 0.5 μg/ml of BK-mMusclin or BC-mMusclin at 4°C for 30 minutes; after 1 time of washing, the lyzate was stained with PE-labeled Streptavidin, and the cells bound to each Musclin were analyzed. Also, incubation was performed in the presence of 100 μg/ml full-length mMusclin, mCA, or His-Tyr-Cys-mMusclin, or 10 μM ANP (Atrial Natriuretic Peptide, Rat, Mouse), CANP (Atrial Natriuretic Peptide 4-23-NH2 Des-[Gln18 Ser19 Gly20 Leu21 Gly22], Rat), or LANP (Atrial Natriuretic Peptide 104-126 Des-[Cys105 Cys121], Rat) (Phoenix Pharmaceuticals, Inc.), whereby changes in the binding of labeled mMusclin were examined. As a result, it was demonstrated that because BK- or BC-labeled mMusclin bound dependently on the amount of the hNpr3 gene introduced into the cells, and also because the binding nearly disappeared in the presence of 100 μg/ml full-length mMusclin, mCA, or His-Tyr-Cys-mMusclin, the binding is specific for mMusclin, and that because the binding nearly disappeared in the presence of 10 μM ANP, CANP, and LANP, which are known ligands of the NPR3 molecule, the binding was dependent on NPR3. Industrial Applicability

According to the present invention, a prophylactic/therapeutic agent and diagnostic agent for a disease associated with an abnormality of cell differentiation or sugar/lipid/protein metabolism in skeletal muscles and the like, and a tool for screening for a pharmaceutical candidate compound that is effective in the prophylaxis/treatment of the disease can be provided.

**Claims**

1. A cell differentiation and/or metabolism regulator comprising a protein comprising the same or substantially the same amino acid sequence as the amino acid sequence shown by SEQ ID NO:2 or a partial peptide thereof or a salt thereof.

2. The agent of claim 1, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism.

3. The agent of claim 1, which is for prevention/treatment of 1 or more diseases selected from the group consisting of obesity, diabetes, impaired glucose tolerance, arteriosclerosis, hypertension, metabolic bone/cartilage diseases and hyperlipemia.

4. A cell differentiation and/or metabolism regulator comprising a nucleic acid that encodes a protein comprising the same or substantially the same amino acid sequence as the amino acid sequence shown by SEQ ID NO:2 or a partial peptide thereof.

5. The agent of claim 4, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism.

6. The agent of claim 4, which is for prevention/treatment of 1 or more diseases selected from the group consisting of obesity, diabetes, impaired glucose tolerance, arteriosclerosis, hypertension, metabolic bone/cartilage diseases and hyperlipemia.

7. A diagnostic agent for cell differentiation abnormalities and/or metabolic abnormalities comprising a nucleic acid that encodes a protein comprising the same or substantially the same amino acid sequence as the amino acid sequence shown by SEQ ID NO:2 or a partial polynucleotide thereof.

8. The agent of claim 7, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism.

9. The agent of claim 7, which is for diagnosis of 1 or more diseases selected from the group consisting of obesity, diabetes, impaired glucose tolerance, arteriosclerosis, hypertension, metabolic bone/cartilage diseases and hyperlipemia.

10. A diagnostic agent for cell differentiation abnormalities and/or metabolic abnormalities comprising an antibody against a protein comprising the same or substantially the same amino acid sequence as the amino acid sequence shown by SEQ ID NO:2 or a partial peptide thereof or a salt thereof.

11. The agent of claim 10, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism.

12. The agent of claim 10, which is for diagnosis of 1 or more diseases selected from the group consisting of obesity, diabetes, impaired glucose tolerance, arteriosclerosis, hypertension, metabolic bone/cartilage diseases and hyperlipemia.

13. A cell differentiation and/or metabolism regulator comprising an antibody against a protein comprising the same or substantially the same amino acid sequence as the amino acid sequence shown by SEQ ID NO:2 or a partial peptide thereof or a salt thereof.

14. The agent of claim 13, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism.

15. The agent of claim 13, which is for prevention/treatment of 1 or more diseases selected from the group consisting of obesity, diabetes, impaired glucose tolerance, arteriosclerosis, hypertension, metabolic bone/cartilage diseases and hyperlipemia.

16. A cell differentiation and/or metabolism regulator comprising a nucleic acid comprising a base sequence complementary to a nucleic acid that encodes a protein comprising the same or substantially the same amino acid sequence as the amino acid sequence shown by SEQ ID NO:2 or a portion thereof.

17. The agent of claim 16, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism.

18. The agent of claim 16, which is for prevention/treatment of 1 or more diseases selected from the group consisting of obesity, diabetes, impaired glucose tolerance, arteriosclerosis, hypertension, metabolic bone/cartilage diseases and hyperlipemia.

19. A screening method for a substance that exhibits cell differentiation and/or metabolism regulatory action, comprising using a protein comprising the same or substantially the same amino acid sequence as the amino acid sequence shown by SEQ ID NO:2 or a partial peptide thereof or a salt thereof.

20. The method of claim 19, wherein the protein comprising the same or substantially the same amino acid sequence as the amino acid sequence shown by SEQ ID NO:2 or the partial peptide thereof or the salt thereof is provided in the form of a cell that produces the same.

21. The method of claim 20, further comprising using a protein comprising a nucleic acid that encodes the same or substantially the same amino acid sequence as the amino acid sequence shown by SEQ ID NO:2 or a partial polynucleotide thereof, or an antibody against a protein comprising the same or substantially the same amino acid sequence as the amino acid sequence shown by SEQ ID NO:2 or a partial peptide thereof or a salt thereof.

22. The method of claim 19, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism.

**23.** The method of claim 19, wherein the substance possesses prophylactic/therapeutic activity on 1 or more diseases selected from the group consisting of obesity, diabetes, impaired glucose tolerance, arteriosclerosis, hypertension, metabolic bone/cartilage diseases and hyperlipemia.

**24.** A screening method for a substance that alters the bindability of (i) a protein comprising the same or substantially the same amino acid sequence as the amino acid sequence shown by SEQ ID NO:2 or a partial peptide thereof or a salt thereof, and (ii) musclin or a partial peptide thereof or a salt thereof, comprising using both.

**25.** The method of claim 24, wherein the protein comprising the same or substantially the same amino acid sequence as the amino acid sequence shown by SEQ ID NO:2 or the partial peptide thereof or the salt thereof is provided in the form of a cell that produces the same.

**26.** The method of claim 25, wherein sugar uptake in cells or glycogen synthesis in cells is used as an index.

**27.** The method of claim 24, wherein the substance exhibits cell differentiation and/or metabolism regulatory action.

**28.** The method of claim 27, wherein the cell is selected from the group consisting of skeletal muscle cells, fat cells, islet cells, bone cells, cartilage cells and hepatocytes, and wherein the metabolism is selected from the group consisting of sugar metabolism, lipid metabolism and protein metabolism.

**29.** The method of claim 27, wherein the substance possesses prophylactic/therapeutic activity on 1 or more diseases selected from the group consisting of obesity, diabetes, impaired glucose tolerance, arteriosclerosis, hypertension, metabolic bone/cartilage diseases and hyperlipemia, and the like.

**INTERNATIONAL SEARCH REPORT**

International application No.
PCT/JP2006/319605

A. CLASSIFICATION OF SUBJECT MATTER
(See extra sheet)

According to International Patent Classification (IPC) or to both national classification and IPC

B. FIELDS SEARCHED

Minimum documentation searched (classification system followed by classification symbols)
A61K38/00, A61K31/7088, A61K39/395, A61K48/00, A61K49/00, A61P3/04, A61P3/06, A61P3/10, A61P9/10, A61P9/12, A61P19/00, G01N33/15, G01N33/50

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS/CAplus/EMBASE/MEDLINE(STN), JMEDPlus/JST7580/JSTPlus(JDream2), GenBank/EMBL/DDBJ/GeneSeq, SwissProt/PIR/Geneseq

C. DOCUMENTS CONSIDERED TO BE RELEVANT

| Category* | Citation of document, with indication, where appropriate, of the relevant passages | Relevant to claim No. |
|---|---|---|
| X | WO 2004/011618 A2 (HMGENE INC.), 05 February, 2004 (05.02.04), Claims 92, 93, 95, 97 to 116; page 29 to lines 15 to 27; page 66, line 15 to page 69, line 12 & US 2004/259789 A1 & US 2005/065092 A1 | 1-23 |
| X | Potter, L.R. et al., Natriuretic peptides, their receptors, and cyclic guanosine monophosphate-dependent signaling functions, Endocrine Reviews, February 2006, Vol.27, No.1, pp.47-72, Abstract, page 51, left column, 'Osteocrin/musclin' | 1-29 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 November, 2006 (17.11.06) | 28 November, 2006 (28.11.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/319605

C (Continuation). DOCUMENTS CONSIDERED TO BE RELEVANT

| Category* | Citation of document, with indication, where appropriate, of the relevant passages | Relevant to claim No. |
|---|---|---|
| E,X | JP 2006-298798 A (TAKEDA CHEM IND LTD.), 02 November, 2006 (02.11.06), Full text (Family: none) | 1-29 |
| A | WO 2004/111234 A1 (TAKEDA CHEM IND LTD.), 23 December, 2004 (23.12.04), & EP 1634951 A | 1-29 |
| A | Taira WADA, "Kokkakukin Hormone mo Himan ni Kanyo Shiteiru?", Chemistry & chemical industry, 2005, Vol.58, No.1, page 42 | 1-29 |

Form PCT/ISA/210 (continuation of second sheet) (April 2005)

**INTERNATIONAL SEARCH REPORT**

International application No.
PCT/JP2006/319605

Continuation of A. CLASSIFICATION OF SUBJECT MATTER
(International Patent Classification (IPC))

*A61K38/00*(2006.01)i, *A61K31/7088*(2006.01)i, *A61K39/395*(2006.01)i, *A61K48/00*(2006.01)i, *A61K49/00*(2006.01)i, *A61P3/04*(2006.01)i, *A61P3/06*(2006.01)i, *A61P3/10*(2006.01)i, *A61P9/10*(2006.01)i, *A61P9/12*(2006.01)i, *A61P19/00*(2006.01)i, *G01N33/15*(2006.01)i, *G01N33/50*(2006.01)i

(According to International Patent Classification (IPC) or to both national classification and IPC)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 20041112 A
- WO 03054005 A


### Non-patent literature cited in the description


- **Nishizawa, H. et al.** *Journal of Biological Chemistry,* 2004, vol. 279 (19), 19391-19395
- **Thomas, G. et al.** *Journal of Biological Chemistry,* 2003, vol. 278 (50), 50563-50571
- **Bowie et al.** *Science,* 1990, vol. 247, 1306-1310
- **Karlin et al.** *Proc. Natl. Acad. Sci. USA,* 1993, vol. 90, 5873-5877
- **Altschul et al.** *Nucleic Acids Res.,* 1997, vol. 25, 3389-3402
- **Needleman et al.** *J. Mol. Biol.,* 1970, vol. 48, 444-453
- **Myers ; Miller.** *CABIOS,* 1988, vol. 4, 11-17
- **Pearson et al.** *Proc. Natl. Acad. Sci. USA,* 1988, vol. 85, 2444-2448
- **M. Bodanszky ; M.A. Ondetti.** Peptide Synthesis. Interscience Publishers, 1966
- **Schroeder ; Luebke.** The Peptide. Academic Press, 1965
- **Nobuo Izumiya et al.** Peptide Gosei-no-Kiso to Jikken. Maruzen Co, 1975
- Seikagaku Jikken Koza. **Haruaki Yajima ; Shunpei Sakakibara.** Tanpakushitsu no Kagaku. 1977, 205
- Zoku Iyakuhin no Kaihatsu. Peptide Synthesis. Hirokawa Shoten, vol. 14
- **J. Sambrook et al.** Molecular Cloning. Cold Spring Harbor Lab. Press, 1989
- *Proceedings of the National Academy of Sciences of the USA,* 1968, vol. 60, 160
- *Nucleic Acids Research,* 1981, vol. 9, 309
- *Journal of Molecular Biology,* 1978, vol. 120, 517
- *Journal of Molecular Biology,* 1969, vol. 41, 459
- *Genetics,* 1954, vol. 39, 440
- *Gene,* 1983, vol. 24, 255
- *Journal of Biochemistry,* 1984, vol. 95, 87
- **Vaughn, J.L. et al.** *In Vivo,* 1977, vol. 13, 213-217
- **Maeda et al.** *Nature,* 1985, vol. 315, 592
- *Proceedings of the National Academy of Sciences of the USA,* 1972, vol. 69, 2110
- *Gene,* 1982, vol. 17, 107
- *Molecular & General Genetics,* 1979, vol. 168, 111
- *Methods in Enzymology,* 1991, vol. 194, 182-187
- *Proceedings of the National Academy of Sciences of the USA,* 1978, vol. 75, 1929
- *Bio/Technology,* 1988, vol. 6, 47-55
- Saibo Kogaku. Shin Saibo Kogaku Jikken Protocol. Shujunsha, 1995, vol. 8, 263-267
- *Virology,* 1973, vol. 52, 456
- **Miller.** Journal of Experiments in Molecular Genetics. Cold Spring Harbor Laboratory, 1972, 431-433
- **Bostian, K.L. et al.** *Proceedings of the National Academy of Sciences of the USA,* 1980, vol. 77, 4505
- **Bitter, G.A. et al.** *Proceedings of the National Academy of Sciences of the USA,* 1984, vol. 81, 5330
- **Grace, T.C.C.** *Nature,* 1962, vol. 195, 788
- *Science,* 1952, vol. 122, 501
- *Virology,* 1959, vol. 8, 396
- *The Journal of the American Medical Association,* 1967, vol. 199, 519
- *Proceeding of the Society for the Biological Medicine,* 1950, vol. 73, 1
- **J. Kawakami et al.** *Pharm. Tech. Japan,* 1992, vol. 8, 247
- Antisense Research and Applications. CRC Press, 1993
- *Proc. Natl. Acad. Sci. USA,* 2001, vol. 98 (10), 5572-5577
- *Nucleic Acids Res.,* 2001, vol. 29 (13), 2780-2788
- *Nature,* 2001, vol. 411 (6836), 494-498
- **Koehler ; Milstein.** *Nature,* 1975, vol. 256, 495
- *Genomics,* 1989, vol. 5, 874-879
- *Proceedings of the National Academy of Sciences of the United States of America,* 1989, vol. 86, 2766-2770
- Radioimmunoassay. Kodansha Ltd, 1974

- Sequel to the Radioimmunoassay. Kodansha Ltd, 1979
- Enzyme Immonoassay. Igakushoin, 1978
- Enzyme Immonoassay. Igakushoin, 1982
- Enzyme Immonoassay. Igakushoin, 1987
- Immunochemical Techniques (Part A). Methods in ENZYMOLOGY. vol. 70
- METKODS IN ENZYMOLOGY. vol. 73
- (Immunochemical Techniques (Part B)
- Immunochemical Techniques (Part C). METKODS IN ENZYMOLOGY. vol. 74
- Immunochemical Techniques (Part D: Selected Immunoassays). METKODS IN ENZYMOLOGY. vol. 84
- Immunochemical Techniques (Part E: Monoclonal Antibodies and General Immunoassay Methods). METKODS IN ENZYMOLOGY. vol. 92
- Immunochemical Techniques (Part I: Hybridoma Technology and Monoclonal Antibodies). METKODS IN ENZYMOLOGY. Academic Press, vol. 121
- **M.J. Evans ; M.H. Kaufman.** *Nature,* 1981, vol. 292, 154
- **G. R. Martin.** *Proceedings of the National Academy of Sciences of the USA,* 1981, vol. 78, 7634
- **T.C. Doetschman et al.** *Journal of Embryology and Experimental Morphology,* 1985, vol. 87, 27

SEQUENCE LISTING

```
<110>  Takeda Pharmaceutical Company Limited
       OSAKA UNIVERSITY

<120>  MUSCLIN RECEPTOR AND USE THEREOF

<130>  09994

<160>  11

<170>  PatentIn version 3.2

<210>  1
<211>  1620
<212>  DNA
<213>  Homo sapiens


<220>
<221>  CDS
<222>  (1)..(1620)

<400>  1
atg ccg tct ctg ctg gtg ctc act ttc tcc ccg tgc gta cta ctc ggc       48
Met Pro Ser Leu Leu Val Leu Thr Phe Ser Pro Cys Val Leu Leu Gly
1               5                   10                  15

tgg gcg ttg ctg gcc ggc ggc acc ggt ggc ggt ggc gtt ggc ggc ggc       96
Trp Ala Leu Leu Ala Gly Gly Thr Gly Gly Gly Gly Val Gly Gly Gly
            20                  25                  30

ggc ggt ggc gcg ggc ata ggc ggc gga cgc cag gag aga gag gcg ctg      144
Gly Gly Gly Ala Gly Ile Gly Gly Gly Arg Gln Glu Arg Glu Ala Leu
        35                  40                  45

ccg cca cag aag atc gag gtg ctg gtg tta ctg ccc cag gat gac tcg      192
Pro Pro Gln Lys Ile Glu Val Leu Val Leu Leu Pro Gln Asp Asp Ser
    50                  55                  60

tac ttg ttt tca ctc acc cgg gtg cgg ccg gcc atc gag tat gct ctg      240
Tyr Leu Phe Ser Leu Thr Arg Val Arg Pro Ala Ile Glu Tyr Ala Leu
65                  70                  75                  80

cgc agc gtg gag ggc aac ggg act ggg agg cgg ctt ctg ccg ccg ggc      288
Arg Ser Val Glu Gly Asn Gly Thr Gly Arg Arg Leu Leu Pro Pro Gly
                85                  90                  95

act cgc ttc cag gtg gct tac gag gat tca gac tgt ggg aac cgt gcg      336
Thr Arg Phe Gln Val Ala Tyr Glu Asp Ser Asp Cys Gly Asn Arg Ala
            100                 105                 110

ctc ttc agc ttg gtg gac cgc gtg gcg gcg gcg cgg ggc gcc aag cca      384
Leu Phe Ser Leu Val Asp Arg Val Ala Ala Ala Arg Gly Ala Lys Pro
        115                 120                 125

gac ctt atc ctg ggg cca gtg tgc gag tat gca gca gcg cca gtg gcc      432
Asp Leu Ile Leu Gly Pro Val Cys Glu Tyr Ala Ala Ala Pro Val Ala
    130                 135                 140

cgg ctt gca tcg cac tgg gac ctg ccc atg ctg tcg gct ggg gcg ctg      480
```

```
Arg Leu Ala Ser His Trp Asp Leu Pro Met Leu Ser Ala Gly Ala Leu
145                 150                 155                 160

gcc gct ggc ttc cag cac aag gac tct gag tac tcg cac ctc acg cgc      528
Ala Ala Gly Phe Gln His Lys Asp Ser Glu Tyr Ser His Leu Thr Arg
                165                 170                 175

gtg gcg ccc gcc tac gcc aag atg ggc gag atg atg ctc gcc ctg ttc      576
Val Ala Pro Ala Tyr Ala Lys Met Gly Glu Met Met Leu Ala Leu Phe
            180                 185                 190

cgc cac cac cac tgg agc cgc gct gca ctg gtc tac agc gac gac aag      624
Arg His His His Trp Ser Arg Ala Ala Leu Val Tyr Ser Asp Asp Lys
        195                 200                 205

ctg gag cgg aac tgc tac ttc acc ctc gag ggg gtc cac gag gtc ttc      672
Leu Glu Arg Asn Cys Tyr Phe Thr Leu Glu Gly Val His Glu Val Phe
    210                 215                 220

cag gag gag ggt ttg cac acg tcc atc tac agt ttc gac gag acc aaa      720
Gln Glu Glu Gly Leu His Thr Ser Ile Tyr Ser Phe Asp Glu Thr Lys
225                 230                 235                 240

gac ttg gat ctg gaa gac atc gtg cgc aat atc cag gcc agt gag aga      768
Asp Leu Asp Leu Glu Asp Ile Val Arg Asn Ile Gln Ala Ser Glu Arg
                245                 250                 255

gtg gtg atc atg tgt gcg agc agt gac acc atc cgg agc atc atg ctg      816
Val Val Ile Met Cys Ala Ser Ser Asp Thr Ile Arg Ser Ile Met Leu
            260                 265                 270

gtg gcg cac agg cat ggc atg acc agt gga gac tac gcc ttc ttc aac      864
Val Ala His Arg His Gly Met Thr Ser Gly Asp Tyr Ala Phe Phe Asn
        275                 280                 285

att gag ctc ttc aac agc tct tcc tat gga gat ggc tca tgg aag aga      912
Ile Glu Leu Phe Asn Ser Ser Ser Tyr Gly Asp Gly Ser Trp Lys Arg
    290                 295                 300

gga gac aaa cac gac ttt gaa gct aag caa gca tac tcg tcc ctc cag      960
Gly Asp Lys His Asp Phe Glu Ala Lys Gln Ala Tyr Ser Ser Leu Gln
305                 310                 315                 320

aca gtc act cta ctg agg aca gtg aaa cct gag ttt gag aag ttt tcc     1008
Thr Val Thr Leu Leu Arg Thr Val Lys Pro Glu Phe Glu Lys Phe Ser
                325                 330                 335

atg gag gtg aaa agt tca gtt gag aaa caa ggg ctc aat atg gag gat     1056
Met Glu Val Lys Ser Ser Val Glu Lys Gln Gly Leu Asn Met Glu Asp
            340                 345                 350

tac gtt aac atg ttt gtt gaa gga ttc cac gat gcc atc ctc ctc tac     1104
Tyr Val Asn Met Phe Val Glu Gly Phe His Asp Ala Ile Leu Leu Tyr
        355                 360                 365

gtc ttg gct cta cat gaa gta ctc aga gct ggt tac agc aaa aag gat     1152
Val Leu Ala Leu His Glu Val Leu Arg Ala Gly Tyr Ser Lys Lys Asp
    370                 375                 380

gga ggg aaa att ata cag cag act tgg aac aga aca ttt gaa ggt atc     1200
Gly Gly Lys Ile Ile Gln Gln Thr Trp Asn Arg Thr Phe Glu Gly Ile
```

```
385                 390                 395                 400

gcc ggg cag gtg tcc ata gat gcc aac gga gac cga tat ggg gat ttc      1248
Ala Gly Gln Val Ser Ile Asp Ala Asn Gly Asp Arg Tyr Gly Asp Phe
                405                 410                 415

tct gtg att gcc atg act gat gtg gag gcg ggc acc cag gag gtt att      1296
Ser Val Ile Ala Met Thr Asp Val Glu Ala Gly Thr Gln Glu Val Ile
            420                 425                 430

ggt gat tat ttt gga aaa gaa ggt cgt ttt gaa atg cgg ccg aat gtc      1344
Gly Asp Tyr Phe Gly Lys Glu Gly Arg Phe Glu Met Arg Pro Asn Val
        435                 440                 445

aaa tat cct tgg ggc cct tta aaa ctg aga ata gat gaa aac cga att      1392
Lys Tyr Pro Trp Gly Pro Leu Lys Leu Arg Ile Asp Glu Asn Arg Ile
    450                 455                 460

gta gag cat aca aac agc tct ccc tgc aaa tca tgt ggc cta gaa gaa      1440
Val Glu His Thr Asn Ser Ser Pro Cys Lys Ser Cys Gly Leu Glu Glu
465                 470                 475                 480

tcg gca gtg aca gga att gtc gtg ggg gct tta cta gga gct ggc ttg      1488
Ser Ala Val Thr Gly Ile Val Val Gly Ala Leu Leu Gly Ala Gly Leu
                485                 490                 495

cta atg gcc ttc tac ttt ttc agg aag aaa tac aga ata acc att gag      1536
Leu Met Ala Phe Tyr Phe Phe Arg Lys Lys Tyr Arg Ile Thr Ile Glu
            500                 505                 510

agg cga acc cag caa gaa gaa agt aac ctt gga aaa cat cgg gaa tta      1584
Arg Arg Thr Gln Gln Glu Glu Ser Asn Leu Gly Lys His Arg Glu Leu
        515                 520                 525

cgg gaa gat tcc atc aga tcc cat ttt tca gta gct                      1620
Arg Glu Asp Ser Ile Arg Ser His Phe Ser Val Ala
    530                 535                 540


<210>  2
<211>  540
<212>  PRT
<213>  Homo sapiens

<400>  2

Met Pro Ser Leu Leu Val Leu Thr Phe Ser Pro Cys Val Leu Leu Gly
1               5                   10                  15


Trp Ala Leu Leu Ala Gly Gly Thr Gly Gly Gly Gly Val Gly Gly Gly
            20                  25                  30


Gly Gly Gly Ala Gly Ile Gly Gly Gly Arg Gln Glu Arg Glu Ala Leu
        35                  40                  45


Pro Pro Gln Lys Ile Glu Val Leu Val Leu Leu Pro Gln Asp Asp Ser
    50                  55                  60
```

```
Tyr Leu Phe Ser Leu Thr Arg Val Arg Pro Ala Ile Glu Tyr Ala Leu
65                  70                  75                  80

Arg Ser Val Glu Gly Asn Gly Thr Gly Arg Arg Leu Leu Pro Pro Gly
                85                  90                  95

Thr Arg Phe Gln Val Ala Tyr Glu Asp Ser Asp Cys Gly Asn Arg Ala
            100                 105                 110

Leu Phe Ser Leu Val Asp Arg Val Ala Ala Ala Arg Gly Ala Lys Pro
        115                 120                 125

Asp Leu Ile Leu Gly Pro Val Cys Glu Tyr Ala Ala Ala Pro Val Ala
    130                 135                 140

Arg Leu Ala Ser His Trp Asp Leu Pro Met Leu Ser Ala Gly Ala Leu
145                 150                 155                 160

Ala Ala Gly Phe Gln His Lys Asp Ser Glu Tyr Ser His Leu Thr Arg
                165                 170                 175

Val Ala Pro Ala Tyr Ala Lys Met Gly Glu Met Met Leu Ala Leu Phe
            180                 185                 190

Arg His His His Trp Ser Arg Ala Ala Leu Val Tyr Ser Asp Asp Lys
        195                 200                 205

Leu Glu Arg Asn Cys Tyr Phe Thr Leu Glu Gly Val His Glu Val Phe
    210                 215                 220

Gln Glu Glu Gly Leu His Thr Ser Ile Tyr Ser Phe Asp Glu Thr Lys
225                 230                 235                 240

Asp Leu Asp Leu Glu Asp Ile Val Arg Asn Ile Gln Ala Ser Glu Arg
                245                 250                 255

Val Val Ile Met Cys Ala Ser Ser Asp Thr Ile Arg Ser Ile Met Leu
            260                 265                 270

Val Ala His Arg His Gly Met Thr Ser Gly Asp Tyr Ala Phe Phe Asn
        275                 280                 285

Ile Glu Leu Phe Asn Ser Ser Ser Tyr Gly Asp Gly Ser Trp Lys Arg
    290                 295                 300
```

```
Gly Asp Lys His Asp Phe Glu Ala Lys Gln Ala Tyr Ser Ser Leu Gln
305                 310                 315                 320

Thr Val Thr Leu Leu Arg Thr Val Lys Pro Glu Phe Glu Lys Phe Ser
                325                 330                 335

Met Glu Val Lys Ser Ser Val Glu Lys Gln Gly Leu Asn Met Glu Asp
            340                 345                 350

Tyr Val Asn Met Phe Val Glu Gly Phe His Asp Ala Ile Leu Leu Tyr
        355                 360                 365

Val Leu Ala Leu His Glu Val Leu Arg Ala Gly Tyr Ser Lys Lys Asp
    370                 375                 380

Gly Gly Lys Ile Ile Gln Gln Thr Trp Asn Arg Thr Phe Glu Gly Ile
385                 390                 395                 400

Ala Gly Gln Val Ser Ile Asp Ala Asn Gly Asp Arg Tyr Gly Asp Phe
                405                 410                 415

Ser Val Ile Ala Met Thr Asp Val Glu Ala Gly Thr Gln Glu Val Ile
            420                 425                 430

Gly Asp Tyr Phe Gly Lys Glu Gly Arg Phe Glu Met Arg Pro Asn Val
        435                 440                 445

Lys Tyr Pro Trp Gly Pro Leu Lys Leu Arg Ile Asp Glu Asn Arg Ile
    450                 455                 460

Val Glu His Thr Asn Ser Ser Pro Cys Lys Ser Cys Gly Leu Glu Glu
465                 470                 475                 480

Ser Ala Val Thr Gly Ile Val Val Gly Ala Leu Leu Gly Ala Gly Leu
                485                 490                 495

Leu Met Ala Phe Tyr Phe Phe Arg Lys Lys Tyr Arg Ile Thr Ile Glu
            500                 505                 510

Arg Arg Thr Gln Gln Glu Glu Ser Asn Leu Gly Lys His Arg Glu Leu
        515                 520                 525

Arg Glu Asp Ser Ile Arg Ser His Phe Ser Val Ala
    530                 535                 540
```

<210>  3

<211> 23
<212> DNA
<213> Artificial

<220>
<223> Primer

<400> 3
ttagcatcac aggagtttgg aac 23

<210> 4
<211> 23
<212> DNA
<213> Artificial

<220>
<223> Primer

<400> 4
tcagcctctg gaactggaga gcc 23

<210> 5
<211> 306
<212> DNA
<213> Artificial

<220>
<223> PCR Product

<400> 5
ttagcatcac aggagtttgg aacagcaagc ttgcagtctc cacccacagc cagagaagag 60

aagtcagcca ctgagctttc ggctaagctc ctgcgtcttg atgatctggt gtccttagag 120

aatgacgtat ttgagaccaa gaaaaagaga agcttctctg gctttgggtc tccccttgac 180

agactctcag ctgggtctgt agagcataga gggaaacaaa ggaaagcagt agatcattca 240

aaaaagcggt ttggtattcc catggatcgg attggtagaa accggctctc cagttccaga 300

ggctga 306

<210> 6
<211> 87
<212> DNA
<213> Mus musculus

<220>
<221> misc_feature
<222> (1)..(87)
<223> DNA sequence encoding for signal peptide of mMusclin.

<400> 6
atgctggact ggagattggc aagtacacac ttcatcctgg ctatgattgt gatgctgtgg 60

ggctcaggaa aggcattctc tgtggac 87

<210> 7
<211> 303
<212> DNA
<213> Mus musculus

<220>
<221> misc_feature
<222> (1)..(303)
<223> DNA sequence encoding for mature mMusclin.

<400> 7
ttagcatcac aggagtttgg aacagcaagc ttgcagtctc cacccacagc cagagaagag 60

aagtcagcca ctgagctttc ggctaagctc ctgcgtcttg atgatctggt gtccttagag 120

aatgacgtat ttgagaccaa gaaaaagaga agcttctctg gctttgggtc tccccttgac 180

agactctcag ctgggtctgt agagcataga gggaaacaaa ggaaagcagt agatcattca 240

aaaaagcggt ttggtattcc catggatcgg attggtagaa accggctctc cagttccaga 300

ggc 303

<210> 8
<211> 369
<212> DNA
<213> Artificial

<220>
<223> Recombinant DNA encoding for His-tagged mMusclin.

<400> 8
atgggcagca gccatcatca tcatcatcac agcagcggcc tggtgccgta cggctgccat 60

atgttagcat cacaggagtt tggaacagca agcttgcagt ctccacccac agccagagaa 120

gagaagtcag ccactgagct ttcggctaag ctcctgcgtc ttgatgatct ggtgtcctta 180

gagaatgacg tatttgagac caagaaaaag agaagcttct ctggctttgg gtctcccctt 240

gacagactct cagctgggtc tgtagagcat agagggaaac aaaggaaagc agtagatcat 300

tcaaaaaagc ggtttggtat tcccatggat cggattggta gaaaccggct ctccagttcc 360

agaggctga 369

<210> 9
<211> 40
<212> DNA
<213> Artificial

<220>
<223> Primer

<400> 9
ggtattgagg gtcgcttagc atcacaggag tttggaacag 40

```
<210>  10
<211>  42
<212>  DNA
<213>  Artificial

<220>
<223>  Primer

<400>  10
agaggagagt tagagcctca gcctctggaa ctggagagcc gg                    42


<210>  11
<211>  40
<212>  DNA
<213>  Artificial

<220>
<223>  Primer

<400>  11
ggtattgagg gtcgcagctt ctctggcttt gggtctcccc                      40
```